# EUROPEAN PATENT APPLICATION

(11) **EP 3 318 124 A2**
(43) Date of publication of application: **09.05.2018**
(21) Application number: 17186961.3
(22) Date of filing: 14.02.2014
(51) Int. Cl.: A01K 67/00

(54) **PHARMACOKINETIC ANIMAL MODEL**

(30) Priority: 16.02.2013 EP 13155554; 15.08.2013 EP 13180587
(62) Divisional of application: 14705493.6
(71) Applicant: Albumedix A/S, 2800 Kgs. Lyngby (DK)
(72) Inventor: CAMERON, Jason, Nottingham NG3 6HB (GB); SLEEP, Darrell, Nottingham NG2 5DS (GB); SANDLIE, Inger, 0752 Oslo (NO); ANDERSEN, Jan Terje, 1256 Oslo (NO)
(74) Representative: Williams, Rachel Clare

(57) **Abstract**

The present invention relates to a method of assessing pharmacokinetic properties of a variant of human serum albumin using a non-primate animal species where the native albumin of the animal provides minimal competition for HSA binding to the FcRn receptor in said animal. In the non-primate animal species, the binding affinity of wild type HSA to the native FcRn of said animal is the same as or higher than the binding affinity of the native albumin of said animal to the native FcRn. The present invention also relate to animal models which are particularly suitable for assessing pharmacokinetics of human serum albumin variants.

SEQUENCE LISTING

<110>  Albumedix A/S

<120>  Pharmacokinetic animal model

<130>  12568-EP-ETD

<150>  EP13155554.2
<151>  2013-02-16

<150>  EP13180587.1
<151>  2013-08-15

<150>  PCT/EP2014/052944
<151>  2014-02-14

<150>  EP14705493.6
<151>  2014-02-14

<160>  23

<170>  PatentIn version 3.5

<210>  1
<211>  609
<212>  PRT
<213>  Homo sapiens

<400>  1

<210>  2
<211>  585

<212>  PRT
<213>  Homo sapiens

<400>  2

<210>  3
<211>  608
<212>  PRT
<213>  Macaca mulatta

<400>  3

Met Lys Trp Val Thr Phe Ile Ser Leu Leu Phe Leu Phe Ser Ser Ala
1               5                   10                  15

Tyr Ser Arg Gly Val Phe Arg Arg Asp Thr His Lys Ser Glu Val Ala
            20                  25                  30

His Arg Phe Lys Asp Leu Gly Glu Glu His Phe Lys Gly Leu Val Leu
        35                  40                  45

Val Ala Phe Ser Gln Tyr Leu Gln Gln Cys Pro Phe Glu Glu His Val
    50                  55                  60

Lys Leu Val Asn Glu Val Thr Glu Phe Ala Lys Thr Cys Val Ala Asp
65                  70                  75                  80

Glu Ser Ala Glu Asn Cys Asp Lys Ser Leu His Thr Leu Phe Gly Asp
                85                  90                  95

Lys Leu Cys Thr Val Ala Thr Leu Arg Glu Thr Tyr Gly Glu Met Ala
            100                 105                 110

Asp Cys Cys Ala Lys Gln Glu Pro Glu Arg Asn Glu Cys Phe Leu Gln

115                 120                 125

His Lys Asp Asp Asn Pro Asn Leu Pro Pro Leu Val Arg Pro Glu Val
    130                 135                 140

Asp Val Met Cys Thr Ala Phe His Asp Asn Glu Ala Thr Phe Leu Lys
145                 150                 155                 160

Lys Tyr Leu Tyr Glu Val Ala Arg Arg His Pro Tyr Phe Tyr Ala Pro
                165                 170                 175

Glu Leu Leu Phe Phe Ala Ala Arg Tyr Lys Ala Ala Phe Ala Glu Cys
            180                 185                 190

Cys Gln Ala Ala Asp Lys Ala Ala Cys Leu Leu Pro Lys Leu Asp Glu
        195                 200                 205

Leu Arg Asp Glu Gly Lys Ala Ser Ser Ala Lys Gln Arg Leu Lys Cys
    210                 215                 220

Ala Ser Leu Gln Lys Phe Gly Asp Arg Ala Phe Lys Ala Trp Ala Val
225                 230                 235                 240

Ala Arg Leu Ser Gln Lys Phe Pro Lys Ala Glu Phe Ala Glu Val Ser
                245                 250                 255

Lys Leu Val Thr Asp Leu Thr Lys Val His Thr Glu Cys Cys His Gly
            260                 265                 270

Asp Leu Leu Glu Cys Ala Asp Asp Arg Ala Asp Leu Ala Lys Tyr Met
        275                 280                 285

Cys Glu Asn Gln Asp Ser Ile Ser Ser Lys Leu Lys Glu Cys Cys Asp
    290                 295                 300

Lys Pro Leu Leu Glu Lys Ser His Cys Leu Ala Glu Val Glu Asn Asp
305                 310                 315                 320

Glu Met Pro Ala Asp Leu Pro Ser Leu Ala Ala Asp Tyr Val Glu Ser
                325                 330                 335

Lys Asp Val Cys Lys Asn Tyr Ala Glu Ala Lys Asp Val Phe Leu Gly
            340                 345                 350

Met Phe Leu Tyr Glu Tyr Ala Arg Arg His Pro Asp Tyr Ser Val Met
        355                 360                 365

Leu Leu Leu Arg Leu Ala Lys Ala Tyr Glu Ala Thr Leu Glu Lys Cys
    370                 375                 380

Cys Ala Ala Ala Asp Pro His Glu Cys Tyr Ala Lys Val Phe Asp Glu
385                 390                 395                 400

Phe Gln Pro Leu Val Glu Glu Pro Gln Asn Leu Val Lys Gln Asn Cys
                405                 410                 415

Glu Leu Phe Glu Gln Leu Gly Glu Tyr Lys Phe Gln Asn Ala Leu Leu
            420                 425                 430

Val Arg Tyr Thr Lys Lys Val Pro Gln Val Ser Thr Pro Thr Leu Val
        435                 440                 445

Glu Val Ser Arg Asn Leu Gly Lys Val Gly Ala Lys Cys Cys Lys Leu
    450                 455                 460

Pro Glu Ala Lys Arg Met Pro Cys Ala Glu Asp Tyr Leu Ser Val Val
465                 470                 475                 480

Leu Asn Arg Leu Cys Val Leu His Glu Lys Thr Pro Val Ser Glu Lys
                485                 490                 495

Val Thr Lys Cys Cys Thr Glu Ser Leu Val Asn Arg Arg Pro Cys Phe
            500                 505                 510

Ser Ala Leu Glu Leu Asp Glu Ala Tyr Val Pro Lys Ala Phe Asn Ala
        515                 520                 525

Glu Thr Phe Thr Phe His Ala Asp Met Cys Thr Leu Ser Glu Lys Glu
    530                 535                 540

Lys Gln Val Lys Lys Gln Thr Ala Leu Val Glu Leu Val Lys His Lys
545                 550                 555                 560

Pro Lys Ala Thr Lys Glu Gln Leu Lys Gly Val Met Asp Asn Phe Ala
                565                 570                 575

Ala Phe Val Glu Lys Cys Cys Lys Ala Asp Asp Lys Glu Ala Cys Phe
            580                 585                 590

Ala Glu Glu Gly Pro Lys Phe Val Ala Ala Ser Gln Ala Ala Leu Ala
        595                 600                 605

<210>  4
<211>  608
<212>  PRT

<213>  Canis lupus familiaris

<400>  4

Met Lys Trp Val Thr Phe Ile Ser Leu Phe Phe Leu Phe Ser Ser Ala
1               5                   10                  15

Tyr Ser Arg Gly Leu Val Arg Arg Glu Ala Tyr Lys Ser Glu Ile Ala
            20                  25                  30

His Arg Tyr Asn Asp Leu Gly Glu Glu His Phe Arg Gly Leu Val Leu
        35                  40                  45

Val Ala Phe Ser Gln Tyr Leu Gln Gln Cys Pro Phe Glu Asp His Val
    50                  55                  60

Lys Leu Ala Lys Glu Val Thr Glu Phe Ala Lys Ala Cys Ala Ala Glu
65                  70                  75                  80

Glu Ser Gly Ala Asn Cys Asp Lys Ser Leu His Thr Leu Phe Gly Asp
                85                  90                  95

Lys Leu Cys Thr Val Ala Ser Leu Arg Asp Lys Tyr Gly Asp Met Ala
            100                 105                 110

Asp Cys Cys Glu Lys Gln Glu Pro Asp Arg Asn Glu Cys Phe Leu Ala
        115                 120                 125

His Lys Asp Asp Asn Pro Gly Phe Pro Pro Leu Val Ala Pro Glu Pro
    130                 135                 140

Asp Ala Leu Cys Ala Ala Phe Gln Asp Asn Glu Gln Leu Phe Leu Gly
145                 150                 155                 160

Lys Tyr Leu Tyr Glu Ile Ala Arg Arg His Pro Tyr Phe Tyr Ala Pro
                165                 170                 175

Glu Leu Leu Tyr Tyr Ala Gln Gln Tyr Lys Gly Val Phe Ala Glu Cys
            180                 185                 190

Cys Gln Ala Ala Asp Lys Ala Ala Cys Leu Gly Pro Lys Ile Glu Ala
        195                 200                 205

Leu Arg Glu Lys Val Leu Leu Ser Ser Ala Lys Glu Arg Phe Lys Cys
    210                 215                 220

Ala Ser Leu Gln Lys Phe Gly Asp Arg Ala Phe Lys Ala Trp Ser Val
225                 230                 235                 240

Ala Arg Leu Ser Gln Arg Phe Pro Lys Ala Asp Phe Ala Glu Ile Ser
                245                 250                 255

Lys Val Val Thr Asp Leu Thr Lys Val His Lys Glu Cys Cys His Gly
            260                 265                 270

Asp Leu Leu Glu Cys Ala Asp Asp Arg Ala Asp Leu Ala Lys Tyr Met
        275                 280                 285

Cys Glu Asn Gln Asp Ser Ile Ser Thr Lys Leu Lys Glu Cys Cys Asp
    290                 295                 300

Lys Pro Val Leu Glu Lys Ser Gln Cys Leu Ala Glu Val Glu Arg Asp
305                 310                 315                 320

Glu Leu Pro Gly Asp Leu Pro Ser Leu Ala Ala Asp Phe Val Glu Asp
                325                 330                 335

Lys Glu Val Cys Lys Asn Tyr Gln Glu Ala Lys Asp Val Phe Leu Gly
            340                 345                 350

Thr Phe Leu Tyr Glu Tyr Ala Arg Arg His Pro Glu Tyr Ser Val Ser
        355                 360                 365

Leu Leu Leu Arg Leu Ala Lys Glu Tyr Glu Ala Thr Leu Glu Lys Cys
    370                 375                 380

Cys Ala Thr Asp Asp Pro Pro Thr Cys Tyr Ala Lys Val Leu Asp Glu
385                 390                 395                 400

Phe Lys Pro Leu Val Asp Glu Pro Gln Asn Leu Val Lys Thr Asn Cys
                405                 410                 415

Glu Leu Phe Glu Lys Leu Gly Glu Tyr Gly Phe Gln Asn Ala Leu Leu
            420                 425                 430

Val Arg Tyr Thr Lys Lys Ala Pro Gln Val Ser Thr Pro Thr Leu Val
        435                 440                 445

Glu Val Ser Arg Lys Leu Gly Lys Val Gly Thr Lys Cys Cys Lys Lys
    450                 455                 460

Pro Glu Ser Glu Arg Met Ser Cys Ala Glu Asp Phe Leu Ser Val Val
465                 470                 475                 480

Leu Asn Arg Leu Cys Val Leu His Glu Lys Thr Pro Val Ser Glu Arg
                485                 490                 495

Val Thr Lys Cys Cys Ser Glu Ser Leu Val Asn Arg Arg Pro Cys Phe
                500                 505                 510

Ser Gly Leu Glu Val Asp Glu Thr Tyr Val Pro Lys Glu Phe Asn Ala
            515                 520                 525

Glu Thr Phe Thr Phe His Ala Asp Leu Cys Thr Leu Pro Glu Ala Glu
    530                 535                 540

Lys Gln Val Lys Lys Gln Thr Ala Leu Val Glu Leu Leu Lys His Lys
545                 550                 555                 560

Pro Lys Ala Thr Asp Glu Gln Leu Lys Thr Val Met Gly Asp Phe Gly
                565                 570                 575

Ala Phe Val Glu Lys Cys Cys Ala Ala Glu Asn Lys Glu Gly Cys Phe
                580                 585                 590

Ser Glu Glu Gly Pro Lys Leu Val Ala Ala Ala Gln Ala Ala Leu Val
            595                 600                 605

<210>  5
<211>  607
<212>  PRT
<213>  Sus scrofa

<400>  5

Met Lys Trp Val Thr Phe Ile Ser Leu Leu Phe Leu Phe Ser Ser Ala
1               5                   10                  15

Tyr Ser Arg Gly Val Phe Arg Arg Asp Thr Tyr Lys Ser Glu Ile Ala
            20                  25                  30

His Arg Phe Lys Asp Leu Gly Glu Gln Tyr Phe Lys Gly Leu Val Leu
        35                  40                  45

Ile Ala Phe Ser Gln His Leu Gln Gln Cys Pro Tyr Glu Glu His Val
    50                  55                  60

Lys Leu Val Arg Glu Val Thr Glu Phe Ala Lys Thr Cys Val Ala Asp
65                  70                  75                  80

Glu Ser Ala Glu Asn Cys Asp Lys Ser Ile His Thr Leu Phe Gly Asp
                85                  90                  95

Lys Leu Cys Ala Ile Pro Ser Leu Arg Glu His Tyr Gly Asp Leu Ala
            100                 105                 110

Asp Cys Cys Glu Lys Glu Glu Pro Glu Arg Asn Glu Cys Phe Leu Gln
        115                 120                 125

His Lys Asn Asp Asn Pro Asp Ile Pro Lys Leu Lys Pro Asp Pro Val
    130                 135                 140

Ala Leu Cys Ala Asp Phe Gln Glu Asp Glu Gln Lys Phe Trp Gly Lys
145                 150                 155                 160

Tyr Leu Tyr Glu Ile Ala Arg Arg His Pro Tyr Phe Tyr Ala Pro Glu
                165                 170                 175

Leu Leu Tyr Tyr Ala Ile Ile Tyr Lys Asp Val Phe Ser Glu Cys Cys
            180                 185                 190

Gln Ala Ala Asp Lys Ala Ala Cys Leu Leu Pro Lys Ile Glu His Leu
        195                 200                 205

Arg Glu Lys Val Leu Thr Ser Ala Ala Lys Gln Arg Leu Lys Cys Ala
    210                 215                 220

Ser Ile Gln Lys Phe Gly Glu Arg Ala Phe Lys Ala Trp Ser Leu Ala
225                 230                 235                 240

Arg Leu Ser Gln Arg Phe Pro Lys Ala Asp Phe Thr Glu Ile Ser Lys
                245                 250                 255

Ile Val Thr Asp Leu Ala Lys Val His Lys Glu Cys Cys His Gly Asp
            260                 265                 270

Leu Leu Glu Cys Ala Asp Asp Arg Ala Asp Leu Ala Lys Tyr Ile Cys
        275                 280                 285

Glu Asn Gln Asp Thr Ile Ser Thr Lys Leu Lys Glu Cys Cys Asp Lys
    290                 295                 300

Pro Leu Leu Glu Lys Ser His Cys Ile Ala Glu Ala Lys Arg Asp Glu
305                 310                 315                 320

Leu Pro Ala Asp Leu Asn Pro Leu Glu His Asp Phe Val Glu Asp Lys
                325                 330                 335

Glu Val Cys Lys Asn Tyr Lys Glu Ala Lys His Val Phe Leu Gly Thr
            340                 345                 350

Phe Leu Tyr Glu Tyr Ser Arg Arg His Pro Asp Tyr Ser Val Ser Leu

355                 360                 365

Leu Leu Arg Ile Ala Lys Ile Tyr Glu Ala Thr Leu Glu Asp Cys Cys
    370                 375                 380

Ala Lys Glu Asp Pro Pro Ala Cys Tyr Ala Thr Val Phe Asp Lys Phe
385                 390                 395                 400

Gln Pro Leu Val Asp Glu Pro Lys Asn Leu Ile Lys Gln Asn Cys Glu
                405                 410                 415

Leu Phe Glu Lys Leu Gly Glu Tyr Gly Phe Gln Asn Ala Leu Ile Val
            420                 425                 430

Arg Tyr Thr Lys Lys Val Pro Gln Val Ser Thr Pro Thr Leu Val Glu
        435                 440                 445

Val Ala Arg Lys Leu Gly Leu Val Gly Ser Arg Cys Cys Lys Arg Pro
    450                 455                 460

Glu Glu Glu Arg Leu Ser Cys Ala Glu Asp Tyr Leu Ser Leu Val Leu
465                 470                 475                 480

Asn Arg Leu Cys Val Leu His Glu Lys Thr Pro Val Ser Glu Lys Val
                485                 490                 495

Thr Lys Cys Cys Thr Glu Ser Leu Val Asn Arg Arg Pro Cys Phe Ser
            500                 505                 510

Ala Leu Thr Pro Asp Glu Thr Tyr Lys Pro Lys Glu Phe Val Glu Gly
        515                 520                 525

Thr Phe Thr Phe His Ala Asp Leu Cys Thr Leu Pro Glu Asp Glu Lys
    530                 535                 540

Gln Ile Lys Lys Gln Thr Ala Leu Val Glu Leu Leu Lys His Lys Pro
545                 550                 555                 560

His Ala Thr Glu Glu Gln Leu Arg Thr Val Leu Gly Asn Phe Ala Ala
                565                 570                 575

Phe Val Gln Lys Cys Cys Ala Ala Pro Asp His Glu Ala Cys Phe Ala
            580                 585                 590

Val Glu Gly Pro Lys Phe Val Ile Glu Ile Arg Gly Ile Leu Ala
        595                 600                 605

<210>  6
<211>  608
<212>  PRT
<213>  Rattus norvegicus

<400>  6

Met Lys Trp Val Thr Phe Leu Leu Leu Leu Phe Ile Ser Gly Ser Ala
1               5                   10                  15

Phe Ser Arg Gly Val Phe Arg Arg Glu Ala His Lys Ser Glu Ile Ala
            20                  25                  30

His Arg Phe Lys Asp Leu Gly Glu Gln His Phe Lys Gly Leu Val Leu
        35                  40                  45

Ile Ala Phe Ser Gln Tyr Leu Gln Lys Cys Pro Tyr Glu Glu His Ile
    50                  55                  60

Lys Leu Val Gln Glu Val Thr Asp Phe Ala Lys Thr Cys Val Ala Asp
65                  70                  75                  80

Glu Asn Ala Glu Asn Cys Asp Lys Ser Ile His Thr Leu Phe Gly Asp
                85                  90                  95

Lys Leu Cys Ala Ile Pro Lys Leu Arg Asp Asn Tyr Gly Glu Leu Ala
            100                 105                 110

Asp Cys Cys Ala Lys Gln Glu Pro Glu Arg Asn Glu Cys Phe Leu Gln
        115                 120                 125

His Lys Asp Asp Asn Pro Asn Leu Pro Pro Phe Gln Arg Pro Glu Ala
    130                 135                 140

Glu Ala Met Cys Thr Ser Phe Gln Glu Asn Pro Thr Ser Phe Leu Gly
145                 150                 155                 160

His Tyr Leu His Glu Val Ala Arg Arg His Pro Tyr Phe Tyr Ala Pro
                165                 170                 175

Glu Leu Leu Tyr Tyr Ala Glu Lys Tyr Asn Glu Val Leu Thr Gln Cys
            180                 185                 190

Cys Thr Glu Ser Asp Lys Ala Ala Cys Leu Thr Pro Lys Leu Asp Ala
        195                 200                 205

Val Lys Glu Lys Ala Leu Val Ala Ala Val Arg Gln Arg Met Lys Cys
    210                 215                 220

Ser Ser Met Gln Arg Phe Gly Glu Arg Ala Phe Lys Ala Trp Ala Val
225                 230                 235                 240

Ala Arg Met Ser Gln Arg Phe Pro Asn Ala Glu Phe Ala Glu Ile Thr
                245                 250                 255

Lys Leu Ala Thr Asp Val Thr Lys Ile Asn Lys Glu Cys Cys His Gly
            260                 265                 270

Asp Leu Leu Glu Cys Ala Asp Asp Arg Ala Glu Leu Ala Lys Tyr Met
        275                 280                 285

Cys Glu Asn Gln Ala Thr Ile Ser Ser Lys Leu Gln Ala Cys Cys Asp
    290                 295                 300

Lys Pro Val Leu Gln Lys Ser Gln Cys Leu Ala Glu Ile Glu His Asp
305                 310                 315                 320

Asn Ile Pro Ala Asp Leu Pro Ser Ile Ala Ala Asp Phe Val Glu Asp
                325                 330                 335

Lys Glu Val Cys Lys Asn Tyr Ala Glu Ala Lys Asp Val Phe Leu Gly
            340                 345                 350

Thr Phe Leu Tyr Glu Tyr Ser Arg Arg His Pro Asp Tyr Ser Val Ser
        355                 360                 365

Leu Leu Leu Arg Leu Ala Lys Lys Tyr Glu Ala Thr Leu Glu Lys Cys
    370                 375                 380

Cys Ala Glu Gly Asp Pro Pro Ala Cys Tyr Gly Thr Val Leu Ala Glu
385                 390                 395                 400

Phe Gln Pro Leu Val Glu Glu Pro Lys Asn Leu Val Lys Thr Asn Cys
                405                 410                 415

Glu Leu Tyr Glu Lys Leu Gly Glu Tyr Gly Phe Gln Asn Ala Val Leu
            420                 425                 430

Val Arg Tyr Thr Gln Lys Ala Pro Gln Val Ser Thr Pro Thr Leu Val
        435                 440                 445

Glu Ala Ala Arg Asn Leu Gly Arg Val Gly Thr Lys Cys Cys Thr Leu
    450                 455                 460

Pro Glu Ala Gln Arg Leu Pro Cys Val Glu Asp Tyr Leu Ser Ala Ile
465                 470                 475                 480

Leu Asn Arg Leu Cys Val Leu His Glu Lys Thr Pro Val Ser Glu Lys
                485                 490                 495

Val Thr Lys Cys Cys Ser Gly Ser Leu Val Glu Arg Arg Pro Cys Phe
            500                 505                 510

Ser Ala Leu Thr Val Asp Glu Thr Tyr Val Pro Lys Glu Phe Lys Ala
        515                 520                 525

Glu Thr Phe Thr Phe His Ser Asp Ile Cys Thr Leu Pro Asp Lys Glu
    530                 535                 540

Lys Gln Ile Lys Lys Gln Thr Ala Leu Ala Glu Leu Val Lys His Lys
545                 550                 555                 560

Pro Lys Ala Thr Glu Asp Gln Leu Lys Thr Val Met Gly Asp Phe Ala
                565                 570                 575

Gln Phe Val Asp Lys Cys Cys Lys Ala Ala Asp Lys Asp Asn Cys Phe
            580                 585                 590

Ala Thr Glu Gly Pro Asn Leu Val Ala Arg Ser Lys Glu Ala Leu Ala
        595                 600                 605

<210>  7
<211>  608
<212>  PRT
<213>  Mus musculus

<400>  7

Met Lys Trp Val Thr Phe Leu Leu Leu Leu Phe Val Ser Gly Ser Ala
1               5                   10                  15

Phe Ser Arg Gly Val Phe Arg Arg Glu Ala His Lys Ser Glu Ile Ala
            20                  25                  30

His Arg Tyr Asn Asp Leu Gly Glu Gln His Phe Lys Gly Leu Val Leu
        35                  40                  45

Ile Ala Phe Ser Gln Tyr Leu Gln Lys Cys Ser Tyr Asp Glu His Ala
    50                  55                  60

Lys Leu Val Gln Glu Val Thr Asp Phe Ala Lys Thr Cys Val Ala Asp
65                  70                  75                  80

Glu Ser Ala Ala Asn Cys Asp Lys Ser Leu His Thr Leu Phe Gly Asp
                85                  90                  95

Lys Leu Cys Ala Ile Pro Asn Leu Arg Glu Asn Tyr Gly Glu Leu Ala
            100                 105                 110

Asp Cys Cys Thr Lys Gln Glu Pro Glu Arg Asn Glu Cys Phe Leu Gln
        115                 120                 125

His Lys Asp Asp Asn Pro Ser Leu Pro Pro Phe Glu Arg Pro Glu Ala
    130                 135                 140

Glu Ala Met Cys Thr Ser Phe Lys Glu Asn Pro Thr Thr Phe Met Gly
145                 150                 155                 160

His Tyr Leu His Glu Val Ala Arg Arg His Pro Tyr Phe Tyr Ala Pro
                165                 170                 175

Glu Leu Leu Tyr Tyr Ala Glu Gln Tyr Asn Glu Ile Leu Thr Gln Cys
            180                 185                 190

Cys Ala Glu Ala Asp Lys Glu Ser Cys Leu Thr Pro Lys Leu Asp Gly
        195                 200                 205

Val Lys Glu Lys Ala Leu Val Ser Ser Val Arg Gln Arg Met Lys Cys
    210                 215                 220

Ser Ser Met Gln Lys Phe Gly Glu Arg Ala Phe Lys Ala Trp Ala Val
225                 230                 235                 240

Ala Arg Leu Ser Gln Thr Phe Pro Asn Ala Asp Phe Ala Glu Ile Thr
                245                 250                 255

Lys Leu Ala Thr Asp Leu Thr Lys Val Asn Lys Glu Cys Cys His Gly
            260                 265                 270

Asp Leu Leu Glu Cys Ala Asp Asp Arg Ala Glu Leu Ala Lys Tyr Met
        275                 280                 285

Cys Glu Asn Gln Ala Thr Ile Ser Ser Lys Leu Gln Thr Cys Cys Asp
    290                 295                 300

Lys Pro Leu Leu Lys Lys Ala His Cys Leu Ser Glu Val Glu His Asp
305                 310                 315                 320

Thr Met Pro Ala Asp Leu Pro Ala Ile Ala Ala Asp Phe Val Glu Asp
                325                 330                 335

Gln Glu Val Cys Lys Asn Tyr Ala Glu Ala Lys Asp Val Phe Leu Gly
            340                 345                 350

Thr Phe Leu Tyr Glu Tyr Ser Arg Arg His Pro Asp Tyr Ser Val Ser
        355                 360                 365

Leu Leu Leu Arg Leu Ala Lys Lys Tyr Glu Ala Thr Leu Glu Lys Cys
    370                 375                 380

Cys Ala Glu Ala Asn Pro Pro Ala Cys Tyr Gly Thr Val Leu Ala Glu
385                 390                 395                 400

Phe Gln Pro Leu Val Glu Glu Pro Lys Asn Leu Val Lys Thr Asn Cys
                405                 410                 415

Asp Leu Tyr Glu Lys Leu Gly Glu Tyr Gly Phe Gln Asn Ala Ile Leu
            420                 425                 430

Val Arg Tyr Thr Gln Lys Ala Pro Gln Val Ser Thr Pro Thr Leu Val
        435                 440                 445

Glu Ala Ala Arg Asn Leu Gly Arg Val Gly Thr Lys Cys Cys Thr Leu
    450                 455                 460

Pro Glu Asp Gln Arg Leu Pro Cys Val Glu Asp Tyr Leu Ser Ala Ile
465                 470                 475                 480

Leu Asn Arg Val Cys Leu Leu His Glu Lys Thr Pro Val Ser Glu His
                485                 490                 495

Val Thr Lys Cys Cys Ser Gly Ser Leu Val Glu Arg Arg Pro Cys Phe
            500                 505                 510

Ser Ala Leu Thr Val Asp Glu Thr Tyr Val Pro Lys Glu Phe Lys Ala
        515                 520                 525

Glu Thr Phe Thr Phe His Ser Asp Ile Cys Thr Leu Pro Glu Lys Glu
    530                 535                 540

Lys Gln Ile Lys Lys Gln Thr Ala Leu Ala Glu Leu Val Lys His Lys
545                 550                 555                 560

Pro Lys Ala Thr Ala Glu Gln Leu Lys Thr Val Met Asp Asp Phe Ala
                565                 570                 575

Gln Phe Leu Asp Thr Cys Cys Lys Ala Ala Asp Lys Asp Thr Cys Phe
            580                 585                 590

Ser Thr Glu Gly Pro Asn Leu Val Thr Arg Cys Lys Asp Ala Leu Ala

595                 600                 605

<210>  8
<211>  608
<212>  PRT
<213>  Oryctolagus cuniculus

<400>  8

Met Lys Trp Val Thr Phe Ile Ser Leu Leu Phe Leu Phe Ser Ser Ala
1               5                   10                  15

Tyr Ser Arg Gly Val Phe Arg Arg Glu Ala His Lys Ser Glu Ile Ala
            20                  25                  30

His Arg Phe Asn Asp Val Gly Glu Glu His Phe Ile Gly Leu Val Leu
        35                  40                  45

Ile Thr Phe Ser Gln Tyr Leu Gln Lys Cys Pro Tyr Glu Glu His Ala
    50                  55                  60

Lys Leu Val Lys Glu Val Thr Asp Leu Ala Lys Ala Cys Val Ala Asp
65                  70                  75                  80

Glu Ser Ala Ala Asn Cys Asp Lys Ser Leu His Asp Ile Phe Gly Asp
                85                  90                  95

Lys Ile Cys Ala Leu Pro Ser Leu Arg Asp Thr Tyr Gly Asp Val Ala
            100                 105                 110

Asp Cys Cys Glu Lys Lys Glu Pro Glu Arg Asn Glu Cys Phe Leu His
        115                 120                 125

His Lys Asp Asp Lys Pro Asp Leu Pro Pro Phe Ala Arg Pro Glu Ala
    130                 135                 140

Asp Val Leu Cys Lys Ala Phe His Asp Asp Glu Lys Ala Phe Phe Gly
145                 150                 155                 160

His Tyr Leu Tyr Glu Val Ala Arg Arg His Pro Tyr Phe Tyr Ala Pro
                165                 170                 175

Glu Leu Leu Tyr Tyr Ala Gln Lys Tyr Lys Ala Ile Leu Thr Glu Cys
            180                 185                 190

Cys Glu Ala Ala Asp Lys Gly Ala Cys Leu Thr Pro Lys Leu Asp Ala
        195                 200                 205

Leu Glu Gly Lys Ser Leu Ile Ser Ala Ala Gln Glu Arg Leu Arg Cys

210                 215                 220

Ala Ser Ile Gln Lys Phe Gly Asp Arg Ala Tyr Lys Ala Trp Ala Leu
225                 230                 235                 240

Val Arg Leu Ser Gln Arg Phe Pro Lys Ala Asp Phe Thr Asp Ile Ser
                245                 250                 255

Lys Ile Val Thr Asp Leu Thr Lys Val His Lys Glu Cys Cys His Gly
            260                 265                 270

Asp Leu Leu Glu Cys Ala Asp Asp Arg Ala Asp Leu Ala Lys Tyr Met
        275                 280                 285

Cys Glu His Gln Glu Thr Ile Ser Ser His Leu Lys Glu Cys Cys Asp
    290                 295                 300

Lys Pro Ile Leu Glu Lys Ala His Cys Ile Tyr Gly Leu His Asn Asp
305                 310                 315                 320

Glu Thr Pro Ala Gly Leu Pro Ala Val Ala Glu Glu Phe Val Glu Asp
                325                 330                 335

Lys Asp Val Cys Lys Asn Tyr Glu Glu Ala Lys Asp Leu Phe Leu Gly
            340                 345                 350

Lys Phe Leu Tyr Glu Tyr Ser Arg Arg His Pro Asp Tyr Ser Val Val
        355                 360                 365

Leu Leu Leu Arg Leu Gly Lys Ala Tyr Glu Ala Thr Leu Lys Lys Cys
    370                 375                 380

Cys Ala Thr Asp Asp Pro His Ala Cys Tyr Ala Lys Val Leu Asp Glu
385                 390                 395                 400

Phe Gln Pro Leu Val Asp Glu Pro Lys Asn Leu Val Lys Gln Asn Cys
                405                 410                 415

Glu Leu Tyr Glu Gln Leu Gly Asp Tyr Asn Phe Gln Asn Ala Leu Leu
            420                 425                 430

Val Arg Tyr Thr Lys Lys Val Pro Gln Val Ser Thr Pro Thr Leu Val
        435                 440                 445

Glu Ile Ser Arg Ser Leu Gly Lys Val Gly Ser Lys Cys Cys Lys His
    450                 455                 460

Pro Glu Ala Glu Arg Leu Pro Cys Val Glu Asp Tyr Leu Ser Val Val
465                 470                 475                 480

Leu Asn Arg Leu Cys Val Leu His Glu Lys Thr Pro Val Ser Glu Lys
                485                 490                 495

Val Thr Lys Cys Cys Ser Glu Ser Leu Val Asp Arg Arg Pro Cys Phe
            500                 505                 510

Ser Ala Leu Gly Pro Asp Glu Thr Tyr Val Pro Lys Glu Phe Asn Ala
        515                 520                 525

Glu Thr Phe Thr Phe His Ala Asp Ile Cys Thr Leu Pro Glu Thr Glu
    530                 535                 540

Arg Lys Ile Lys Lys Gln Thr Ala Leu Val Glu Leu Val Lys His Lys
545                 550                 555                 560

Pro His Ala Thr Asn Asp Gln Leu Lys Thr Val Val Gly Glu Phe Thr
                565                 570                 575

Ala Leu Leu Asp Lys Cys Cys Ser Ala Glu Asp Lys Glu Ala Cys Phe
            580                 585                 590

Ala Val Glu Gly Pro Lys Leu Val Glu Ser Ser Lys Ala Thr Leu Gly
        595                 600                 605

<210>  9
<211>  365
<212>  PRT
<213>  Homo sapiens

<400>  9

Met Gly Val Pro Arg Pro Gln Pro Trp Ala Leu Gly Leu Leu Leu Phe
1               5                   10                  15

Leu Leu Pro Gly Ser Leu Gly Ala Glu Ser His Leu Ser Leu Leu Tyr
            20                  25                  30

His Leu Thr Ala Val Ser Ser Pro Ala Pro Gly Thr Pro Ala Phe Trp
        35                  40                  45

Val Ser Gly Trp Leu Gly Pro Gln Gln Tyr Leu Ser Tyr Asn Ser Leu
    50                  55                  60

Arg Gly Glu Ala Glu Pro Cys Gly Ala Trp Val Trp Glu Asn Gln Val
65                  70                  75                  80

Ser Trp Tyr Trp Glu Lys Glu Thr Thr Asp Leu Arg Ile Lys Glu Lys
                85                  90                  95

Leu Phe Leu Glu Ala Phe Lys Ala Leu Gly Gly Lys Gly Pro Tyr Thr
            100                 105                 110

Leu Gln Gly Leu Leu Gly Cys Glu Leu Gly Pro Asp Asn Thr Ser Val
        115                 120                 125

Pro Thr Ala Lys Phe Ala Leu Asn Gly Glu Glu Phe Met Asn Phe Asp
    130                 135                 140

Leu Lys Gln Gly Thr Trp Gly Gly Asp Trp Pro Glu Ala Leu Ala Ile
145                 150                 155                 160

Ser Gln Arg Trp Gln Gln Gln Asp Lys Ala Ala Asn Lys Glu Leu Thr
                165                 170                 175

Phe Leu Leu Phe Ser Cys Pro His Arg Leu Arg Glu His Leu Glu Arg
            180                 185                 190

Gly Arg Gly Asn Leu Glu Trp Lys Glu Pro Pro Ser Met Arg Leu Lys
        195                 200                 205

Ala Arg Pro Ser Ser Pro Gly Phe Ser Val Leu Thr Cys Ser Ala Phe
    210                 215                 220

Ser Phe Tyr Pro Pro Glu Leu Gln Leu Arg Phe Leu Arg Asn Gly Leu
225                 230                 235                 240

Ala Ala Gly Thr Gly Gln Gly Asp Phe Gly Pro Asn Ser Asp Gly Ser
                245                 250                 255

Phe His Ala Ser Ser Ser Leu Thr Val Lys Ser Gly Asp Glu His His
            260                 265                 270

Tyr Cys Cys Ile Val Gln His Ala Gly Leu Ala Gln Pro Leu Arg Val
        275                 280                 285

Glu Leu Glu Ser Pro Ala Lys Ser Ser Val Leu Val Val Gly Ile Val
    290                 295                 300

Ile Gly Val Leu Leu Leu Thr Ala Ala Ala Val Gly Gly Ala Leu Leu
305                 310                 315                 320

Trp Arg Arg Met Arg Ser Gly Leu Pro Ala Pro Trp Ile Ser Leu Arg
                325                 330                 335

Gly Asp Asp Thr Gly Val Leu Leu Pro Thr Pro Gly Glu Ala Gln Asp
            340                 345                 350

Ala Asp Leu Lys Asp Val Asn Val Ile Pro Ala Thr Ala
        355                 360                 365

<210>  10
<211>  365
<212>  PRT
<213>  Macaca mulatta

<400>  10

Met Arg Val Pro Arg Pro Gln Pro Trp Ala Leu Gly Leu Leu Leu Phe
1               5                   10                  15

Leu Leu Pro Gly Ser Leu Gly Ala Glu Ser His Leu Ser Leu Leu Tyr
            20                  25                  30

His Leu Thr Ala Val Ser Ser Pro Ala Pro Gly Thr Pro Ala Phe Trp
        35                  40                  45

Val Ser Gly Trp Leu Gly Pro Gln Gln Tyr Leu Ser Tyr Asp Ser Leu
    50                  55                  60

Arg Gly Gln Ala Glu Pro Cys Gly Ala Trp Val Trp Glu Asn Gln Val
65                  70                  75                  80

Ser Trp Tyr Trp Glu Lys Glu Thr Thr Asp Leu Arg Ile Lys Glu Lys
                85                  90                  95

Leu Phe Leu Glu Ala Phe Lys Ala Leu Gly Gly Lys Gly Pro Tyr Thr
            100                 105                 110

Leu Gln Gly Leu Leu Gly Cys Glu Leu Ser Pro Asp Asn Thr Ser Val
        115                 120                 125

Pro Thr Ala Lys Phe Ala Leu Asn Gly Glu Glu Phe Met Asn Phe Asp
    130                 135                 140

Leu Lys Gln Gly Thr Trp Gly Gly Asp Trp Pro Glu Ala Leu Ala Ile
145                 150                 155                 160

Ser Gln Arg Trp Gln Gln Gln Asp Lys Ala Ala Asn Lys Glu Leu Thr
                165                 170                 175

Phe Leu Leu Phe Ser Cys Pro His Arg Leu Arg Glu His Leu Glu Arg
            180                 185                 190

Gly Arg Gly Asn Leu Glu Trp Lys Glu Pro Pro Ser Met Arg Leu Lys
        195                 200                 205

Ala Arg Pro Gly Asn Pro Gly Phe Ser Val Leu Thr Cys Ser Ala Phe
    210                 215                 220

Ser Phe Tyr Pro Pro Glu Leu Gln Leu Arg Phe Leu Arg Asn Gly Met
225                 230                 235                 240

Ala Ala Gly Thr Gly Gln Gly Asp Phe Gly Pro Asn Ser Asp Gly Ser
                245                 250                 255

Phe His Ala Ser Ser Ser Leu Thr Val Lys Ser Gly Asp Glu His His
            260                 265                 270

Tyr Cys Cys Ile Val Gln His Ala Gly Leu Ala Gln Pro Leu Arg Val
        275                 280                 285

Glu Leu Glu Thr Pro Ala Lys Ser Ser Val Leu Val Val Gly Ile Val
    290                 295                 300

Ile Gly Val Leu Leu Leu Thr Ala Ala Ala Val Gly Gly Ala Leu Leu
305                 310                 315                 320

Trp Gly Arg Met Arg Ser Gly Leu Pro Ala Pro Trp Ile Ser Leu Arg
                325                 330                 335

Gly Asp Asp Thr Gly Ser Leu Leu Pro Thr Pro Gly Glu Ala Gln Asp
            340                 345                 350

Ala Asp Ser Lys Asp Ile Asn Val Ile Pro Ala Thr Ala
        355                 360                 365

<210>  11
<211>  358
<212>  PRT
<213>  Sus scrofa

<400>  11

Met Arg Ser Pro Gly Ser Ala Leu Val Ala Arg Leu Leu Leu Leu Leu
1               5                   10                  15

Leu Pro Gly Thr Pro Arg Ala Asp Asn His Arg Ser Leu Leu Tyr His
            20                  25                  30

Leu Thr Ala Val Ser Ala Pro Thr Pro Gly Ala Pro Ala Phe Trp Val
        35                  40                  45

Ser Gly Trp Leu Gly Pro Gln Gln Tyr Leu Ser Tyr Asn Asn Leu Arg
    50                  55                  60

Ala Gln Ala Glu Pro Tyr Gly Ala Trp Val Trp Glu Ser Gln Val Ser
65                  70                  75                  80

Trp Tyr Trp Glu Lys Glu Thr Ala Asp Leu Arg Asn Lys Gln Lys Leu
                85                  90                  95

Phe Leu Glu Ala Leu Lys Thr Leu Glu Glu Gly Gly Pro Phe Thr Leu
            100                 105                 110

Gln Gly Leu Leu Gly Cys Glu Leu Gly Pro Asp Asn Val Ser Val Pro
        115                 120                 125

Val Ala Thr Phe Ala Leu Asn Gly Glu Glu Phe Met Lys Phe Asp Thr
    130                 135                 140

Lys Leu Gly Thr Trp Asp Gly Glu Trp Pro Glu Ala Arg Thr Ile Gly
145                 150                 155                 160

Ser Lys Trp Met Gln Glu Pro Asp Ala Val Asn Lys Glu Lys Thr Phe
                165                 170                 175

Leu Leu Tyr Ser Cys Pro His Arg Leu Leu Gly His Leu Glu Arg Gly
            180                 185                 190

Arg Gly Asn Leu Glu Trp Lys Glu Pro Pro Ser Met Arg Met Lys Ala
        195                 200                 205

Arg Pro Gly Thr Ala Pro Gly Phe Ser Val Leu Thr Cys Ile Ala Phe
    210                 215                 220

Ser Phe Tyr Pro Pro Glu Leu Gln Leu Arg Phe Leu Arg Asn Gly Leu
225                 230                 235                 240

Ala Ala Gly Ser Gly Glu Ser Asp Ile Gly Pro Asn Gly Asp Gly Ser
                245                 250                 255

Phe His Ala Trp Ser Ser Leu Thr Val Lys Ser Gly Asp Glu His His
            260                 265                 270

Tyr Cys Cys Val Val Gln His Ala Gly Leu Ala Gln Pro Leu Thr Val
        275                 280                 285

Glu Leu Glu Ser Pro Ala Lys Ser Ser Met Pro Val Val Gly Ile Met
    290                 295                 300

Val Gly Phe Leu Leu Leu Leu Ile Val Ala Gly Gly Gly Ala Leu Leu
305                 310                 315                 320

Trp Arg Arg Met Thr Lys Gly Leu Pro Ala Pro Trp Ile Ser Phe His
                325                 330                 335

Gly Gly Arg Arg Arg Gly Pro Pro Ala His Pro Arg Pro Gly Gln Gly
            340                 345                 350

Cys Leu Asn Leu Arg Ile
        355

<210>  12
<211>  354
<212>  PRT
<213>  Canis lupus familiaris

<400>  12

Met Gly Val Pro Arg Pro Arg Ser Trp Gly Leu Gly Phe Leu Leu Phe
1               5                   10                  15

Leu Leu Pro Thr Leu Arg Ala Asp Ser His Leu Ser Leu Leu Tyr His
            20                  25                  30

Leu Thr Ala Val Ser Ala Pro Pro Pro Gly Thr Pro Ala Phe Trp Ala
        35                  40                  45

Ser Gly Trp Leu Gly Pro Gln Gln Tyr Leu Ser Tyr Asn Asn Leu Arg
    50                  55                  60

Ala Gln Ala Glu Pro Tyr Gly Ala Trp Val Trp Glu Asn Gln Val Ser
65                  70                  75                  80

Trp Tyr Trp Glu Lys Glu Thr Thr Asp Leu Arg Thr Lys Glu Gly Leu
                85                  90                  95

Phe Leu Glu Ala Leu Lys Ala Leu Gly Asp Gly Gly Pro Tyr Thr Leu
            100                 105                 110

Gln Gly Leu Leu Gly Cys Glu Leu Gly Pro Asp Asn Thr Ser Val Pro
        115                 120                 125

Val Ala Lys Phe Ala Leu Asn Gly Glu Asp Phe Met Thr Phe Asp Pro
    130                 135                 140

Lys Leu Gly Thr Trp Asn Gly Asp Trp Pro Glu Thr Glu Thr Val Ser
145                 150                 155                 160

Lys Arg Trp Met Gln Gln Ala Gly Ala Val Ser Lys Glu Arg Thr Phe
                165                 170                 175

Leu Leu Tyr Ser Cys Pro Gln Arg Leu Leu Gly His Leu Glu Arg Gly
            180                 185                 190

Arg Gly Asn Leu Glu Trp Lys Glu Pro Pro Ser Met Arg Leu Lys Ala
        195                 200                 205

Arg Pro Gly Ser Pro Gly Phe Ser Val Leu Thr Cys Ser Ala Phe Ser
    210                 215                 220

Phe Tyr Pro Pro Glu Leu Gln Leu Arg Phe Leu Arg Asn Gly Leu Ala
225                 230                 235                 240

Ala Gly Ser Gly Glu Gly Asp Phe Gly Pro Asn Gly Asp Gly Ser Phe
                245                 250                 255

His Ala Trp Ser Ser Leu Thr Val Lys Ser Gly Asp Glu His His Tyr
            260                 265                 270

Arg Cys Leu Val Gln His Ala Gly Leu Pro Gln Pro Leu Thr Val Glu
        275                 280                 285

Leu Glu Ser Pro Ala Lys Ser Ser Val Pro Val Val Gly Ile Val Ile
    290                 295                 300

Gly Phe Leu Leu Leu Thr Ala Val Ala Val Gly Gly Ala Leu Leu Trp
305                 310                 315                 320

Arg Arg Met Arg Lys Gly Leu Pro Ala Pro Trp Met Ser Leu Arg Gly
                325                 330                 335

Asp Asp Val Gly Ala Leu Leu Pro Thr Pro Gly Val Pro Lys Asp Ala
            340                 345                 350

Asp Ser

<210>  13
<211>  365
<212>  PRT
<213>  Mus musculus

<400>  13

Met Gly Met Pro Leu Pro Trp Ala Leu Ser Leu Leu Leu Val Leu Leu
1               5                   10                  15

Pro Gln Thr Trp Gly Ser Glu Thr Arg Pro Pro Leu Met Tyr His Leu
            20                  25                  30

Thr Ala Val Ser Asn Pro Ser Thr Gly Leu Pro Ser Phe Trp Ala Thr
        35                  40                  45

Gly Trp Leu Gly Pro Gln Gln Tyr Leu Thr Tyr Asn Ser Leu Arg Gln
    50                  55                  60

Glu Ala Asp Pro Cys Gly Ala Trp Met Trp Glu Asn Gln Val Ser Trp
65                  70                  75                  80

Tyr Trp Glu Lys Glu Thr Thr Asp Leu Lys Ser Lys Glu Gln Leu Phe
                85                  90                  95

Leu Glu Ala Leu Lys Thr Leu Glu Lys Ile Leu Asn Gly Thr Tyr Thr
            100                 105                 110

Leu Gln Gly Leu Leu Gly Cys Glu Leu Ala Ser Asp Asn Ser Ser Val
        115                 120                 125

Pro Thr Ala Val Phe Ala Leu Asn Gly Glu Glu Phe Met Lys Phe Asn
    130                 135                 140

Pro Arg Ile Gly Asn Trp Thr Gly Glu Trp Pro Glu Thr Glu Ile Val
145                 150                 155                 160

Ala Asn Leu Trp Met Lys Gln Pro Asp Ala Ala Arg Lys Glu Ser Glu
                165                 170                 175

Phe Leu Leu Asn Ser Cys Pro Glu Arg Leu Leu Gly His Leu Glu Arg
            180                 185                 190

Gly Arg Arg Asn Leu Glu Trp Lys Glu Pro Pro Ser Met Arg Leu Lys
        195                 200                 205

Ala Arg Pro Gly Asn Ser Gly Ser Ser Val Leu Thr Cys Ala Ala Phe
    210                 215                 220

Ser Phe Tyr Pro Pro Glu Leu Lys Phe Arg Phe Leu Arg Asn Gly Leu
225                 230                 235                 240

Ala Ser Gly Ser Gly Asn Cys Ser Thr Gly Pro Asn Gly Asp Gly Ser
                245                 250                 255

Phe His Ala Trp Ser Leu Leu Glu Val Lys Arg Gly Asp Glu His His

260                 265                 270

Tyr Gln Cys Gln Val Glu His Glu Gly Leu Ala Gln Pro Leu Thr Val
        275                 280                 285

Asp Leu Asp Ser Ser Ala Arg Ser Ser Val Pro Val Val Gly Ile Val
    290                 295                 300

Leu Gly Leu Leu Leu Val Val Val Ala Ile Ala Gly Gly Val Leu Leu
305                 310                 315                 320

Trp Gly Arg Met Arg Ser Gly Leu Pro Ala Pro Trp Leu Ser Leu Ser
                325                 330                 335

Gly Asp Asp Ser Gly Asp Leu Leu Pro Gly Gly Asn Leu Pro Pro Glu
            340                 345                 350

Ala Glu Pro Gln Gly Ala Asn Ala Phe Pro Ala Thr Ser
        355                 360                 365

<210>  14
<211>  366
<212>  PRT
<213>  Rattus norvegicus

<400>  14

Met Gly Met Ser Gln Pro Gly Val Leu Leu Ser Leu Leu Leu Val Leu
1               5                   10                  15

Leu Pro Gln Thr Trp Gly Ala Glu Pro Arg Leu Pro Leu Met Tyr His
            20                  25                  30

Leu Ala Ala Val Ser Asp Leu Ser Thr Gly Leu Pro Ser Phe Trp Ala
        35                  40                  45

Thr Gly Trp Leu Gly Ala Gln Gln Tyr Leu Thr Tyr Asn Asn Leu Arg
    50                  55                  60

Gln Glu Ala Asp Pro Cys Gly Ala Trp Ile Trp Glu Asn Gln Val Ser
65                  70                  75                  80

Trp Tyr Trp Glu Lys Glu Thr Thr Asp Leu Lys Ser Lys Glu Gln Leu
                85                  90                  95

Phe Leu Glu Ala Ile Arg Thr Leu Glu Asn Gln Ile Asn Gly Thr Phe
            100                 105                 110

Thr Leu Gln Gly Leu Leu Gly Cys Glu Leu Ala Pro Asp Asn Ser Ser

115                 120                 125

Leu Pro Thr Ala Val Phe Ala Leu Asn Gly Glu Glu Phe Met Arg Phe
    130                 135                 140

Asn Pro Arg Thr Gly Asn Trp Ser Gly Glu Trp Pro Glu Thr Asp Ile
145                 150                 155                 160

Val Gly Asn Leu Trp Met Lys Gln Pro Glu Ala Ala Arg Lys Glu Ser
                165                 170                 175

Glu Phe Leu Leu Thr Ser Cys Pro Glu Arg Leu Leu Gly His Leu Glu
            180                 185                 190

Arg Gly Arg Gln Asn Leu Glu Trp Lys Glu Pro Pro Ser Met Arg Leu
        195                 200                 205

Lys Ala Arg Pro Gly Asn Ser Gly Ser Ser Val Leu Thr Cys Ala Ala
    210                 215                 220

Phe Ser Phe Tyr Pro Pro Glu Leu Lys Phe Arg Phe Leu Arg Asn Gly
225                 230                 235                 240

Leu Ala Ser Gly Ser Gly Asn Cys Ser Thr Gly Pro Asn Gly Asp Gly
                245                 250                 255

Ser Phe His Ala Trp Ser Leu Leu Glu Val Lys Arg Gly Asp Glu His
            260                 265                 270

His Tyr Gln Cys Gln Val Glu His Glu Gly Leu Ala Gln Pro Leu Thr
        275                 280                 285

Val Asp Leu Asp Ser Pro Ala Arg Ser Ser Val Pro Val Val Gly Ile
    290                 295                 300

Ile Leu Gly Leu Leu Leu Val Val Val Ala Ile Ala Gly Gly Val Leu
305                 310                 315                 320

Leu Trp Asn Arg Met Arg Ser Gly Leu Pro Ala Pro Trp Leu Ser Leu
                325                 330                 335

Ser Gly Asp Asp Ser Gly Asp Leu Leu Pro Gly Gly Asn Leu Pro Pro
            340                 345                 350

Glu Ala Glu Pro Gln Gly Val Asn Ala Phe Pro Ala Thr Ser
        355                 360                 365

<210>  15
<211>  119
<212>  PRT
<213>  Homo sapiens

<400>  15

Met Ser Arg Ser Val Ala Leu Ala Val Leu Ala Leu Leu Ser Leu Ser
1               5                   10                  15

Gly Leu Glu Ala Ile Gln Arg Thr Pro Lys Ile Gln Val Tyr Ser Arg
            20                  25                  30

His Pro Ala Glu Asn Gly Lys Ser Asn Phe Leu Asn Cys Tyr Val Ser
        35                  40                  45

Gly Phe His Pro Ser Asp Ile Glu Val Asp Leu Leu Lys Asn Gly Glu
    50                  55                  60

Arg Ile Glu Lys Val Glu His Ser Asp Leu Ser Phe Ser Lys Asp Trp
65                  70                  75                  80

Ser Phe Tyr Leu Leu Tyr Tyr Thr Glu Phe Thr Pro Thr Glu Lys Asp
                85                  90                  95

Glu Tyr Ala Cys Arg Val Asn His Val Thr Leu Ser Gln Pro Lys Ile
            100                 105                 110

Val Lys Trp Asp Arg Asp Met
        115


<210>  16
<211>  342
<212>  PRT
<213>  Homo sapiens

<400>  16

Ala Glu Ser His Leu Ser Leu Leu Tyr His Leu Thr Ala Val Ser Ser
1               5                   10                  15

Pro Ala Pro Gly Thr Pro Ala Phe Trp Val Ser Gly Trp Leu Gly Pro
            20                  25                  30

Gln Gln Tyr Leu Ser Tyr Asn Ser Leu Arg Gly Glu Ala Glu Pro Cys
        35                  40                  45

Gly Ala Trp Val Trp Glu Asn Gln Val Ser Trp Tyr Trp Glu Lys Glu
    50                  55                  60

Thr Thr Asp Leu Arg Ile Lys Glu Lys Leu Phe Leu Glu Ala Phe Lys

65                  70                  75                  80

Ala Leu Gly Gly Lys Gly Pro Tyr Thr Leu Gln Gly Leu Leu Gly Cys
                85                  90                  95

Glu Leu Gly Pro Asp Asn Thr Ser Val Pro Thr Ala Lys Phe Ala Leu
            100                 105                 110

Asn Gly Glu Glu Phe Met Asn Phe Asp Leu Lys Gln Gly Thr Trp Gly
        115                 120                 125

Gly Asp Trp Pro Glu Ala Leu Ala Ile Ser Gln Arg Trp Gln Gln Gln
    130                 135                 140

Asp Lys Ala Ala Asn Lys Glu Leu Thr Phe Leu Leu Phe Ser Cys Pro
145                 150                 155                 160

His Arg Leu Arg Glu His Leu Glu Arg Gly Arg Gly Asn Leu Glu Trp
                165                 170                 175

Lys Glu Pro Pro Ser Met Arg Leu Lys Ala Arg Pro Ser Ser Pro Gly
            180                 185                 190

Phe Ser Val Leu Thr Cys Ser Ala Phe Ser Phe Tyr Pro Pro Glu Leu
        195                 200                 205

Gln Leu Arg Phe Leu Arg Asn Gly Leu Ala Ala Gly Thr Gly Gln Gly
    210                 215                 220

Asp Phe Gly Pro Asn Ser Asp Gly Ser Phe His Ala Ser Ser Ser Leu
225                 230                 235                 240

Thr Val Lys Ser Gly Asp Glu His His Tyr Cys Cys Ile Val Gln His
                245                 250                 255

Ala Gly Leu Ala Gln Pro Leu Arg Val Glu Leu Glu Ser Pro Ala Lys
            260                 265                 270

Ser Ser Val Leu Val Val Gly Ile Val Ile Gly Val Leu Leu Leu Thr
        275                 280                 285

Ala Ala Ala Val Gly Gly Ala Leu Leu Trp Arg Arg Met Arg Ser Gly
    290                 295                 300

Leu Pro Ala Pro Trp Ile Ser Leu Arg Gly Asp Asp Thr Gly Val Leu
305                 310                 315                 320

Leu Pro Thr Pro Gly Glu Ala Gln Asp Ala Asp Leu Lys Asp Val Asn
                325                 330                 335

Val Ile Pro Ala Thr Ala
            340

<210>  17
<211>  354
<212>  PRT
<213>  Cavia porcellus

<400>  17

Met Gly Glu Pro Gly Pro Pro Arg Pro Trp Ala Leu Gly Leu Leu Leu
1               5                   10                  15

Leu Leu Leu Pro Gln Thr Trp Gly Ala Glu Asn His Leu Ser Leu Leu
            20                  25                  30

Tyr His Leu Thr Ala Val Ser Ser Pro Ala Thr Gly Ser Pro Ala Phe
        35                  40                  45

Trp Val Ser Gly Trp Leu Gly Pro Gln Gln Tyr Leu Ser Tyr Ser Ser
    50                  55                  60

Leu Arg Arg Glu Ala Glu Pro Cys Gly Ala Trp Val Trp Glu Thr Gln
65                  70                  75                  80

Val Ser Trp Tyr Trp Glu Met Glu Thr Thr Asp Leu Lys Asn Lys Glu
                85                  90                  95

Ser Leu Phe Leu Glu Ala Leu Lys Ala Leu Gly Gly Lys Gly Pro Tyr
            100                 105                 110

Thr Leu Gln Gly Ile Leu Gly Cys Glu Leu Ser Pro Asp Asn Ser Ser
        115                 120                 125

Val Pro Thr Ala Leu Phe Ala Leu Asn Gly Glu Asp Phe Met Glu Phe
    130                 135                 140

His Pro Glu Asn Gly Ser Trp Thr Gly Asp Trp Pro Glu Ala Leu Ser
145                 150                 155                 160

Ile Ser Lys Gln Trp Glu Lys Lys Ala Glu Val Val Ser Lys Glu Lys
                165                 170                 175

Thr Phe Leu Leu Ser Ser Cys Pro Gln Arg Leu Leu Asp His Leu Glu
            180                 185                 190

Arg Ser Arg Arg Asn Leu Glu Trp Lys Glu Pro Pro Ser Met Arg Leu
        195                 200                 205

Lys Ala Arg Pro Gly Asp Ala Gly Phe Ser Val Leu Thr Cys Ser Ala
    210                 215                 220

Phe Ser Phe Tyr Pro Pro Glu Leu Gln Leu Arg Phe Leu His Asn Gly
225                 230                 235                 240

Leu Ala Ala Gly Thr Gly Glu Gly Gly Phe Gly Pro Asn Gly Asp Gly
                245                 250                 255

Ser Phe His Ala Trp Ser Ser Leu Thr Val Gln Arg Gly Asp Glu His
            260                 265                 270

Asn Tyr Arg Cys Leu Val Gln His Ala Gly Leu Pro Gln Pro Leu Ser
        275                 280                 285

Val Glu Leu Glu Ser Pro Ala Gly Ser Ser Val Pro Ile Val Gly Ile
    290                 295                 300

Val Leu Gly Phe Leu Leu Leu Ile Ala Thr Ala Ala Gly Gly Val Leu
305                 310                 315                 320

Leu Trp Arg Arg Met Arg Ser Gly Leu Pro Ala Pro Trp Ile Ser Leu
                325                 330                 335

Arg Gly Asp Asp Val Gly Ser Leu Leu Pro Gly Pro Leu Gln Glu Pro
            340                 345                 350

Asp Ser

<210>  18
<211>  608
<212>  PRT
<213>  Cavia porcellus

<400>  18

Met Lys Trp Val Thr Phe Ile Ser Leu Leu Phe Leu Phe Ser Ser Val
1               5                   10                  15

Tyr Ser Arg Gly Val Phe Arg Arg Glu Ala His Lys Ser Glu Ile Ala
            20                  25                  30

His Arg Phe Asn Asp Leu Gly Glu Gly His Phe Lys Gly Leu Val Leu
        35                  40                  45

Ile Thr Leu Ser Gln His Leu Gln Lys Ser Pro Phe Glu Glu His Val
    50                  55                  60

Lys Leu Val Asn Glu Val Thr Asp Phe Ala Lys Ala Cys Val Ala Asp
65                  70                  75                  80

Glu Ser Ala Gln Asn Cys Gly Lys Ala Ile Ala Thr Leu Phe Gly Asp
                85                  90                  95

Lys Val Cys Ala Ile Pro Ser Leu Arg Glu Thr Tyr Gly Glu Leu Ala
            100                 105                 110

Asp Cys Cys Ala Lys Glu Asp Pro Asp Arg Val Glu Cys Phe Leu Gln
        115                 120                 125

His Lys Asp Asp Asn Pro Asn Leu Pro Pro Phe Glu Arg Pro Glu Pro
    130                 135                 140

Glu Ala Leu Cys Thr Ala Phe Lys Glu Asn Asn Asp Arg Phe Ile Gly
145                 150                 155                 160

His Tyr Leu Tyr Glu Val Ser Arg Arg His Pro Tyr Phe Tyr Ala Pro
                165                 170                 175

Glu Leu Leu Tyr Tyr Ala Glu Lys Tyr Lys Asn Ala Leu Thr Glu Cys
            180                 185                 190

Cys Glu Ala Ala Asp Lys Ala Ala Cys Leu Thr Pro Lys Leu Asp Ala
        195                 200                 205

Ile Lys Glu Lys Ala Leu Val Ser Ser Ala Gln Gln Arg Leu Lys Cys
    210                 215                 220

Ala Ser Leu Gln Lys Phe Gly Glu Arg Ala Phe Lys Ala Trp Ser Val
225                 230                 235                 240

Ala Arg Leu Ser Gln Lys Phe Pro Lys Ala Glu Phe Ala Glu Ile Ser
                245                 250                 255

Thr Ile Val Thr Ser Leu Thr Lys Val Thr Lys Glu Cys Cys His Gly
            260                 265                 270

Asp Leu Leu Glu Cys Ala Asp Asp Arg Gln Glu Leu Ala Lys Tyr Met
        275                 280                 285

Cys Glu His Gln Asp Ser Ile Ser Ser Lys Leu Lys Glu Cys Cys Val
    290                 295                 300

Lys Pro Thr Leu Gln Lys Ala His Cys Ile Leu Glu Ile Gln Arg Asp
305                 310                 315                 320

Glu Leu Pro Thr Glu Leu Pro Asp Leu Ala Val Asp Phe Val Glu Asp
                325                 330                 335

Lys Glu Val Cys Lys Asn Phe Ala Glu Ala Lys Asp Val Phe Leu Gly
            340                 345                 350

Thr Phe Leu Tyr Glu Tyr Ser Arg Arg His Pro Glu Tyr Ser Ile Gly
        355                 360                 365

Met Leu Leu Arg Ile Ala Lys Gly Tyr Glu Ala Lys Leu Glu Lys Cys
    370                 375                 380

Cys Ala Glu Ala Asp Pro His Ala Cys Tyr Ala Lys Val Phe Asp Glu
385                 390                 395                 400

Leu Gln Pro Leu Ile Asp Glu Pro Lys Lys Leu Val Gln Gln Asn Cys
                405                 410                 415

Glu Leu Phe Asp Lys Leu Gly Glu Tyr Gly Phe Gln Asn Ala Leu Ala
            420                 425                 430

Val Arg Tyr Thr Gln Lys Ala Pro Gln Val Ser Thr Pro Thr Leu Val
        435                 440                 445

Glu Tyr Ala Arg Lys Leu Gly Ser Val Gly Thr Lys Cys Cys Ser Leu
    450                 455                 460

Pro Glu Thr Glu Arg Leu Ser Cys Thr Glu Asn Tyr Leu Ala Leu Ile
465                 470                 475                 480

Leu Asn Arg Leu Cys Ile Leu His Glu Lys Thr Pro Val Ser Glu Arg
                485                 490                 495

Val Thr Lys Cys Cys Thr Glu Ser Leu Val Asn Arg Arg Pro Cys Phe
            500                 505                 510

Ser Ala Leu His Val Asp Glu Thr Tyr Val Pro Lys Pro Phe His Ala
        515                 520                 525

Asp Ser Phe Thr Phe His Ala Asp Ile Cys Thr Leu Pro Glu Lys Glu
    530                 535                 540

Lys Gln Val Lys Lys Gln Met Ala Leu Val Glu Leu Val Lys His Lys
545                 550                 555                 560

Pro Lys Ala Ser Glu Glu Gln Met Lys Thr Val Met Gly Asp Phe Ala
                565                 570                 575

Ala Phe Leu Lys Lys Cys Cys Asp Ala Asp Asn Lys Glu Ala Cys Phe
            580                 585                 590

Thr Glu Asp Gly Pro Lys Leu Val Ala Lys Cys Gln Ala Thr Leu Ala
        595                 600                 605

<210>  19
<211>  608
<212>  PRT
<213>  Macaca fascicularis

<400>  19

Met Lys Trp Val Thr Phe Ile Ser Leu Leu Phe Leu Phe Ser Ser Ala
1               5                   10                  15

Tyr Ser Arg Gly Val Phe Arg Arg Asp Thr His Lys Ser Glu Val Ala
            20                  25                  30

His Arg Phe Lys Asp Leu Gly Glu Glu His Phe Lys Gly Leu Val Leu
        35                  40                  45

Val Ala Phe Ser Gln Tyr Leu Gln Gln Cys Pro Phe Glu Glu His Val
    50                  55                  60

Lys Leu Val Asn Glu Val Thr Glu Phe Ala Lys Thr Cys Val Ala Asp
65                  70                  75                  80

Glu Ser Ala Glu Asn Cys Asp Lys Ser Leu His Thr Leu Phe Gly Asp
                85                  90                  95

Lys Leu Cys Thr Val Ala Thr Leu Arg Glu Thr Tyr Gly Glu Met Ala
            100                 105                 110

Asp Cys Cys Ala Lys Gln Glu Pro Glu Arg Asn Glu Cys Phe Leu Gln
        115                 120                 125

His Lys Asp Asp Asn Pro Asn Leu Pro Pro Leu Val Arg Pro Glu Val
    130                 135                 140

Asp Val Met Cys Thr Ala Phe His Asp Asn Glu Ala Thr Phe Leu Lys
145                 150                 155                 160

Lys Tyr Leu Tyr Glu Val Ala Arg Arg His Pro Tyr Phe Tyr Ala Pro
                165                 170                 175

Glu Leu Leu Phe Phe Ala Ala Arg Tyr Lys Ala Ala Phe Ala Glu Cys
            180                 185                 190

Cys Gln Ala Ala Asp Lys Ala Ala Cys Leu Leu Pro Lys Leu Asp Glu
        195                 200                 205

Leu Arg Asp Glu Gly Lys Ala Ser Ser Ala Lys Gln Arg Leu Lys Cys
    210                 215                 220

Ala Ser Leu Gln Lys Phe Gly Asp Arg Ala Phe Lys Ala Trp Ala Val
225                 230                 235                 240

Ala Arg Leu Ser Gln Lys Phe Pro Lys Ala Glu Phe Ala Glu Val Ser
                245                 250                 255

Lys Leu Val Thr Asp Leu Thr Lys Val His Thr Glu Cys Cys His Gly
            260                 265                 270

Asp Leu Leu Glu Cys Ala Asp Asp Arg Ala Asp Leu Ala Lys Tyr Met
        275                 280                 285

Cys Glu Asn Gln Asp Ser Ile Ser Ser Lys Leu Lys Glu Cys Cys Asp
    290                 295                 300

Lys Pro Leu Leu Glu Lys Ser His Cys Leu Ala Glu Val Glu Asn Asp
305                 310                 315                 320

Glu Met Pro Ala Asp Leu Pro Ser Leu Ala Ala Asp Tyr Val Glu Ser
                325                 330                 335

Lys Asp Val Cys Lys Asn Tyr Ala Glu Ala Lys Asp Val Phe Leu Gly
            340                 345                 350

Met Phe Leu Tyr Glu Tyr Ala Arg Arg His Pro Asp Tyr Ser Val Met
        355                 360                 365

Leu Leu Leu Arg Leu Ala Lys Ala Tyr Glu Ala Thr Leu Glu Lys Cys
    370                 375                 380

Cys Ala Ala Ala Asp Pro His Glu Cys Tyr Ala Lys Val Phe Asp Glu
385                 390                 395                 400

Phe Gln Pro Leu Val Glu Glu Pro Gln Asn Leu Val Lys Gln Asn Cys
                405                 410                 415

Glu Leu Phe Glu Gln Leu Gly Glu Tyr Lys Phe Gln Asn Ala Leu Leu

420                 425                 430

Val Arg Tyr Thr Lys Lys Val Pro Gln Val Ser Thr Pro Thr Leu Val
        435                 440                 445

Glu Val Ser Arg Asn Leu Gly Lys Val Gly Ala Lys Cys Cys Lys Leu
    450                 455                 460

Pro Glu Ala Lys Arg Met Pro Cys Ala Glu Asp Tyr Leu Ser Val Val
465                 470                 475                 480

Leu Asn Arg Leu Cys Val Leu His Glu Lys Thr Pro Val Ser Glu Lys
                485                 490                 495

Val Thr Lys Cys Cys Thr Glu Ser Leu Val Asn Arg Arg Pro Cys Phe
            500                 505                 510

Ser Ala Leu Glu Leu Asp Glu Ala Tyr Val Pro Lys Ala Phe Asn Ala
        515                 520                 525

Glu Thr Phe Thr Phe His Ala Asp Met Cys Thr Leu Ser Glu Lys Glu
    530                 535                 540

Lys Gln Val Lys Lys Gln Thr Ala Leu Val Glu Leu Val Lys His Lys
545                 550                 555                 560

Pro Lys Ala Thr Lys Glu Gln Leu Lys Gly Val Met Asp Asn Phe Ala
                565                 570                 575

Ala Phe Val Glu Lys Cys Cys Lys Ala Asp Asp Lys Glu Ala Cys Phe
            580                 585                 590

Ala Glu Glu Gly Pro Lys Phe Val Ala Ala Ser Gln Ala Ala Leu Ala
        595                 600                 605

<210>  20
<211>  365
<212>  PRT
<213>  Macaca fascicularis

<400>  20

Met Arg Val Pro Arg Pro Gln Pro Trp Ala Leu Gly Leu Leu Leu Phe
1               5                   10                  15

Leu Leu Pro Gly Ser Leu Gly Ala Glu Ser His Leu Ser Leu Leu Tyr
            20                  25                  30

His Leu Thr Ala Val Ser Ser Pro Ala Pro Gly Thr Pro Ala Phe Trp

35                  40                  45

Val Ser Gly Trp Leu Gly Pro Gln Gln Tyr Leu Ser Tyr Asp Ser Leu
    50                  55                  60

Arg Gly Gln Ala Glu Pro Cys Gly Ala Trp Val Trp Glu Asn Gln Val
65                  70                  75                  80

Ser Trp Tyr Trp Glu Lys Glu Thr Thr Asp Leu Arg Ile Lys Glu Lys
                85                  90                  95

Leu Phe Leu Glu Ala Phe Lys Ala Leu Gly Gly Lys Gly Pro Tyr Thr
            100                 105                 110

Leu Gln Gly Leu Leu Gly Cys Glu Leu Ser Pro Asp Asn Thr Ser Val
        115                 120                 125

Pro Thr Ala Lys Phe Ala Leu Asn Gly Glu Glu Phe Met Asn Phe Asp
    130                 135                 140

Leu Lys Gln Gly Thr Trp Gly Gly Asp Trp Pro Glu Ala Leu Ala Ile
145                 150                 155                 160

Ser Gln Arg Trp Gln Gln Gln Asp Lys Ala Ala Asn Lys Glu Leu Thr
                165                 170                 175

Phe Leu Leu Phe Ser Cys Pro His Arg Leu Arg Glu His Leu Glu Arg
            180                 185                 190

Gly Arg Gly Asn Leu Glu Trp Lys Glu Pro Pro Ser Met Arg Leu Lys
        195                 200                 205

Ala Arg Pro Gly Asn Pro Gly Phe Ser Val Leu Thr Cys Ser Ala Phe
    210                 215                 220

Ser Phe Tyr Pro Pro Glu Leu Gln Leu Arg Phe Leu Arg Asn Gly Met
225                 230                 235                 240

Ala Ala Gly Thr Gly Gln Gly Asp Phe Gly Pro Asn Ser Asp Gly Ser
                245                 250                 255

Phe His Ala Ser Ser Ser Leu Thr Val Lys Ser Gly Asp Glu His His
            260                 265                 270

Tyr Cys Cys Ile Val Gln His Ala Gly Leu Ala Gln Pro Leu Arg Val
        275                 280                 285

Glu Leu Glu Thr Pro Ala Lys Ser Ser Val Leu Val Val Gly Ile Val
    290                 295                 300

Ile Gly Val Leu Leu Leu Thr Ala Ala Ala Val Gly Gly Ala Leu Leu
305                 310                 315                 320

Trp Gly Arg Met Arg Ser Gly Leu Pro Ala Pro Trp Ile Ser Leu Arg
                325                 330                 335

Gly Asp Asp Thr Gly Ser Leu Leu Pro Thr Pro Gly Glu Ala Gln Asp
            340                 345                 350

Ala Asp Ser Lys Asp Ile Asn Val Ile Pro Ala Thr Ala
        355                 360                 365

<210>  21
<211>  119
<212>  PRT
<213>  Macaca fascicularis

<400>  21

Met Ser Arg Ser Val Ala Leu Ala Val Leu Ala Leu Leu Ser Leu Ser
1               5                   10                  15

Gly Leu Glu Ala Ile Gln Arg Thr Pro Lys Ile Gln Val Tyr Ser Arg
            20                  25                  30

His Pro Pro Glu Asn Gly Lys Pro Asn Phe Leu Asn Cys Tyr Val Ser
        35                  40                  45

Gly Phe His Pro Ser Asp Ile Glu Val Asp Leu Leu Lys Asn Gly Glu
    50                  55                  60

Lys Met Gly Lys Val Glu His Ser Asp Leu Ser Phe Ser Lys Asp Trp
65                  70                  75                  80

Ser Phe Tyr Leu Leu Tyr Tyr Thr Glu Phe Thr Pro Asn Glu Lys Asp
                85                  90                  95

Glu Tyr Ala Cys Arg Val Asn His Val Thr Leu Ser Gly Pro Arg Thr
            100                 105                 110

Val Lys Trp Asp Arg Asp Met
        115

<210>  22
<211>  118
<212>  PRT
<213>  Sus scrofa

<400>  22

Met Ala Pro Leu Val Ala Leu Val Leu Leu Gly Leu Leu Ser Leu Ser
1               5                   10                  15

Gly Leu Asp Ala Val Ala Arg Pro Pro Lys Val Gln Val Tyr Ser Arg
            20                  25                  30

His Pro Ala Glu Asn Gly Lys Pro Asn Tyr Leu Asn Cys Tyr Val Ser
        35                  40                  45

Gly Phe His Pro Pro Gln Ile Glu Ile Asp Leu Leu Lys Asn Gly Glu
    50                  55                  60

Lys Met Asn Ala Glu Gln Ser Asp Leu Ser Phe Ser Lys Asp Trp Ser
65                  70                  75                  80

Phe Tyr Leu Leu Val His Thr Glu Phe Thr Pro Asn Ala Val Asp Gln
                85                  90                  95

Tyr Ser Cys Arg Val Lys His Val Thr Leu Asp Lys Pro Lys Ile Val
            100                 105                 110

Lys Trp Asp Arg Asp His
        115


<210>  23
<211>  125
<212>  PRT
<213>  Cavia porcellus

<400>  23

Met Ala Arg Ser Val Ala Leu Val Ser Leu Ala Leu Leu Ala Val Leu
1               5                   10                  15

Ala Leu Leu Ser Leu Pro Ala Val Asp Ala Val Leu His Ala Pro Arg
            20                  25                  30

Val Gln Val Tyr Ser Arg His Pro Ala Glu Asn Gly Lys Gln Asn Phe
        35                  40                  45

Ile Asn Cys Tyr Val Ser Gly Phe His Pro Pro Gln Ile Glu Val Glu
    50                  55                  60

Leu Leu Lys Asn Gly Lys Lys Ile Asp Asn Val Glu Met Ser Asp Leu
65                  70                  75                  80

Ser Phe Ser Lys Asp Trp Thr Phe Tyr Leu Leu Val His Ala Ala Phe

85                  90                  95

Thr Pro Asn Asp Ser Asp Glu Tyr Ser Cys Arg Val Ser His Ile Thr
            100                 105                 110

Leu Ser Glu Pro Lys Ile Val Lys Trp Asp Pro Asn Lys
        115                 120                 125

## Description

### Reference to sequence listing

This application contains a Sequence Listing in computer readable form. The computer readable form is incorporated herein by reference.

### FIELD OF THE INVENTION

The present invention relates to a method of assessing one or more (several) pharmacokinetic properties of a variant of human serum albumin (including modified albumin such as genetic fusions, conjugates and associates) using a non-primate animal species. The present invention also relates to animal models which are particularly suitable for assessing one or more (several) pharmacokinetic properties of human serum albumin variants or modifications thereof.

### BACKGROUND OF THE INVENTION

Albumin is a protein naturally found in the blood plasma of mammals where it is the most abundant protein. It has important roles in maintaining the desired osmotic pressure of the blood and also in transport of various substances in the blood stream.

The neonatal Fc receptor (FcRn) "Brambell" is a bifunctional molecule that contributes to maintaining a high level of immunoglobulins of isotype G (IgGs) and albumin in serum in mammals such as human beings. FcRn has been found to salvage albumin and IgG from intracellular degradation by a pH dependent mechanism thus prolonging its serum half-life. The plasma half-life of wild type human serum albumin (HSA) has been found to be approximately 19 days.

The use of albumin in drug delivery is well described. Therapeutic active agents may for example be conjugated to albumin (WO 2000/69902) or therapeutic active polypeptides may be fused genetically to albumin and expressed as chimeric proteins (WO 2001/79271 and WO 2003/59934) or small acidic or hydrophobic therapeutic active agents may associate reversibly to albumin (Kragh-Hansen et al, 2002, Biol. Pharm. Bull. 25, 695 and WO 2000/71079). Reversible binding to albumin can also be achieved for pharmaceutically beneficial compounds, which have little or no albumin binding properties by associating such compounds to a moiety having albumin-binding properties (Kurtzhals et al, 1997, J. Pharm. Sci. 86: 1365, and WO 2010/065950). Kratz, 2008, J. Controlled Release 132, 171-183 provides a review of all these technologies. Benefits of using albumin for drug delivery are longer half-life and/or controlled release of a therapeutic agent and/or targeting to selective tissues or organs.

A number of natural albumin variants have been described. Otagiri et al, 2009, Biol. Pharm. Bull. 32(4), 527-534, discloses 77 known albumin variants, 22 are found in domain I, 30 in domain II and 25 are found in domain III. A number of other natural variants have been identified and some of these have been analyzed for FcRn binding (Andersen et al (2010), Clinical Biochemistry 43, 367-372; Galliano et al (1993) Biochim. Biophys. Acta 1225, 27-32; Minchiotti et al (1987) Biochim. Biophys. Acta 916, 411-418; Takahashi et al (1987) Proc. Natl. Acad. Sci. USA 84, 4413-4417; Carlson et al (1992). Proc. Nat. Acad. Sci. USA 89, 8225- 8229; (Peach, R. J. and Brennan, S. O., (1991) Biochim Biophys Acta.1097:49-54). The half-life of naturally occurring human albumin variants using a mouse model was described in Iwao, et. al. (2007) B.B.A. Proteins and Proteomics 1774, 1582-1590. Furthermore, a series of human made albumin variants with altered binding to the FcRn has been described in WO 2011/051489, WO2011/124718, WO 2012/059486, WO 2012/150319 WO 2011/103076, and WO 2012/112188, none of these publications disclose data on half-life measurements of albumin variant in animal models.

Animals are often used in preclinical development to predict the pharmacokinetics of therapeutic agents in humans prior to the first in man administration. To assist in these predictions animals of different sizes and weight are often used. Interspecies allometric scaling is based on the assumption that there are anatomical, physiological and biochemical similarities among animals which can be described by simple mathematical models. A number of animals species have successfully been used in interspecies allometric scaling including mouse, rat, guinea pig, rabbit, cynomolgus monkey, baboon, rhesus monkey, dog, pig and sheep (Mahmood I. (2004) New Drug Development, Regulatory Paradigms for Clinical Pharmacology and Biopharmaceutics, Edited by Chandrahas G . Sahajwalla, Informa Healthcare, pages 137-163, Print ISBN: 978-0-8247-5465-5). Pigs are an accepted model for small molecules (Hall C. et al. (2012) J. Pharma Sci. 101, 1221-1241) and proteins (Larsen M. O. and Rolin B. (2004) ILAR Journal 45, 303-313; Zheng Y. et al. (2012) mAbs 4, 243-255). Indeed the Göttingen minipig is gaining importance as a large animal model in pharmaceutical research due to its physiological and anatomical similarities to human and is increasingly replacing dog and non-human primate in preclinical studies (Suenderhauf C. and Parrott N. (2013) Pharm. Res. 30, 1-15).

The only non-primate animal model currently available to test proof of concept that the improved FcRn binding HSA variants will have an extended half-life is the human FcRn transgenic mice (homozygous knock-out (KO) of the mouse gene and a heterozygous knock-in (KI) of the human gene) (Roopenian et al (2003) J. Immunol. Vol 170, pp. 3528-3533). This model, however, has important limitations from the standpoint for measuring half-life of HSA since the mouse contains a high circulating concentration of mouse serum albumin that binds human FcRn with a 6 fold greater affinity than Wt HSA (Andersen J.T. (2010) J Biol. Chem. 12;285(7): 4826-36).

### BRIEF DESCRIPTION OF DRAWINGS

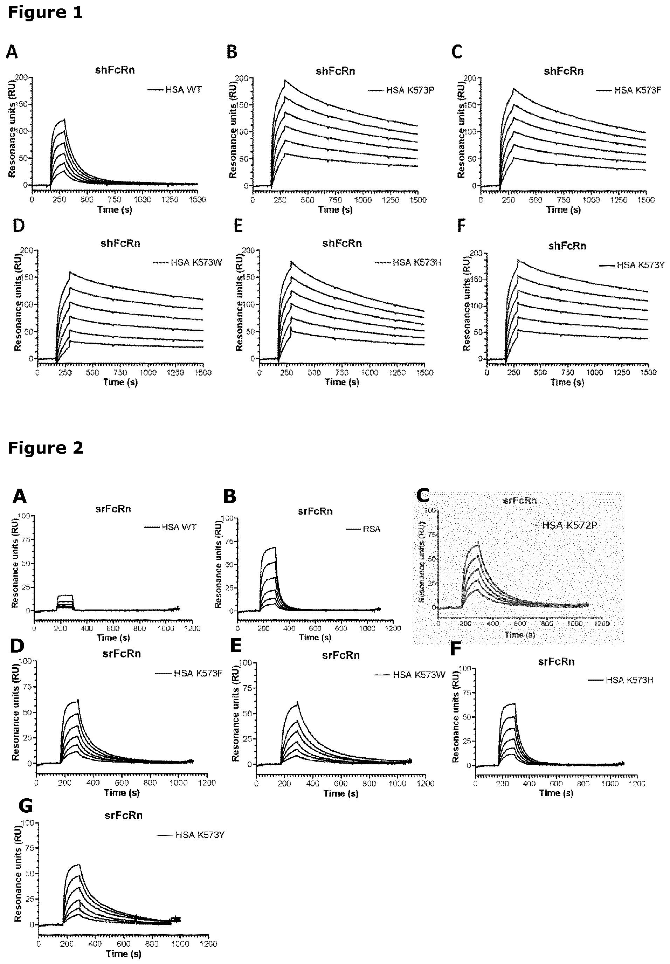

Figure 1 shows binding of albumin variants to shFcRn. Serial dilutions of each albumin variant (10 µM - 0.3 µM) were injected over immobilized shFcRn at pH 6.0. (A) HSA Wt, (B) HSA K573P, (C) HSA K573F, (D) HSA K573W, (E) HSA K573H and (F) HSA K573Y.
Figure 2 shows binding of albumin variants to soluble rat FcRn (srFcRn). Serial dilutions of each albumin variant (10 µM-0.3 µM) were injected over immobilized srFcRn at pH 6.0. (A) HSA Wt, (B) wild-type rat serum albumin (RSA), (C) HSA K573P, (D) HSA K573F, (E) HSA K573W, (F) HSA K573H and (G) HSA K573Y.

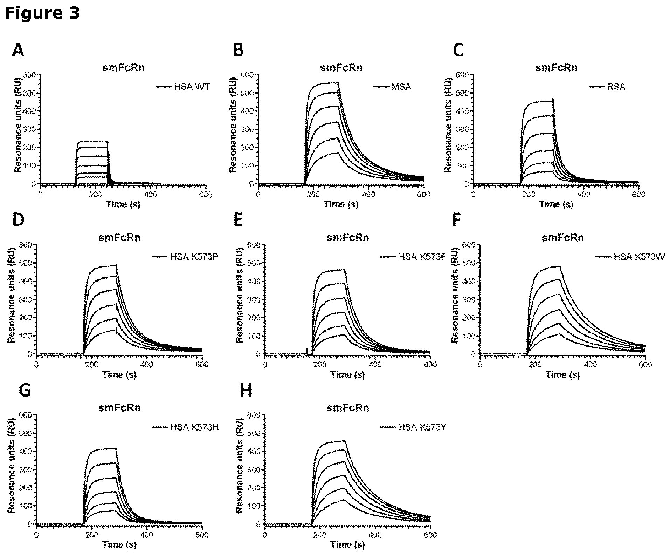

Figure 3 shows binding of albumin variants to soluble mouse serum albumin (smFcRn). Serial dilutions of each albumin variant (10 µM-0.3 µM) were injected over immobilized smFcRn at pH 6.0, with the exception of Wt HSA (100 µM-3.0 µM). (A) HSA Wt, (B) MSA , (C) RSA , (D) HSA K573P, (E) HSA K573F, (F) HSA K573W, (G) HSA K573H and (H) HSA K573Y.

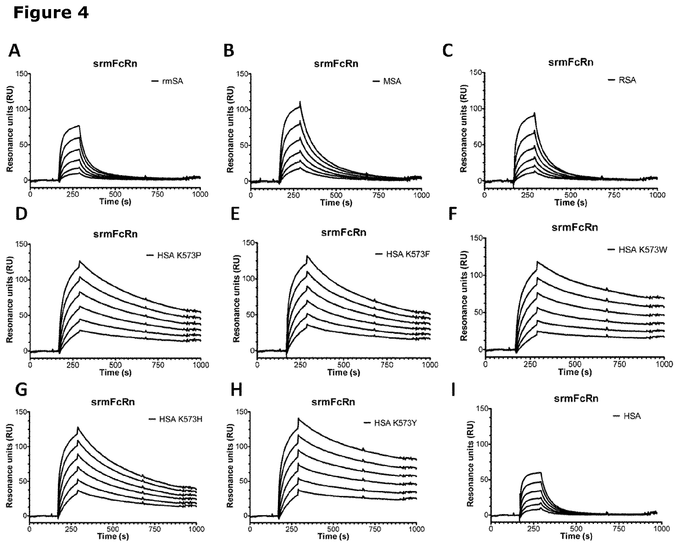

Figure 4 shows binding of albumin variants to soluble rhesus macaque FcRn (srmFcRn). Serial dilutions of each albumin variant (10 µM-0.3 µM) were injected over immobilized srmFcRn at pH 6.0. (A) wild-type rhesus macaque serum albumin (rmSA ), (B) MSA , (C) RSA , (D) HSA K573P, (E) HSA K573F, (F) HSA K573W, (G) HSA K573H, (H) HSA K573Y and (I) HSA Wt.

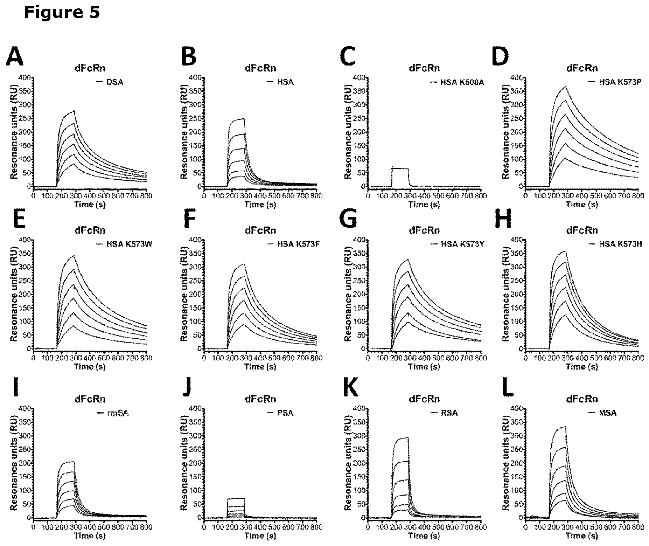

Figure 5 shows binding of albumin variants to soluble dog FcRn (dFcRn). Serial dilutions of albumin variants injected over immobilized soluble dog FcRn at pH 6.0. (A) wild-type dog serum albumin (DSA ) (b) HSA , (C) HSA K500A (D) HSA K573P, (E) HSA K573W, (F) HSA K573F, (G) HSA K573Y and (H) HSA K573H (I) rmSA , (J) wild-type pig serum albumin (PSA ), (K) RSA , (L) MSA.

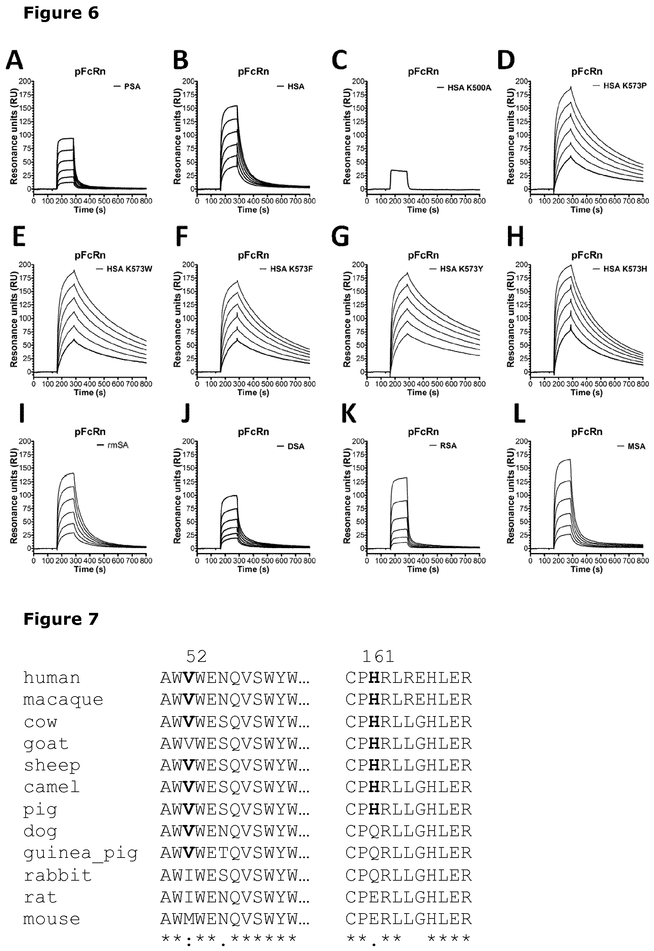

Figure 6 shows binding of albumin variants to soluble pig FcRn (pFcRn). Serial dilutions of albumin variants injected over immobilized soluble pig FcRn at pH 6.0. (A) PSA (b) HSA , (C) HSA K500A (D) HSA K573P, (E) HSA K573W, (F) HSA K573F, (G) HSA K573Y and (H) HSA K573H (I) rmSA , (J) DSA , (K) RSA , (L) MSA.
Figure 7 shows selected areas of a ClustalW alignment of FcRn HC from (human, macaque, cow, goat, sheep, camel, pig, dog, guinea pig, rabbit, rat and mouse). Amino acid residues that are identical in all sequences are indicated by (*), conserved substitutions are indicated by (:), and semi-conservative substitutions are indicated by (.). V52 and H161 of SEQ ID NO: 16 are highlighted in bold. The alignment parameters were Opening and end gap penalty 10, extending and separation gap penalty 0.5 using scoring matrix Blosum.

### DETAILED DESCRIPTION OF THE INVENTION

The present invention provides a method for assessing one or more (several) pharmacokinetic properties of a variant human serum albumin (HSA) compared to wild type HSA. The pharmacokinetic properties of molecules where the Wt HSA and variant HSA is modified by fusion, conjugation or association with a partner such as therapeutic agents, vaccines or diagnostic agents are of particular interest.

An advantage of the present invention is that it reduces the need for primate animal models for screening of albumin containing drugs by providing a non-primate animal model that can produce profiles of one or more (several) pharmacokinetic properties that can reasonably be extrapolated to indicate what the human pharmacokinetic profiles are likely to look like.

The animal model used in the method of the present invention is characterized in that the binding affinity of wild type HSA to the native FcRn of said animal is the same as or higher than the binding affinity of the native albumin of said animal.

### Definitions

The term "binding affinity" generally refers to the strength of the sum total of the non-covalent interactions between a single binding site of a molecule (e.g., IgG or albumin) and its binding partner (e.g., an antigen or FcRn). Unless indicated otherwise, as used herein, "binding affinity" refers to intrinsic binding affinity which reflects a 1: 1 interaction between members of a binding pair (e.g., albumin and FcRn). The affinity of a molecule (X) for its partner (Y) can generally be represented by the equilibrium dissociation constant (K_{D}), which is calculated as the ratio k_{off}/kₒₙ (k_{d}/kₐ). Binding affinity can be measured by methods known in the art. A preferred method is surface plasmon resonance (SPR) for example using a Biacore (GE Healthcare) instrument as exemplified herein. The binding affinity of endogenous pairs of FcRn and albumin (e.g. HSA to hFcRn, dog albumin to dog FcRn and so forth) generally range from 0.2 to 3.2 micro Molar)

The term "modified" or "modification" in relation to albumin means to change the albumin by adding or deleting molecules unrelated to the amino acid sequence of the albumin, e.g. removing fatty acids or adding a partner molecule. The albumin can in in particular be modified by conjugation, fusion or association of a partner. Changes to the amino acid sequence of the albumin (e.g. SEQ ID NO: 2) is termed variants and are not considered modifications.

The term "conjugated", "conjugate", or "conjugation" in relation to albumin refers to Wt HSA or a variant HSA or a fragment thereof which is conjugated to a conjugation partner such as a beneficial agent, *e.g*. a therapeutic agent and/or diagnostic agent. Conjugation can be made to the N-terminal and/or C-terminal of the albumin, but can alternatively or in addition be made to one or more (several) suitable amino acid positions within the albumin. In particular cysteine residues which are not involved in disulfide bonds are suitable for conjugation. WO 2010/092135 describes a variant albumin with additional cysteine residues suitable for conjugation. Techniques for conjugating a conjugation partner to an albumin or fragment thereof are known in the art. WO 2009/019314 discloses examples of techniques suitable for conjugating a conjugation partner, e.g. a therapeutic agent, to a polypeptide which techniques can also be applied to the present invention. Furthermore, page 37 to 44 of WO 2009/019314 (hereby incorporated by reference) discloses examples of compounds and moieties that may be conjugated to transferrin and these compounds and moieties may also be conjugated to an albumin variant of the present invention.

The term "fused" or "fusion" in relation to albumin refers to Wt HSA or a variant HSA or a fragment thereof which is genetically fused to a fusion partner such as a beneficial agent *e.g*. a therapeutic polypeptide and/or diagnostic polypeptide. Fusions are normally either made at the N-terminal or C-terminal of the albumin, or sometimes at both ends. Fusions can in principal alternatively or in addition be made within the albumin molecule, in that case it is preferred to locate the fusion partner between domains of albumin. For example, a fusion partner may be located between Domain I and Domain II and/or between Domain II and Domain III. Teachings relating to fusions of albumin or a fragment thereof are known in the art and the skilled person will appreciate that such teachings can also be applied to the present invention. Table 1 of WO 2001/79271, Table 1 (page 11) of WO 2001/79258, Table 1 (page 11) of WO 2001/79442, Table 1 (page 12) of WO 2001/79443, Table 1 (page 11) of WO 2001/79443, Table 1 of WO 2003/060071, Table 1 of WO 2003/59934, Table 1 of WO 2005/003296, Table 1 of WO 2007/021494 and Table 1 of WO 2009/058322 (all tables are hereby incorporated by reference) contain examples of fusion partners, *e.g*. therapeutic polypeptides, that may be fused to albumin or fragments thereof, and these examples apply also to the present invention.

The term "associated", "associate", or "association" in relation to albumin refers to a composition comprising Wt HSA or variant HSA or a fragment thereof and an association partner, such as a therapeutic agent and/or diagnostic agent, bound or associated to the albumin or fragment thereof by non-covalent binding. An example of such an associate is an albumin and a lipid associated to the albumin by a hydrophobic interaction. Such associates are known in the art and they may be prepared using well known techniques. Molecules which are suitable for association with albumin are known in the art, preferably they are acidic, lipophilic and/or have electronegative features. Examples of such molecules are given in Table 1 of Kragh-Hansen et al, 2002, Biol. Pharm. Bull. 25, 695 (hereby incorporated by reference). Furthermore, WO 2000/71079 describes the association of albumin with paclitaxel and paclitaxel is included in the present invention.

The term "native" in relation to albumin and FcRn refers to the albumin or FcRn proteins that are genetically expressed in a specific animal. The native albumin in a mouse is normally the endogenous mouse serum albumin corresponding to UniProt accession number P07724. FcRn comprises a FcRn heavy chain (HC) and a beta2 microglobulin (beta2m). The native FcRn in a mouse is normally the endogenous mouse FcRn HC corresponding to UniProt accession number Q61559 and mouse beta2mwith UniProt accession number Q91Z73. A native albumin or FcRn may however be a transgenic gene which is integrated into the genome of the animal in a stable manner and where the corresponding gene of the animal has been knocked out. An example is the transgenic mouse where human FcRn HC has been integrated into the genome of the mouse and the mouse FcRn HC has been knocked out (Roopenian et al (2003) J. Immunol. Vol 170, pp. 3528-3533). In such an animal the human FcRn HC would be considered to be a part of the native FcRn of the transgenic animal. The native FcRn may also be an FcRn variant, either naturally occurring or a variant produced by human intervention. An example of such a variant is a mouse FcRn (SEQ ID NO: 13) with one or more of the following mutations M73V and E184H.

The term "wild-type" (Wt) in relation to albumin or FcRn means an albumin or FcRn having the same amino acid sequence as the albumin or FcRn naturally found in an animal or in a human (the endogenous gene sequence of the animal or human). It is understood that Wt albumin or Wt FcRn is without genetic alterations produced by human intervention for example by gene knock-out/knock-in as in the production of transgenic animals. SEQ ID NO: 2 is a mature Wt albumin from *Homo sapiens.* More Wt albumins and Wt FcRn molecules are listed in Tables 1 and 3.

The term "sequence identity" describes the relatedness between two amino acid sequences or between two nucleotide sequences. For the purposes of the present invention, the sequence identity between two amino acid sequences is determined using the Needleman-Wunsch algorithm (Needleman and Wunsch, 1970, J. Mol. Biol. 48: 443-453) as implemented in the Needle program of the EMBOSS package (EMBOSS: The European Molecular Biology Open Software Suite, Rice et al., 2000, Trends Genet. 16: 276-277), preferably version 5.0.0 or later. The parameters used are gap open penalty of 10, gap extension penalty of 0.5, and the EBLOSUM62 (EMBOSS version of BLOSUM62) substitution matrix. The output of Needle labeled "longest identity" (obtained using the -nobrief option) is used as the percent identity and is calculated as follows: (Identical Residues x 100)/(Length of Alignment - Total Number of Gaps in Alignment).

The term "therapeutic agent", "therapeutic compound", "therapeutic molecule" or "drug" is used interchangeably and refers to a chemical compound, a mixture of chemical compounds, or a biological macromolecule (*e.g*. a peptide, protein, lipid, nucleic acid (e.g. DNA or RNA), virus) or a biological macromolecule in association with a chemical compound. Therapeutic agents include agents that can either prevent, improve or cure a medical condition. The therapeutic agent may be purified, substantially purified or partially purified. An "agent", according to the present invention, also includes a radiation therapy agent and vaccines.

The term "HSA variant" or "variant HSA" means a polypeptide derived from a human serum albumin comprising an alteration, *i.e*., a substitution, insertion, and/or deletion, at one or more (several) positions. A substitution means a replacement of an amino acid occupying a position with a different amino acid; a deletion means removal of an amino acid occupying a position; and an insertion means adding 1-3 amino acids adjacent to an amino acid occupying a position. The variant may also be a functional fragment of HSA. Fragments may consist of one uninterrupted sequence derived from albumin or may comprise two or more sequences derived from different parts of the albumin. The fragments according to the invention have a size of more than approximately 100 amino acid residues, preferably more than 150 amino acid residues, more preferred more than 200 amino acid residues, more preferred more than 300 amino acid residues, even more preferred more than 400 amino acid residues and most preferred more than 500 amino acid residues. In a preferred embodiment a fragment corresponds to one or more (several) of the albumin domains. Preferred albumin domains of the invention are HSA domain I consisting of amino acid residues 1 to 194 ± 1 to 15 amino acids of SEQ ID NO: 2; HSA domain II consisting of amino acid residues 192 to 387 ± 1 to 15 amino acids of SEQ ID NO: 2 and HSA domain III consisting of amino acid residues 381 to 585 ± 1 to 15 amino acids of SEQ ID NO: 2 or a combination of one or more (several) of these domains, e.g. domain I and II, domain II and III or domain I and III fused together. The altered polypeptide (variant) can be obtained through human intervention by alternation of the polynucleotide sequence encoding the HSA. The variant albumin is preferably at least 70%, preferably at least 75%, more preferably at least 80%, more preferably at least 85%, even more preferably at least 90%, most preferably at least 95%, at least 96%, at least 97%, at least 98%, at least 99%, at least 99.5% or at least 99.8% identical to SEQ ID NO: 2 and maintains at least one of the major properties of HSA. Generally, variants or fragments of HSA will have at least 10% (preferably at least 50%, 60%, 70%, 80%, 90% or 95%) of HSA ligand binding activity (for example bilirubin-binding) and at least 50% (preferably at least 70%, 80%, 90% or 95%) of HSA's oncotic activity, weight for weight. Oncotic activity, also known as colloid osmotic pressure, of albumin, albumin variants or fragments of albumin may be determined by the method described by Hoefs, J.C. (1992) Hepatology 16:396-403. Bilirubin binding may be measured by fluorescence enhancement at 527 nm relative to HSA. Bilirubin (1.0mg) is dissolved in 50microL of 1 M NaOH and diluted to 1.0mL with demineralised water. The bilirubin stock is diluted in 100mM Tris-HCl pH8.5, 1mM EDTA to give 0.6nmol of bilirubin/mL in a fluorometer cuvette. Fluorescence is measured by excitation at 448nm and emission at 527nm (10nm slit widths) during titration with HSA over a range of HSA:bilirubin ratios from 0 to 5 mol:mol. The variant may possess altered binding to FcRn when compared to the HSA. The variant polypeptide sequence is preferably one which is not found in nature.

The term "regulatory sequences" means all components (e.g. nucleic acid sequences) necessary for the expression of a polynucleotide inserted into an animal. Each regulatory sequence may be native (i.e. from the same gene) or foreign (i.e. from a different gene) to the polynucleotide encoding the transgenic polypeptide. Such regulatory sequences include, but are not limited to, a leader, polyadenylation sequence, propeptide sequence, promoter, signal peptide sequence and transcription terminator. At a minimum, the regulatory sequences include a promoter, and transcriptional and translational stop signals.

The term "operably linked" means a configuration in which a regulatory sequence is placed at an appropriate position relative to the transgenic sequence of a polynucleotide such that the regulatory sequence directs the expression of the transgenic sequence.

A number of therapeutic proteins fused to Wt albumin have entered clinical development. Some examples are interferon-alpha fused to Wt albumin, GCSF fused to Wt albumin, GLP-1 fused to Wt albumin and Factor IX fused to Wt albumin. When testing the half-life of these compounds in an animal model the test will compare the therapeutic protein alone against the therapeutic protein fused to the albumin, e.g. GLP1 and GLP1-albumin. In such a case it will be fairly easy to see a change in half-life between the molecules solely due to the difference in renal filtration irrespective of what animal model is used.

However, if it is desired to investigate the difference in one or more (several) pharmacokinetic properties of Wt albumin and albumin variants where the change in half-life will not be due to size difference and consequent renal filtration, it is important to have an animal model that will allow identification of such differences.

Albumins have been characterized from many species including human, pig, mouse, rat, rabbit and goat and they share a high degree of sequence and structural homology (see Table 1). Human serum albumin (HSA) is a well characterized polypeptide of 585 amino acids with a molecular mass of 67 kDa. Many of albumins characteristics are summarized in Peters, T., Jr. (1996) All about Albumin: Biochemistry, Genetics and Medical, Applications pp10, Academic Press, Inc., Orlando (ISBN 0-12-552110-3).

**Table 1. A non-exclusive list of wild type albumins from various species**

| **Common Name** | **Species** | **SwissProt or GenBank Accession No** | **% Identity to SEQ ID NO: 2*** | **Residues of mature sequence** |
|---|---|---|---|---|
| Human | *Homo sapiens* | P02768.2 | 100.0 | 25-609 |
| Chimpanzee | *Pan troglodytes* | XP_517233 (predicted sequence) | 98.8 | 25-609 |
| Sumatran Orangutan | *Pongo abelii* | Q5NVH5.2 | 98.5 | 25-609 |
| Macaque (Rhesus Monkey) | *Macaca mulatta* | NP_001182578 | 93.3 | 25-608 |
| Crab-eating macaque (cynomolgus macaque) | *Macaca fascicularis* | A2V9Z4 | 93.3 | 25-608 |
| Cat | *Felis catus* | P49064.1 | 81.9 | 25-608 |
| Dog | *Canis lupus familiaris* | P49822.3 | 80.0 | 25-608 |
| Cow | *Bos taurus* | P02769.4 | 75.8 | 25-607 |
| Pig | *Sus scrofa* | P08835.2 | 75.1 | 25-607 |
| Sheep | *Ovis aries* | P14639.1 | 75.0 | 25-607 |
| Goat | *Capra hircus* | B3VHM9 | 74.8 | 1-583 |
| Rabbit | *Oryctolagus cuniculus* | P49065.2 SEQ ID NO: 8 | 74.3 | 25-608 |
| Rat | *Rattus norvegicus* | P02770.2 | 73.3 | 25-608 |
| Mouse | *Mus musculus* | P07724.3 | 72.3 | 25-608 |
| Guinea Pig | *Cavia porcellus* | Q6WDN9 | 72.1 | 25-608 |

| | | | | |
|---|---|---|---|---|
| * Sequence identity was calculated using the Needleman-Wunsch algorithm as implemented in the Needle program of EBLOSUM62 (EMBOSS suite of programs, version 6.1.0) using gap open penalty of 10, gap extension penalty of 0.5 and selecting the no brief option to obtain the longest identity. | | | | |

As can be seen from Table 1, pig albumin (75.1% identity to human albumin) has a similar degree of identity to human albumin as mouse albumin (72.3% identity to human albumin), rabbit albumin (74.3% identity to human albumin) or rat albumin (73.3% identity to human albumin), while the non-human primates chimpanzee (98.9% identity to human albumin), macaque (93.3% identity to human albumin) and orangutan (98.5% identity to human albumin), have a higher degree of identity to human albumin than pig albumin.

Human albumin is synthesized predominantly in the liver. Heptocyctes do not contain a large pool of stored intracellular albumin, rather the protein is rapidly secreted from the cell resulting in approximately 13-14g of albumin entering the intravascular space every day, equivalent to 3.7%/day of the total body albumin mass of 360g for a 70kg person. The normal human plasma albumin concentration is 42±3.5 g/L and with an average plasma volume of 2.5-3.0L for a 70kg person, the average intravascular albumin mass is 113-126g (∼120g). Intravascular albumin is constantly being exchanged at a rate of 4-5%/hr by transport across the endothelium with the 240g extravascular albumin pool, resulting in a total body albumin mass of 360g for a 70kg person. Extravascular albumin returns to the vascular compartment by drainage through the lymphatic system. Of the 240g extravascular albumin pool some 175g is in free exchange with the intravascular pool (total exchangeable pool = 295g) while a further 65g is not in free exchange. Approximately 80% of the total extravascular pool is equally divided between the muscle and the skin. A mass of albumin equivalent to that entering the intravascular space (13-14g) is catabolised from the intravascular space every day. The fractional degradation rate, 3.7% of the 120g intravascular pool/day, equates to a half-life (t1/2) of 19 days (Peters, T., Jr. (1996) All about Albumin: Biochemistry, Genetics and Medical, Applications pp10, Academic Press, Inc., Orlando (ISBN 0-12-552110-3); C.L. Anderson, et al (2006) Trends in Immuno. 27: 343-348; and J. Kimet al. (2007) Clinical Immuno. 122: 146-155). Albumin is a component of many secretions from the human body including milk, sweat, tears and saliva. Albumin is mainly lost from the circulation by degradation in the larger organs, such as the skin and the muscle which have the most extensive circulation and consequently a large pool of endothelial cells which line the vasculature.

The fate of an albumin molecule be it degradation, transport across or exchange between pools or compartments, salvage and recycling is controlled in large part by the interaction with albumin receptors gp18 and gp30, (Ghinea A. et al. (1988) J. Cell Biol. 107: 231-239; Schnitzer J. et al. (1992) J. Biol. Chem. 267: 24544-24553; Schnitzer J. et al. (1993) J. Biol. Chem. 268: 7562-7570), gp60 (Schnitzer J. et al. (1994) J. Biol. Chem. 269: 6072-6082; Minshall R. at al. (2002) Histochem. Cell. Biol. 117: 105-112; Malik A. B. (2009) J. Med. Sci. 2, 13-17; and Predescu D. and Palade G. E. (1993) Am. J. Physiol. 265, H725-H733) and FcRn (Anderson C. L. et al. (2006) Trends in Immuno. 7, 343-348; Roopenian D. C. and Akilesh, S. (2007), Nat. Rev. Immunol 7, 715-725, Baker K. et. al (2009) Semin Immunopathol. 31, 223-236; Andersen J. T. and Sandlie I. (2009) Drug Metab. Pharmacokinet. 24, 318-332 and Kuo T. T.et al. (2010) J. Clin. Immunol 30, 777-789). gp18 and gp30 are present in cultured fibroblasts, smooth muscle cells and endothelial cells; they are also distributed ubiquitously being found in heart, lung, muscle, kidney, fat, brain, adrenal, pancreas and liver. Damaged albumin (e.g. point mutations, truncations, glycosylation mutants, oxidation or even iodination) has a 1000-fold higher affinity for both gp18 and gp30 than native albumin. Damaged albumins, once internalized, are degraded, a process which can be inhibited by known inhibitors of lysosomal degradation as well as inhibited gp18 and gp30 mediated damaged albumin degradation. Therefore gp18 and gp30 resemble scavenger proteins and so may mediate the high affinity binding, endocytosis and degradation of damaged albumins, but not native albumins.

An analysis of urine from healthy subjects reveals trace amounts (<0.03g/L) of albumin, which is significantly less than the 3-6g of albumin which passes through the glomerulus every day even though human kidneys process blood containing 37kg of albumin daily (Peters, T., Jr. (1996) All about Albumin: Biochemistry, Genetics and Medical, Applications pp10, Academic Press, Inc., Orlando (ISBN 0-12-552110-3); Gekle M. (2005) Ann. Rev. Physiol. 67, 573-594). In healthy individuals less than 1% of the daily glomerular filtered albumin load appears in the urine.

In humans the long circulatory half-life of albumin is dependent upon functional interaction with FcRn and the nature of the glomerular filtration barrier which retains proteins of greater than ∼60kDa in the glomerular retentate while proteins smaller than ∼60kDa are filtered and appear to a progressively greater extent in the glomerular ultrafiltrate as the size of the protein decreases.

The circulatory half-life of albumin has been shown to be impacted to various degrees by glycation, glycosylation, oxidation, structural changes and point mutations within the albumin primary sequence, especially damage affecting hydrophobicity and net charge of the molecules reduces half-life (Nakajou et al. Biochim Biophys Acta. (2003) 1623, 88-97; (Iwao Y. et al. (2006) Biochim Biophys Acta. 1764, 743-749;Iwao Y et al. (2007) Biochim Biophys Acta. 1774, 1582-1590; Sheffield W. P. et al. (2000) Thrombosis Research 99, 613-621).

Given the importance of the interaction with FcRn it is not unexpected that damaged or variant albumins which do not interact with FcRn have reduced half-life, with the consequence that the plasma concentration of albumin is reduced, a condition known as analbuminaemia. Natural variants of albumin (Bartin, Bazzano, Venezia) which have truncations at the C-terminus of albumin all have reduced half-life and in the case of variants Bazzano and Venezia this is also associated with an increase in liver, kidney and spleen uptake. Further investigations have revealed that in the case of the Bartin variant that the reduced half-life was also associated with an absence of any pH dependent FcRn binding (Iwao Y. et al. (2009) Biochim Biophys Acta. 1794, 634-641; Andersen J. T. et al. (2010) Clin Biochem. 43, 367-372). It has yet to be established if many of the altered half-lives and organ uptake of damaged or variant albumins observed in vivo are in fact as the result of altered FcRn binding.

The circulatory half-lives of wild-type (Wt) albumin in various animals has been studied in vivo by a number of different techniques. Table 2, below, summarizes some of the published half-lives of albumin in different species.

**Table 2. Half-life of albumin in different species**

| **Animal** | **Albumin half-life (days)** | **Reference** |
|---|---|---|
| Mouse | 1.2 | Dixon et al. (1953) Exp. Biol. Med. 83, 287-288 |
| | 1 | Stevens et al. (1992) Fundam. Appl. Toxicol. 19, 336-342 |
| Rat | 2.0-2.5 | Sell S. (1974) Cancer Research 34, 1608-1611 |
| Rabbit | 5.7 ± 0.3 | Dixon et al. (1953) Exp. Biol. Med. 83, 287-288 |
| | 5.5 ± 0.11 | Hatton et al. (1993) J. Theor. Biol. 161, 481-490 |
| Dog | 8.2 ± 1.2 | Dixon et al. (1953) Exp. Biol. Med. 83, 287-288 |
| Pig | 7.4 to 9.5 | Dich & Nielsen (1963) Can. J. Comp. Med. Vet. Sci.27, 269-273 |
| Sheep | 14 to 28 | Campbell et al (1961) J. Physiol. 158 113 |
| Human | 19 | Peters, T., Jr. (1996) All About Albumin: Biochemistry, Genetics and Medical, Applications pp10, Academic Press, Inc., Orlando |
| | 15.0 ± 1.9 | Dixon et al. (1953) Exp. Biol. Med. 83, 287-288 |
| | 14 to 23 | Cohen et al (1961) Clin. Sci. 20 161 |
| | 12.7 to 18.2 | Beeken et al (1962) J. Clin. Invest 62 1312 |
| Cow | 20.7 ± 1.1 | Dixon et al. (1953) Exp. Biol. Med. 83, 287-288 |
| | 14 to 19 | Cornelius et al (1962) Amer. J. Vet. Res. 23 837 |

Many of the references from the 1960's have used I-131 labeled albumin to measure the half-life of albumin, a general challenge when using I-131 labeled albumin is variable degrees of denaturation incurred during the preparation of the protein and its radioisotopic labeling (Beeken et al (1962) J. Clin. Invest 62 1312). Generally it can be observed that the half-life of albumin increases with the size of the species

Albumin binds *in vivo* to its receptor, the neonatal Fc receptor (FcRn) "Brambell" and this interaction is known to be important for the plasma half-life of albumin in that it salvage albumin from intracellular degradation (Roopenian D. C. and Akilesh, S. (2007), Nat. Rev. Immunol 7, 715-725.). FcRn is a membrane bound protein, expressed in many cell and tissue types including vascular, renal (podocytes and proximal convoluted tubule (PCT)) and brain endoethelia; antigen presenting cells; gut, upper airway and alveolar epithelia. FcRn is a heterodimeric receptor consisting of a 46kDa MHC class-I-like transmembrane heavy chain (HC) that is non-covalently associated with a 12kDa (beta2m). FcRn only has affinity for albumin and IgG at acidic pH (below pH6.5). IgG and albumin:FcRn complexes, formed at the acidic pH of the endosome, are sorted into separate vesicles, thus diverting the molecules away from the default lysosomal degradation pathway. The albumin:FcRn complexes are recycled back to the plasma membrane surface where they encounter physiological pH and both albumin and IgG are released from FcRn which is then ready to rescue more albumin and IgG, thereby increasing the plasma half-life of albumin and IgG. Conversely internalized proteins which are not bound to FcRn are sorted for degradation in the lysosome.

The major FcRn binding site is localized within DIII (381-585), (Andersen et al (2010), Clinical Biochemistry 43,367-372). A model of the interaction of human albumin with human FcRn has been described. A number of key amino acids in albumin have been shown to be important in binding, notably histidines H464, H510 and H536 and lysine Lys500 (Andersen et al (2010), Nat. Commun. 3:610. DOI:10.1038/ ncomms1607). Data indicates that amino acids within DI (1-197) of albumin contribute to the interaction of albumin with FcRn. Importantly, albumin interacts with the FcRn HC and not the beta2munit (Andersen et al (2010), Clinical Biochemistry 43,367-372; (Andersen et al (2012) Nature Communications Vol. 3, pp. 610). Data indicates that IgG and albumin bind non-cooperatively to distinct sites on FcRn (Andersen et al. (2006), Eur. J. Immunol 36, 3044-3051; Chaudhury et al. (2006), Biochemistry 45, 4983-4990; (Anderson C. L. et al. (2006) Trends in Immuno. 7, 343-348; Roopenian D. C. and Akilesh, S. (2007), Nat. Rev. Immunol 7, 715-725; Baker K. et. al (2009) Semin Immunopathol. 31, 223-236; Andersen J. T. and Sandlie I. (2009) Drug Metab. Pharmacokinet. 24, 318-332 and Kuo T. T.et al. (2010) J. Clin. Immunol 30, 777-789).

Crystal structures of FcRn show the extracellular part of the heavy chain with an amino-terminal alpha1-alpha 2 platform of eight antiparallel beta-pleated strands topped by two long alpha-helices followed by the membrane proximal alpha3-domain (reviewed in Roopenian D. C. and Akilesh, S. (2007), Nat. Rev. Immunol 7, 715-725.). The beta2munit is tightly bound to residues located below the alpha1-alpha2 platform and to the alpha3-domain of the heavy chain.

The FcRn HC has been characterized from many species including human, pig, mouse, rat, rabbit and goat and they share a high degree of sequence and structural homology (see Table 3).

**Table 3. Non-exclusive list of wild type FcRn HC from various species**

| **Common name** | **Species** | **Accession number** | **Length (aa)** | **Mature sequence** | **% Identity to human FcRn HC (SEQ ID NO: 9)*** |
|---|---|---|---|---|---|
| Human | Homo sapiens | P55899 | 365 | 24-365 | 100 |
| Chimpanzee | Pan troglodytes | XP_512822 | 370 | 29-370 | 97.8 |
| Sumatran orangutan | Pongo abelii | NP_001125939 | 365 | 24-365 | 97.5 |
| Crab-eating macaque (cynomolgus macaque) | Macaca fascicularis | Q8SPV9 | 365 | 24-365 | 97.0 |
| Macaque (Rhesus Monkey) | Macaca mulatta | I0FJX2 | 365 | 24-365 | 96.7 |
| Western lowland gorilla | Gorilla gorilla gorilla | XP_004061232 | 392 | 51-392 | 84.5 |
| Dog | Canis lupus familiaris | XP_533618 | 354 | 23-392 | 83.1 |
| Cat | Felis catus | XP_003997640 | 448 | 116-448 | 79.7 |
| Cow | Bos taurus | NP_788830 Q3T119 | 354 | 24-354 | 77.1 |
| Camel | Camelus dromedarius | Q2KN22 | 355 | 25-355 | 76.8 |
| Goat | Capra hircus | XM_005692722 | 306 | ? | 75.8 |
| Sheep | Ovis aries | NP_001116875 Q8HZV2 | 354 | 24-354 | 76.3 |
| Pig | Sus scrofa | Q866U4 | 358 | 23-358 | 74.6 |
| Guinea pig | Cavia porcellus | H0VXB0 | 354 | 25-354 | 77.3 |
| Rabbit | Oryctolagus cuniculus | NP_001116409 A9Z0W1 | 358 | 24-358 | 72.9 |
| Mouse | Mus musculus | BAA07110 | 365 | 22-365 | 66.9 |
| Rat | Rattus norvegicus | P13599 | 366 | 23-366 | 65.1 |

| | | | | | |
|---|---|---|---|---|---|
| * Sequence identity was calculated using tne Needleman-Wunsch algorithm as implemented in the Needle program of EBLOSUM62 (EMBOSS suite of programs, version 6.1.0) using gap open penalty of 10, gap extension penalty of 0.5 and selecting the no brief option to obtain the longest identity. | | | | | |

As can be seen from Table 3 (FcRn HC identity), pig FcRn HC (74.6 %identity to human FcRn HC) has a similar degree of identity to human FcRn HC as mouse FcRn HC (66.9% identity to human FcRn HC), or rat FcRn HC (65.1% identity to human FcRn HC), while the non-human primates chimpanzee (97.8% identity to human FcRn HC), macaque (97% identity to human FcRn HC), Rhesus monkey (96.7% identity to human FcRn HC), gorilla (84.5% identity to human FcRn HC) and orangutan (97.5% identity to human FcRn HC), have a higher degree of identity to human FcRn HC than pig FcRn HC.

The pharmacokinetics of HSA, including HSA variants and modifications in an animal model will be influenced by the affinity of HSA for the native animal FcRn compared to the affinity of the native animal albumin for the native animal FcRn. If the affinity of the native animal albumin for the animal FcRn is higher than the affinity of HSA for the same animal FcRn, the native animal albumin will lead to greater competition for the FcRn than would be the case in a human.

It is known that mouse FcRn binds IgG from mice and humans whereas human FcRn appears to be more discriminating (Ober et al. (2001) Int. Immunol 13, 1551-1559). The binding of albumin from various species to human FcRn shows the same picture as their binding to IgG. According to Example 5 of WO 2011/051489, the binding hierarchy of albumin to soluble human FcRn ranging from strongest to weakest binding is guinea pig =/> rabbit > hamster/dog > rat/mouse > donkey > human > bovine > goat/sheep > chicken. These data show that animal albumins have different binding affinities for shFcRn. This species selectivity in relation to the FcRn-albumin interaction is relevant when considering a pharmacokinetic animal model. The cross-species reactivity between mouse albumin (MSA) and HSA to soluble mouse FcRn (smFcRn) and soluble human FcRn (shFcRn) was investigated in Andersen et al (2010) Journal of Biological Chemistry vol 285 pp 4826-4836. An extract from Table 2 of this paper is included here as Table 4:

**Table 4: Kinetics of the albumin interactions with FcRn variants**

| **Albumin species** | **FcRn species** | **Ka 10³/Ms** | **Kd 10⁻³/s** | **KD µM** | **KD steady state** |
|---|---|---|---|---|---|
| MSA | Mouse Wt | 4.2 ± 0.5 | 39.4±3.1 | 9.3 ± 0.4 | ND |
| MSA | Human Wt | 3.8 ± 0.0 | 3.1 ± 0.1 | 0.8 ± 0.2 | ND |
| HSA | Mouse Wt | NA | NA | NA | 86.2±4.1 |
| HSA | Human Wt | 2.7 ± 1.3 | 12.2 ± 5.9 | 4.5 ± 0.1 | 4.6 ± 0.5 |

No binding of albumin from either species was observed at physiological pH to either receptor. The binding affinity hierarchy at pH 6.0 was as follows; shFcRn:MSA > shFcRn:HSA > smFcRn:MSA > smFcRn:HSA. The kinetics of smFcRn:HSA binding were so fast that the binding affinity could not be determined, meaning that in a mouse, HSA would face very strong competition from the native MSA. At acidic pH, the affinity of mouse serum albumin for mouse FcRn is 9.2-fold higher than the affinity of human serum albumin for mouse FcRn, while the affinity of mouse serum albumin for human FcRn is 5.6-fold higher than the affinity of human serum albumin for human FcRn. The affinity of mouse serum albumin for human FcRn was shown to be 107.5-fold higher than the affinity of human serum albumin for the mouse FcRn. In all cases, albumin and IgG could bind the FcRn at the same time (the binding was additive). The effect of these differential binding affinities of human albumin for mouse FcRn is that in a mouse pharmacokinetic model, wild-type human albumin, or fusions, conjugates or associates of therapeutic agents to wild-type human albumin are completely or partially excluded from interacting with the mouse FcRn due to their reduced affinity for the mouse FcRn receptor and/or by competition due to the higher abundance of the endogenous mouse albumin. Consequently, the observed pharmacokinetic profile and the circulatory half-life of the wild-type human albumin, or fusions, conjugates or associates of therapeutic agents to wild-type human albumin is significantly compromised. Indeed when using a mouse to compare the half-life of a therapeutic agent with the same therapeutic agent fused or conjugated to HSA as was done in Muller et al (2007) Journal of Biological Chemistry vol 282 pp. 12650-12660 it will generally be possible to observe an increase in half-life of the HSA fusion. This increase may however simply be due to an increase of the molecular weight of the therapeutic agent above the threshold of renal clearance and not due to FcRn mediated rescue of the therapeutic agents fused, conjugated or associated with albumin.

Mouse FcRn is promiscuous regarding binding specificity and binds IgG of many species (i.e. human, primate, mouse, rabbit, guinea pig, bovine, sheep, and rat). In contrast, human FcRn is more stringent, and binds only IgG of human, primate, rabbit, and guinea pig origin (Ober R. J. et al. (2001) Int. Immunol. 13, 1551-1559; Stein C. et al. (2011) Mamm. Genome 23:259-269). Consequently the pharmacokinetics of human IgGs or monoclonal antibodies can be assessed without competition from endogenous mouse IgG in the human FcRn transgenic mouse (homozygous KO of the mouse gene and a heterozygous KI of the human gene) (Roopenian et al (2003) J. Immunol. Vol 170, pp. 3528-3533), while in the same model the pharmacokinetics of human albumin or compounds fused, conjugated or associated with human albumin will be compromised by competition from endogenous mouse albumin from both the greater affinity of mouse albumin for human FcRn and the greater abundance of the mouse albumin compared to the human albumin fusion, conjugate or associate.

As illustrated in Muller et al (2007) Journal of Biological Chemistry vol 282 pp. 12650, a mouse model may be sufficient to generally observe an increase in half-life of the HSA fusion due to the increase of the molecular weight of the therapeutic agent above the threshold of renal clearance. However, if an animal model is to be used to compare the half-life of a variant HSA or a modified variant HSA with Wt HSA or a modified Wt HSA the FcRn mediated rescue is important and then the competition from the native albumin will play an important role.

A mathematical model has been developed for FcRn recycling to assist in the design of human serum albumin variants with extend circulatory half-lives (Bergmann K. et al. (2012) 21st PAGE meeting, 5-8th June, Venice Italy). The authors use the model to predict the circulatory half-life of human albumin variants with increased affinity for human FcRn in a human FcRn transgenic mouse, monkey and humans, and conclude that the half-life increases observed are smaller for the human FcRn transgenic mouse than for monkey and human.

In the present application, the inventors have realized that when assessing pharmacokinetic differences of a variant HSA compared to wild type HSA there needs to be as little competition from the native albumin as possible. This can be achieved if HSA binds the native FcRn in a manner similar to the binding of native albumin to the same FcRn. This is naturally the case in primates where the amino acid sequences of albumin and FcRn show a high level of identity between species (see Tables 1 and 3). However, when moving to non-primate species the diversity increases and it becomes more challenging to find an animal model suitable for assessing pharmacokinetics of a variant HSA compared to wild type HSA.

Understanding the interaction between HSA and hFcRn is important in identifying a non-primate animal model that mimics this interaction as well as possible. In the alpha-1 domain of mature human FcRn HC (SEQ ID NO: 16) the conserved glutamic acid at position 54 is crucial for HSA binding and mutations in position 56 show decrease in binding to HSA (Anderson et al 2012 Nature Communication 3:610). In the alpha-2 domain of mature human FcRn HC the conserved histidine at position 166 is crucial for HSA binding, and if the H in position 161 (SEQ ID NO: 16) is mutated to an alanine then binding of HSA decreases by 10 fold (Andersen et al. 2006, Eur. J. Immonol. 36, 3044-3051). A cross-species alignment of selected regions of the alpha-1 and alpha-2 domains is shown in figure 7. Based on this alignment the inventors suggest that position 52 and position 161 of mature human FcRn (SEQ ID NO: 16) are partly responsible for the species selectivity described above.

An aspect of the present invention provides a method for assessing one or more (several) pharmacokinetic properties of a variant HSA compared to wild type HSA comprising a) selecting a non-primate animal species where the binding affinity of wild type HSA to the native FcRn of said animal is the same as or higher than the binding affinity of the native albumin of said animal to said FcRn; b) administering the variant HSA to one animal and the wild type HSA to another animal of the animal species selected in a); and c) measuring one or more (several) pharmacokinetic properties of the variant HSA and the wild type HSA.

A preferred non-primate animal species is one where the binding affinity of HSA for the native FcRn is approximately the same (1 fold ± 0.15) as the binding affinity of the native albumin to the native FcRn. Such a model would very much resemble the competition that the Wt HSA or variant HSA would be subject to when injected into a human. In a preferred embodiment of the present invention the binding affinity of wild type HSA to the native FcRn of said animal is between 0.8 and 3.5 fold when compared with the binding affinity of the native albumin of said animal, more preferred between 0.9 and 3, more preferred between 1 and 2.5, more preferred between 1 and 2, more preferred between 1 and 1.5, and most preferred the binding affinity of Wt HSA is between 1 and 1.1 fold higher than the binding affinity of the native albumin of said animal. Another preferred non-primate animal species of the present invention is one where the binding affinity of HSA for the native FcRn would be higher than the binding affinity of the native albumin of said animal species to the native FcRn. Such a model would make it easier to assess the difference in one or more (several) pharmacokinetic properties between Wt HSA and the variant HSA and would be very suitable for screening a number of variant HSAs to select one or more (several) lead candidates for conjugation, fusion or association with a partner such as a therapeutic agent. In a preferred embodiment of the present invention the binding affinity of wild type HSA to the native FcRn of said animal is at least 1.5 fold higher than the binding affinity of the native albumin of said animal, more preferred at least 2 folder higher, more preferred at least 3 fold higher, more preferred at least 3.5 folder higher and most preferred it is at least 4 fold higher than the binding affinity of the native albumin of said animal. Binding affinities between albumin and native FcRn is preferably measured using a soluble FcRn heavy-chain of the selected animal species coupled to a chip and measured in by SPR e.g. using a Biacore instrument. The soluble FcRn HC is an alpha chain without the transmembrane domain or an FcRn HC consisting of three ectodomains (a1-a3). The soluble FcRn may also include amino acids from the connecting peptide of the FcRn, which is between the ectodomains and the transmembrane region of the FcRn. Preferably the soluble FcRn HC is co-expressed with beta2-microglobulin from the same species as described in Example 4. In a preferred embodiment the soluble animal FcRn comprises a FcRn heavy chain and a beta2-globulin from the same animal species. More preferably the soluble FcRn are composed of soluble pig FcRn HC and pig β2-microglobulin. Preferably, the method described in the "Materials and Methods" section is used to select a non-primate animal species in the method of the present invention with the variations in Example 4 are applied.

A preferred non-primate animal species of the present invention is a wild type animal species expressing its wild type FcRn and wild type albumin. Wild type animals include species which have been bred by mating animals with specific naturally occurring genotypes, but does not include animals where genes actively have been knocked out or inserted by human intervention. A preferred non-primate wild type animal is a pig, preferably a Göttingen minipig. Pig has been recognized as an acceptable model for predictive interspecies allometric scaling (Larsen M. O. and Rolin B. (2004) ILAR Journal 45, 303-313; Zheng Y. et al. (2012) mAbs 4, 243-255; Suenderhauf C. and Parrott N. (2013) Pharm. Res. 30, 1-15). There is however nothing in these disclosures that points to pig as a preferred model system to study the pharmacokinetics of human albumin or fusions, conjugates or associates to human albumin, or variant human albumins or fusions, conjugates or associates to variant human albumin.

Example 2 of the present invention shows that the affinity of human albumin for soluble pig FcRn (spFcRn) is 2.9 fold higher than the affinity of pig albumin for soluble pFcRn. The affinity of selected human albumin variants for pFcRn has also been shown to have higher than the affinity of Wt HSA for pFcRn. The ability to differentiate between the pharmacokinetic properties of WT HSA and variant HSA in pig animal studies has been shown in Example 5.

Other preferred wild type non-primate animal species are goat, sheep, cow or camel.

A common feature between primate FcRn and pig, goat, sheep, cow and camel FcRn, as indicated by bold letters in figure 7, is that they have a V in the position corresponding to position 52 when aligned to SEQ ID NO: 16 and an H in the position corresponding to position 161 when aligned to SEQ ID NO: 16. All other amino acids in the aligned regions are fully conserved across all the species, except for position 55, 164 and 165. At position 164 and 165 human and macaque have an R and E respectively whereas all the other species have an L and G respectively. At Position 55 the species have either an N or an S. Since pig has been shown to be a good animal model as illustrated in example 2, we do not expect that the variation in these positions have a significant influence on the species selectivity on the FcRn-albumin interaction. In one embodiment of the present invention the native FcRn has a histidine in the position corresponding to position 161 when aligned to SEQ ID NO: 16.

In another preferred embodiment of the present invention the native FcRn has a valine in the position corresponding to position 52 when aligned to SEQ ID NO: 16.

In an even more preferred embodiment the native FcRn has a valine in the position corresponding to position 52 and a histidine in the position corresponding to position 161 when aligned to SEQ ID NO: 16.

In one aspect of the invention, a pig animal model or a goat or sheep or cow or camel animal model is used to compare one or more (several) pharmacokinetic properties of a control molecule and a test molecule in which the control molecule comprises wild type HSA and the test molecule comprises a variant of HSA.

An alternative to wild-type animal species is a transgenic animal. A preferred transgenic animal for use in the method of the present invention is a non-primate animal which has its wild type (endogenous) FcRn and wild type (endogenous) albumin knocked out and a human FcRn heavy chain and human serum albumin inserted into the genome. Preferably the human FcRn is 90, 91, 92, 93, 94, 95, 96, 97, 98, 99, 99.5, 100% identical to the mature sequence of SEQ ID NO: 9 and the human HSA is 90, 91, 92, 93, 94, 95, 96, 97, 98, 99, 99.5, 100% identical to SEQ ID NO: 2. In such a transgenic animal the human FcRn heavy chain and the Wt HSA are considered to be the native FcRn and native albumin, and it is understood that there is no (or substantially no) underlying expression of the animal's endogenous wild type FcRn heavy chain and albumin. In a preferred embodiment of the present invention the double transgenic animal is a rodent or rabbit. Preferably, the rodent is selected from mouse, guinea pig, and rat. More preferred the rodent is a double transgenic mouse.

The animal species of the present invention may also be an animal where the Wt FcRn has been mutated such that it contains a valine in the position corresponding to position 52 and/or a histidine in the position corresponding to position 161 when aligned to SEQ ID NO: 16. In such an animal the conserved amino acids E54, Q56 and H166 when aligned to SEQ ID NO: 16 are maintained. This animal species can either contain wild type (endogenous) albumin or it can be transgenic with respect to albumin, such that the endogenous albumin is knocked out and substituted with HSA. An example of such an animal could be a mouse with a mouse FcRn HC variant comprising a valine in position 52 and a histidine in position 161 when aligned to SEQ ID NO:16 and where the variant is at least 90, 91, 92, 93, 94, 95, 96, 97, 98, 99% identical to the mature sequence of SEQ ID NO:13 and the mouse also comprises a transgene HSA and preferably the native MSA expression and wt FcRn expression has been abolished.

A further aspect of the present invention is directed to a double transgenic animal. More specifically, the transgenic animal's genome comprises a homozygous disruption in its endogenous FcRn HC gene and serum albumin gene that prevents the expression of a functional animal FcRn HC protein and functional animal serum albumin and the genome further comprises a heterologous DNA sequence encoding a human FcRn HC (hFcRn HC) and a heterologous DNA sequence encoding human serum albumin (HSA), and wherein , and wherein the animal expresses a functional hFcRn HC protein and functional HSA. The relevant sequences for albumin are indicated in Table 1 and the relevant FcRn HC sequences are indicated in Table 3. Preferably the human FcRn is 90, 91, 92, 93, 94, 95, 96, 97, 98, 99, 99.5, 100% identical to the mature sequence of SEQ ID NO: 9 or to SEQ ID NO: 16, more preferred the human FcRn HC has a histidine in position 161 when aligned to SEQ ID NO: 16, and the conserved amino acids E54, Q56 and H166 when aligned to SEQ ID NO: 16 are maintained. Preferably the human HSA is 90, 91, 92, 93, 94, 95, 96, 97, 98, 99, 99.5, 100% identical to SEQ ID NO: 2. Preferably, the double transgenic animal is a rodent or a rabbit. Preferably the rodent is selected from mouse, guinea pig, and rat. The most preferred double transgenic animal is a mouse. The heterologous DNA sequences that have been inserted into the genome of the animal can be operably linked to the same or different regulatory sequences as the endogenous genes. The disruption of the endogenous genes can be done by replacement with the corresponding heterologous DNA (human genes). The disruption can however alternatively be done independently of the insertion of the heterologous DNA (transgenic human genes), allowing the heterologous DNA to be inserted in a different place of the genome than the endogenous gene. The generation of a mouse with transgenic human FcRn HC as native FcRn has been described in US 7,358,416. Likewise US 6,949,691 describes how to produce a mouse with transgenic human serum albumin as the native albumin. A person skilled in the art of producing transgenic animals would, based on these two documents, be capable of producing a transgenic animal which has its wild type FcRn HC gene and wild type albumin gene knocked out and a wild type human FcRn HC DNA and wild type human serum albumin DNA inserted into the genome such that human FcRn HC and HSA is produced in the animal instead of the wild type FcRn and albumin.

In another embodiment of the invention the transgenic animal is a rabbit or rodent whose genome comprises a homozygous disruption in its endogenous FcRn heavy chain gene and endogenous serum albumin gene and further comprises a heterologous DNA sequence that expresses an FcRn with a histidine in the position corresponding to position 161 when aligned to SEQ ID NO: 16 and a heterologous DNA sequence encoding human serum (HSA), where the heterologous DNA sequences are operably linked to the same or different regulatory sequences, and wherein said homozygous disruption prevents the expression of a functional rabbit or rodent FcRn HC protein and functional rabbit or rodent serum albumin, and wherein the animal expresses a functional FcRn HC protein with a histidine in position 161 when aligned to SEQ ID NO: 16 and functional HSA.

In another aspect of the invention a transgenic rabbit or rodent that expresses an FcRn with a histidine in the position corresponding to position 161 when aligned to SEQ ID NO: 16 and human albumin and which has been knocked out for the corresponding native proteins is used to compare one or more (several) pharmacokinetic properties of a control molecule and a test molecule in which the control molecule comprises wild type HSA and the test molecule comprises a variant of HSA. In a further embodiment the FcRn further comprises a valine in position 52 when aligned to SEQ ID NO: 16. In a preferred embodiment the transgene FcRn is selected from human, chimpanzee, macaque, cow, goat, sheep, camel and pig. Alternatively, the FcRn is a variant of the Wt FcRn with a H in the position corresponding to position 161 when aligned to SEQ ID NO: 16 and/or a V in the position corresponding to position 52 when aligned to SEQ ID NO: 16, and wherein the variant is at least 90, 91, 92, 93, 94, 95, 96, 97, 98, 99% identical to the Wt FcRn of the animal. In such a variant the conserved amino acids E54, Q56 and H166 when aligned to SEQ ID NO: 16 are maintained. More preferred other amino acids which are conserved among human, chimpanzee, macaque, cow, goat, sheep, camel, pig, dog, guinea pig, rabbit, rat and mouse are maintained.

As described above, the method of the present invention can be used to screen variant HSA molecules to identify the *in vivo* effect of altered FcRn binding affinity when compared to Wt HSA. Preferably, the variants tested in the method of the present invention have increased human FcRn binding compared to Wt HSA. In a preferred embodiment the KD of the variant albumin is at least 2X lower than the KD of Wt HSA, more preferably the KD of the variant albumin is at least 5X, 10X, 15X or 20X, 30X, 50X, 75X lower than the KD of Wt HSA, most preferably the KD of the variant albumin is at least 100X lower than the KD of Wt HSA (the variant has one hundred times the binding affinity to human FcRn than Wt HSA). In another embodiment the variant albumin has a weaker binding to human FcRn than Wt HSA. In a preferred embodiment the KD of the variant albumin is at least 2X higher than the KD of Wt HSA (the variant has half the binding affinity to human FcRn than Wt HSA), more preferably the KD of the variant albumin is at least 5X, 10X, 15X or 20X, 30X, 50X, 75X higher than the KD of Wt HSA, most preferably the KD of the variant albumin is at least 100X higher than the KD of Wt HSA (the variant has one hundredth the binding affinity to human FcRn than HSA). The method of the present invention can in particular be used to select a variant HSA for use in a pre-clinical trial, where the variant HSA has improved one or more (several) pharmacokinetic properties when compared with wild type HSA. In a preferred embodiment the variant HSA has a longer half-life than wild type HSA.

The present invention also relates to variant HSA molecules as such, which have been selected using the method of the present invention. In particular a variant HSA with one or more (several) improved pharmacokinetic properties when compared with wild type HSA that are selected for use in a pre-clinical trial is encompassed by the present invention. Improved pharmacokinetic properties are properties which are changed compared to Wt HSA and which will result in an advantage in relation to for example administration regime, dose, targeting, or treatment effectiveness compared to Wt HSA. Preferred is a variant HSA that has a longer half-life than Wt HSA.

The method of the present invention can also be used to assay the effect of modifications to HSA or variant HSA. For example, the method may be used to assess the effect of a) various linkers between a partner, such as a therapeutic agent, and HSA (both fusion linkers and conjugation linkers) or b) the position in HSA to which conjugation or fusion is made or c) the chemistry used for conjugation of the partner. This list is not exhaustive and the skilled person will know how to use the method of the present invention to assess other modification effects. Generally, modifications to HSA may affect FcRn binding as seen with some of the natural occurring damages like glycosylation and oxidation described above. The present model is therefore relevant for assessing one or more (several) pharmacokinetic properties of modifications to Wt HSA and variant HSA, since these modifications may affect the FcRn binding of the Wt or variant HSA. In a preferred embodiment the Wt HSA and variant HSA are modified by adding an additional functionality. The additional functionality can take the form of a partner molecule for example a therapeutic agent, diagnostic agent, targeting molecule that can ensure delivery of the HSA to specific cells in a human, a purification tag or identification tag like His, FLAG, GST, or fluorescence markers. In a preferred embodiment the variant and wild type HSA modified by fusion, conjugation or association with a partner. Preferably the partner is a therapeutic agent, including vaccines. In order to compare the difference between the modified variant HSA and modified Wt HSA the modification should be identical for both molecules, for example in the form of a fusion, conjugation or association to the same partner. In a preferred embodiment of the present invention the animal model is used to assess the effect on one or more (several) of the pharmacokinetic properties of a selected partner, e.g. therapeutic agent, and the method of joining the partner to the control and test molecule is assessed. The control molecule can for example be Wt HSA joined to the partner and the test molecule is a variant HSA joined to the same partner at the same position. The method of joining the albumin and the selected partner can for example be one or more (several) of a) to c) mentioned above. In one embodiment the partner is conjugated at different positions on the albumin. The positions are identical for both control and test molecule, so if for example position 1 and 34 in WT HSA (SEQ ID NO:2) is tested for conjugation, the same position is tested in the variant HSA using the same partner, thereby testing the effect of the different positions on FcRn binding. In another embodiment the partner is conjugated with different conjugation technologies to the albumin control and test molecules. The conjugation technology or chemistry used is identical for both control and test molecule, so if for example one or more maleimide groups are tested for conjugation, the same groups are tested in the variant HSA using the same partner, thereby testing the effect of the different conjugation techniques on FcRn binding. In yet another embodiment the partner is fused with or without different linkers at the C-terminal and/or N-terminal of the albumin. The linkers are identical for both control and test molecules. The linker peptide between the fused portions (albumin and partner) provides greater physical separation between the moieties and thus maximizes the accessibility of the fusion partner, e.g. the therapeutic agent, for instance, for binding to its cognate receptor. The linker peptide may consist of amino acids such that it is flexible or more rigid.

In a preferred embodiment the partner, e.g. therapeutic agent, is fused to the N-terminal of the albumin. In an alternative embodiment the partner, e.g. therapeutic agent, is fused to the C-terminal of the albumin.

Therefore, as described above, the albumin fusion polypeptides of the invention may have the following formula R2-R1; R1-R2; R2-R1-R2; R2-L-R1-L-R2; R1-L-R2; R2-L-R1; or R1-L-R2-L-R1, wherein R1 is at least one fusion partner sequence (including fragments or variants thereof), and not necessarily the same polypeptide, L is a linker and R2 is an albumin sequence (including fragments or variants thereof). Examples of linkers include (GGGGS)_{N} or (GGGS)_{N} or (GGS)_{N}, wherein N is an integer greater than or equal to 1 and wherein G represents glycine and S represents serine. The linkers may have a varying length from 1 to 50 amino acids, preferably from 3 to 40 amino acids, more preferably from 5 to 35 amino acids, even more preferably from 7 to 30 amino acids and most preferably from 10 to 25 amino acids. A fusion polypeptide can further comprise a cleavage site between the fusion partner and the albumin, e.g. in the form of a cleavable linker. The site may be cleaved upon secretion of the fusion polypeptide, releasing the two polypeptides. The linker may alternatively be cleavable, e.g. by a protease which exists in the patient, such that the fusion partner moiety is released from the albumin within the patient, potentially in relation to an activation event. One example of a protease driven activation event is the coagulation cascade (WO 91/09125, WO 2007/090584 and WO 2007/144173). Examples of cleavage sites include, but are not limited to, the sites disclosed in Martin et al., 2003, J. Ind. Microbiol. Biotechnol. 3: 568-576; Svetina et al., 2000, J. Biotechnol. 76: 245-251; Rasmussen-Wilson et al., 1997, Appl. Environ. Microbiol. 63: 3488-3493; Ward et al., 1995, Biotechnology 13: 498-503; and Contreras et al., 1991, Biotechnology 9: 378-381; Eaton et al., 1986, Biochemistry 25: 505-512; Collins-Racie et al., 1995, Biotechnology 13: 982-987; Carter et al., 1989, Proteins: Structure, Function, and Genetics 6: 240-248; and Stevens, 2003, Drug Discovery World 4: 35-48.

Albumin is used in preparations of pharmaceutically beneficial compounds for example as fusions, conjugations or associations with a therapeutic agent. The present invention also includes modified variant HSA such as a variant HSA which is fused, conjugated or associated with a therapeutic agent, where the fusion, conjugation or association is selected by a method of the present invention. In particular a variant HSA fused, conjugated or associated with a partner, e.g. therapeutic agent, with one or more (several) improved pharmacokinetic properties when compared with wild type HSA fused, conjugated or associated with the same partner, where the modified variant HSA is selected for use in a pre-clinical trial is encompassed by the present invention. More preferred is a variant HSA fused, conjugated or associated with a partner, e.g. therapeutic agent, , that has a longer half-life than wild type HSA fused, conjugated or associated with the same partner, e.g. therapeutic agent.

Variant HSA and the modified variant HSA of the present invention may be formulated into a pharmaceutical composition comprising the variant HSA and an excipient. One way of formulating variant HSA or modified variant HSA can be as nanoparticles or microparticles. The incorporation of a partner, e.g. therapeutic agent, into an albumin particle has for example been described in WO 00/71079. Techniques for incorporation of a molecule into nano- or microparticles are known in the art. Preferred methods for preparing nano- or microparticles that may be applied to the albumin variant, fragment, fusion, conjugate or associate thereof according to the invention are disclosed in WO 2004/071536 or WO2008/007146 or Oner & Groves (Pharmaceutical Research, Vol 10(9), 1993, pages 1387 to 1388).

For all aspects of the invention fusion partner polypeptides and/or conjugates and/or associates may comprise one or more (several) of: 4-1BB ligand, 5-helix, A human C-C chemokine, A human L105 chemokine, A human L105 chemokine designated huL105_3., A monokine induced by gamma-interferon (MIG), A partial CXCR4B protein, A platelet basic protein (PBP), α1-antitrypsin, ACRP-30 Homologue; Complement Component C1q C, Adenoid-expressed chemokine (ADEC), aFGF; FGF-1, AGF, AGF Protein, albumin, an etoposide, angiostatin, Anthrax vaccine, Antibodies specific for collapsin, antistasin, Anti-TGF beta family antibodies, antithrombin III, APM-1; ACRP-30; Famoxin, apo-lipoprotein species, Arylsulfatase B, b57 Protein, BCMA, Beta-thromboglobulin protein (beta-TG), bFGF; FGF2, Blood coagulation factors, BMP Processing Enzyme Furin, BMP-10, BMP-12, BMP-15, BMP-17, BMP-18, BMP-2B, BMP-4, BMP-5, BMP-6, BMP-9, Bone Morphogenic Protein-2, calcitonin, Calpain-10a, Calpain-10b, Calpain-10c, Cancer Vaccine, Carboxypeptidase, C-C chemokine, MCP2, CCR5 variant, CCR7, CCR7, CD11a Mab, CD137; 4-1BB Receptor Protein, CD20 Mab, CD27, CD27L, CD30, CD30 ligand, CD33 immunotoxin, CD40, CD40L, CD52 Mab, Cerebus Protein, Chemokine Eotaxin., Chemokine hIL-8, Chemokine hMCP1, Chemokine hMCP1a, Chemokine hMCP1b, Chemokine hMCP2, Chemokine hMCP3, Chemokine hSDF1b, Chemokine MCP-4, chemokine TECK and TECK variant, Chemokine-like protein IL-8M1 Full-Length and Mature, Chemokine-like protein IL-8M10 Full-Length and Mature, Chemokine-like protein IL-8M3, Chemokine-like protein IL-8M8 Full-Length and Mature, Chemokine-like protein IL-8M9 Full-Length and Mature, Chemokine-like protein PF4-414 Full-Length and Mature, Chemokine-like protein PF4-426 Full-Length and Mature, Chemokine-like protein PF4-M2 Full-Length and Mature, Cholera vaccine, Chondromodulin-like protein, c-kit ligand; SCF; Mast cell growth factor; MGF; Fibrosarcoma-derived stem cell factor, CNTF and fragment thereof (such as CNTFAx15'(Axokine™)), coagulation factors in both pre and active forms, collagens, Complement C5 Mab, Connective tissue activating protein-III, CTAA16.88 Mab, CTAP-III, CTLA4-Ig, CTLA-8, CXC3, CXC3, CXCR3; CXC chemokine receptor 3, cyanovirin-N, Darbepoetin, designated exodus, designated huL105_7., DIL-40, DNase, EDAR, EGF Receptor Mab, ENA-78, Endostatin, Eotaxin, Epithelial neutrophil activating protein-78, EPO receptor; EPOR, erythropoietin (EPO) and EPO mimics, Eutropin, Exodus protein, Factor IX, Factor VII, Factor VIII, Factor X and Factor XIII, FAS Ligand Inhibitory Protein (DcR3), FasL, FasL, FasL, FGF, FGF-12; Fibroblast growth factor homologous factor-1, FGF-15, FGF-16, FGF-18, FGF-3; INT-2, FGF-4; gelonin, HST-1; HBGF-4, FGF-5, FGF-6; Heparin binding secreted transforming factor-2, FGF-8, FGF-9; Glia activating factor, fibrinogen, flt-1, flt-3 ligand, Follicle stimulating hormone Alpha subunit, Follicle stimulating hormone Beta subunit, Follitropin, Fractalkine, fragment. myofibrillar protein Troponin I, FSH, Galactosidase, Galectin-4, G-CSF, GDF-1, Gene therapy, Glioma-derived growth factor, glucagon, glucagon-like peptides, Glucocerebrosidase, glucose oxidase, Glucosidase, Glycodelin-A; Progesterone-associated endometrial protein, GM-CSF, gonadotropin, Granulocyte chemotactic protein-2 (GCP-2), Granulocyte-macrophage colony stimulating factor, growth hormone, Growth related oncogene-alpha (GRO-alpha), Growth related oncogene-beta (GRO-beta), Growth related oncogene-gamma (GRO-gamma), hAPO-4; TROY, hCG, Hepatitus B surface Antigen, Hepatitus B Vaccine, HER2 Receptor Mab, hirudin, HIV gp120, HIV gp41, HIV Inhibitor Peptide, HIV Inhibitor Peptide, HIV Inhibitor Peptide, HIV protease inhibiting peptides, HIV-1 protease inhibitors, HPV vaccine, Human 6CKine protein, Human Act-2 protein, Human adipogenesis inhibitory factor, human B cell stimulating factor-2 receptor, Human beta-chemokine H1305 (MCP-2), Human C-C chemokine DGWCC, Human CC chemokine ELC protein, Human CC type chemokine interleukin C, Human CCC3 protein, Human CCF18 chemokine, Human CC-type chemokine protein designated SLC (secondary lymphoid chemokine), Human chemokine beta-8 short forms, Human chemokine C10, Human chemokine CC-2, Human chemokine CC-3, Human chemokine CCR-2, Human chemokine Ckbeta-7, Human chemokine ENA-78, Human chemokine eotaxin, Human chemokine GRO alpha, Human chemokine GROalpha, Human chemokine GRObeta, Human chemokine HCC-1, Human chemokine HCC-1, Human chemokine I-309, Human chemokine IP-10, Human chemokine L105_3, Human chemokine L105_7, Human chemokine MIG, Human chemokine MIG-beta protein, Human chemokine MIP-1alpha, Human chemokine MIP1beta, Human chemokine MIP-3alpha, Human chemokine MIP-3beta, Human chemokine PF4, Human chemokine protein 331D5, Human chemokine protein 61164, Human chemokine receptor CXCR3, Human chemokine SDF1alpha, Human chemokine SDF1beta, Human chemokine ZSIG-35, Human Chr19Kine protein, Human CKbeta-9, Human CKbeta-9, Human CX3C 111 amino acid chemokine, Human DNAX interleukin-40, Human DVic-1 C-C chemokine, Human EDIRF I protein sequence, Human EDIRF II protein sequence, Human eosinocyte CC type chemokine eotaxin, Human eosinophil-expressed chemokine (EEC), Human fast twitch skeletal muscle troponin C, Human fast twitch skeletal muscle troponin I, Human fast twitch skeletal muscle Troponin subunit C, Human fast twitch skeletal muscle Troponin subunit I Protein, Human fast twitch skeletal muscle Troponin subunit T, Human fast twitch skeletal muscle troponin T, Human foetal spleen expressed chemokine, FSEC, Human GM-CSF receptor, Human gro-alpha chemokine, Human gro-beta chemokine, Human gro-gamma chemokine, Human IL-16 protein, Human IL-1RD10 protein sequence, Human IL-1RD9, Human IL-5 receptor alpha chain, Human IL-6 receptor, Human IL-8 receptor protein hIL8RA, Human IL-8 receptor protein hIL8RB, Human IL-9 receptor protein, Human IL-9 receptor protein variant #3, Human IL-9 receptor protein variant fragment, Human IL-9 receptor protein variant fragment#3, Human interleukin 1 delta, Human Interleukin 10, Human Interleukin 10, Human interleukin 18, Human interleukin 18 derivatives, Human interleukin-1 beta precursor, Human interleukin-1 beta precursor., Human interleukin-1 receptor accessory protein, Human interleukin-1 receptor antagonist beta, Human interleukin-1 type-3 receptor, Human Interleukin-10 (precursor), Human Interleukin-10 (precursor), Human interleukin-11 receptor, Human interleukin-12 40 kD subunit, Human interleukin-12 beta-1 receptor, Human interleukin-12 beta-2 receptor, Human Interleukin-12 p35 protein, Human Interleukin-12 p40 protein, Human interleukin-12 receptor, Human interleukin-13 alpha receptor, Human interleukin-13 beta receptor, Human interleukin-15, Human interleukin-15 receptor from clone P1, Human interleukin-17 receptor, Human interleukin-18 protein (IL-18), Human interleukin-3, human interleukin-3 receptor, Human interleukin-3 variant, Human interleukin-4 receptor, Human interleukin-5, Human interleukin-6, Human interleukin-7, Human interleukin-7., Human interleukin-8 (IL-8), Human intracellular IL-1 receptor antagonist, Human IP-10 and HIV-1 gp120 hypervariable region fusion protein, Human IP-10 and human Muc-1 core epitope (VNT) fusion protein, human liver and activation regulated chemokine (LARC), Human Lkn-1 Full-Length and Mature protein, Human mammary associated chemokine (MACK) protein Full-Length and Mature, Human mature chemokine Ckbeta-7, Human mature gro-alpha, Human mature gro-gamma polypeptide used to treat sepsis, Human MCP-3 and human Muc-1 core epitope (VNT) fusion protein, Human MI10 protein, Human MI1A protein, Human monocyte chemoattractant factor hMCP-1, Human monocyte chemoattractant factor hMCP-3, Human monocyte chemotactic proprotein (MCPP) sequence, Human neurotactin chemokine like domain, Human non-ELR CXC chemokine H174, Human non-ELR CXC chemokine IP10, Human non-ELR CXC chemokine Mig, Human PAI-1 mutants, Human protein with IL-16 activity, Human protein with IL-16 activity, Human secondary lymphoid chemokine (SLC), Human SISD protein, Human STCP-1, Human stromal cell-derived chemokine, SDF-1, Human T cell mixed lymphocyte reaction expressed chemokine (TMEC), Human thymus and activation regulated cytokine (TARC), Human thymus expressed, Human TNF-alpha, Human TNF-alpha, Human TNF-beta (LT-alpha), Human type CC chemokine eotaxin 3 protein sequence, Human type II interleukin-1 receptor, Human wild-type interleukin-4 (hIL-4) protein, Human ZCHEMO-8 protein, Humanized Anti-VEGF Antibodies, and fragments thereof, Humanized Anti-VEGF Antibodies, and fragments thereof, Hyaluronidase, ICE 10 kD subunit., ICE 20 kD subunit., ICE 22 kD subunit., Iduronate-2-sulfatase, Iduronidase, IL-1 alpha, IL-1 beta, IL-1 inhibitor (IL-1i)., IL-1 mature, IL-10 receptor, IL-11, IL-11, IL-12 p40 subunit., IL-13, IL-14, IL-15, IL-15 receptor, IL-17, IL-17 receptor, II-17 receptor, II-17 receptor, IL-19, IL-1i fragments, IL1-receptor antagonist, IL-21 (TIF), IL-3 containing fusion protein., IL-3 mutant proteins, IL-3 variants, IL-3 variants, IL-4, IL-4 mutein, IL-4 mutein Y124G, IL-4 mutein Y124X, IL-4 muteins, II-5 receptor, IL-6, II-6 receptor, IL-7 receptor clone, IL-8 receptor, IL-9 mature protein variant (Met117 version), immunoglobulins or immunoglobulin-based molecules or fragment of either (e.g. a Small Modular ImmunoPharmaceutical™ ("SMIP") or dAb, Fab' fragments, F(ab')2, scAb, scFv or scFv fragment), including but not limited to plasminogen, Influenza Vaccine, Inhibin alpha, Inhibin beta, insulin, insulin-like growth factor, Integrin Mab, inter-alpha trypsin inhibitor, inter-alpha trypsin inhibitor, Interferon gamma-inducible protein (IP-10), interferons (such as interferon alpha species and sub-species, interferon beta species and sub-species, interferon gamma species and sub-species), interferons (such as interferon alpha species and sub-species, interferon beta species and sub-species, interferon gamma species and sub-species), Interleukin 6, Interleukin 8 (IL-8) receptor, Interleukin 8 receptor B, Interleukin-1alpha, Interleukin-2 receptor associated protein p43, interleukin-3, interleukin-4 muteins, Interleukin-8 (IL-8) protein., interleukin-9, Interleukin-9 (IL-9) mature protein (Thr117 version), interleukins (such as IL10, IL11 and IL2), interleukins (such as IL10, IL11 and IL2), Japanese encephalitis vaccine, Kalikrein Inhibitor, Keratinocyte growth factor, Kunitz domain protein (such as aprotinin, amyloid precursor protein and those described in WO 03/066824, with or without albumin fusions), Kunitz domain protein (such as aprotinin, amyloid precursor protein and those described in WO 03/066824, with or without albumin fusions), LACI, lactoferrin, Latent TGF-beta binding protein II, leptin, Liver expressed chemokine-1 (LVEC-1), Liver expressed chemokine-2 (LVEC-2), LT-alpha, LT-beta, Luteinization Hormone, Lyme Vaccine, Lymphotactin, Macrophage derived chemokine analogue MDC (n+1), Macrophage derived chemokine analogue MDC-eyfy, Macrophage derived chemokine analogue MDC-yl, Macrophage derived chemokine, MDC, Macrophage-derived chemokine (MDC), Maspin; Protease Inhibitor 5, MCP-1 receptor, MCP-1a, MCP-1b, MCP-3, MCP-4 receptor, M-CSF, Melanoma inhibiting protein, Membrane-bound proteins, Met117 human interleukin 9, MIP-3 alpha, MIP-3 beta, MIP-Gamma, MIRAP, Modified Rantes, monoclonal antibody, MP52, Mutant Interleukin 6 S176R, myofibrillar contractile protein Troponin I, Natriuretic Peptide, Nerve Growth Factor-beta, Nerve Growth Factor-beta2, Neuropilin-1, Neuropilin-2, Neurotactin, Neurotrophin-3, Neurotrophin-4, Neurotrophin-4a, Neurotrophin-4b, Neurotrophin-4c, Neurotrophin-4d, Neutrophil activating peptide-2 (NAP-2), NOGO-66 Receptor, NOGO-A, NOGO-B, NOGO-C, Novel beta-chemokine designated PTEC, N-terminal modified chemokine GroHEK/hSDF-1alpha, N-terminal modified chemokine GroHEK/hSDF-1beta., N-terminal modified chemokine met-hSDF-1 alpha, N-terminal modified chemokine met-hSDF-1 beta, OPGL, Osteogenic Protein-1; OP-1; BMP-7, Osteogenic Protein-2, OX40; ACT-4, OX40L, Oxytocin (Neurophysin I), parathyroid hormone, Patched, Patched-2, PDGF-D, Pertussis toxoid, Pituitary expressed chemokine (PGEC), Placental Growth Factor, Placental Growth Factor-2, Plasminogen Activator Inhibitor-1; PAI-1, Plasminogen Activator Inhibitor-2; PAI-2, Plasminogen Activator Inhibitor-2; PAI-2, Platelet derived growth factor, Platelet derived growth factor Bv-sis, Platelet derived growth factor precursor A, Platelet derived growth factor precursor B, Platelet Mab, platelet-derived endothelial cell growth factor (PD-ECGF), Platelet-Derived Growth Factor A chain, Platelet-Derived Growth Factor B chain, polypeptide used to treat sepsis, Preproapolipoprotein "milano" variant, Preproapolipoprotein "paris" variant, pre-thrombin, Primate CC chemokine "ILINCK", Primate CXC chemokine "IBICK", proinsulin, Prolactin, Prolactin2, prosaptide, Protease inhibitor peptides, Protein C, Protein S, pro-thrombin, prourokinase, RANTES, RANTES 8-68, RANTES 9-68, RANTES peptide, RANTES receptor, Recombinant interleukin-16, Resistin, restrictocin, Retroviral protease inhibitors, ricin, Rotavirus Vaccine, RSV Mab, saporin, sarcin, Secreted and Transmembrane polypeptides, Secreted and Transmembrane polypeptides, serum cholinesterase, serum protein (such as a blood clotting factor), Soluble BMP Receptor Kinase Protein-3, Soluble VEGF Receptor, Stem Cell Inhibitory Factor, Straphylococcus Vaccine, Stromal Derived Factor-1 alpha, Stromal Derived Factor-1 beta, Substance P (tachykinin), T1249 peptide, T20 peptide, T4 Endonuclease, TACI, Tarc, TGF-beta 1, TGF-beta 2, Thr117 human interleukin 9, thrombin, thrombopoietin, Thrombopoietin derivative1, Thrombopoietin derivative2, Thrombopoietin derivative3, Thrombopoietin derivative4, Thrombopoietin derivative5, Thrombopoietin derivative6, Thrombopoietin derivative7, Thymus expressed chemokine (TECK), Thyroid stimulating Hormone, tick anticoagulant peptide, Tim-1 protein, TNF-alpha precursor, TNF-R, TNF-RII; TNF p75 Receptor; Death Receptor, tPA, transferrin, transforming growth factor beta, Troponin peptides, Truncated monocyte chemotactic protein 2 (6-76), Truncated monocyte chemotactic protein 2 (6-76), Truncated RANTES protein (3-68), tumour necrosis factor, Urate Oxidase, urokinase, Vasopressin (Neurophysin II), VEGF R-3; flt-4, VEGF Receptor; KDR; flk-1, VEGF-110, VEGF-121, VEGF-138, VEGF-145, VEGF-162, VEGF-165, VEGF-182, VEGF-189, VEGF-206, VEGF-D, VEGF-E; VEGF-X, von Willebrand's factor, Wild type monocyte chemotactic protein 2, Wild type monocyte chemotactic protein 2, ZTGF-beta 9, alternative antibody scaffolds e.g. anticalin(s), adnectin(s), fibrinogen fragment(s), nanobodies such as camelid nanobodies, infestin, and/or any of the molecules mentioned in WO01/79271 (particularly page 9 and/or Table 1), WO 2003/59934 (particularly Table 1), WO03/060071 (particularly Table 1) or WO01/079480 (particularly Table 1) (each incorporated herein by reference in their entirety).

Furthermore, conjugates may comprise one or more (several) of chemotherapy drugs such as: 13-cis-Retinoic Acid, 2-CdA, 2-Chlorodeoxyadenosine, 5-Azacitidine, 5-Fluorouracil, 5-FU, 6-Mercaptopurine, 6-MP, 6-TG, 6-Thioguanine, A, Abraxane, Accutane®, Actinomycin-D, Adriamycin®, Adrucil®, Agrylin®, Ala-Cort®, Aldesleukin, Alemtuzumab, ALIMTA, Alitretinoin, Alkaban-AQ®, Alkeran®, All-transretinoic Acid, Alpha Interferon, Altretamine, Amethopterin, Amifostine, Aminoglutethimide, Anagrelide, Anandron®, Anastrozole, Arabinosylcytosine, Ara-C, Aranesp®, Aredia®, Arimidex®, Aromasin®, Arranon®, Arsenic Trioxide, Asparaginase, ATRA, Avastin®, Azacitidine, BCG, BCNU, Bevacizumab, Bexarotene, BEXXAR®, Bicalutamide, BiCNU, Blenoxane®, Bleomycin, Bortezomib, Busulfan, Busulfex®, C225, Calcium Leucovorin, Campath®, Camptosar®, Camptothecin-11, Capecitabine, Carac™, Carboplatin, Carmustine, Carmustine Wafer, Casodex®, CC-5013, CCNU, CDDP, CeeNU, Cerubidine®, Cetuximab, Chlorambucil, Cisplatin, Citrovorum Factor, Cladribine, Cortisone, Cosmegen®, CPT-11, Cyclophosphamide, Cytadren®, Cytarabine, Cytarabine Liposomal, Cytosar-U®, Cytoxan®, Dacarbazine, Dacogen, Dactinomycin, Darbepoetin Alfa, Dasatinib, Daunomycin, Daunorubicin, Daunorubicin Hydrochloride, Daunorubicin Liposomal, DaunoXome®, Decadron, Decitabine, Delta-Cortef®, Deltasone®, Denileukin diftitox, DepoCyt™, Dexamethasone, Dexamethasone acetate, Dexamethasone Sodium Phosphate, Dexasone, Dexrazoxane, DHAD, DIC, Diodex, Docetaxel, Doxil®, Doxorubicin, Doxorubicin liposomal, Droxia™, DTIC, DTIC-Dome®, Duralone®, Efudex®, Eligard™, Ellence™, Eloxatin™, Elspar®, Emcyt®, Epirubicin, Epoetin alfa, Erbitux™, Erlotinib, Erwinia L-asparaginase, Estramustine, Ethyol, Etopophos®, Etoposide, Etoposide Phosphate, Eulexin®, Evista®, Exemestane, Fareston®, Faslodex®, Femara®, Filgrastim, Floxuridine, Fludara®, Fludarabine, Fluoroplex®, Fluorouracil, Fluorouracil (cream), Fluoxymesterone, Flutamide, Folinic Acid, FUDR®, Fulvestrant, G-CSF, Gefitinib, Gemcitabine, Gemtuzumab ozogamicin, Gemzar®, Gleevec™, Gliadel® Wafer, GM-CSF, Goserelin, Granulocyte - Colony Stimulating Factor, Granulocyte Macrophage Colony Stimulating Factor, Halotestin®, Herceptin®, Hexadrol, Hexalen®, Hexamethylmelamine, HMM, Hycamtin®, Hydrea®, Hydrocort Acetate®, Hydrocortisone, Hydrocortisone Sodium Phosphate, Hydrocortisone Sodium Succinate, Hydrocortone Phosphate, Hydroxyurea, Ibritumomab, Ibritumomab Tiuxetan, Idamycin®, Idarubicin, Ifex®, IFN-alpha, Ifosfamide, IL-11, IL-2, Imatinib mesylate, Imidazole Carboxamide, Interferon alfa, Interferon Alfa-2b (PEG Conjugate), Interleukin-2, Interleukin-11, Intron A® (interferon alfa-2b), Iressa®, Irinotecan, Isotretinoin, Kidrolase®, Lanacort®, Lapatinib, L-asparaginase, LCR, Lenalidomide, Letrozole, Leucovorin, Leukeran, Leukine™, Leuprolide, Leurocristine, Leustatin™, Liposomal Ara-C, Liquid Pred®, Lomustine, L-PAM, L-Sarcolysin, Lupron®, Lupron Depot®, M, Matulane®, Maxidex, Mechlorethamine, Mechlorethamine Hydrochloride, Medralone®, Medrol®, Megace®, Megestrol, Megestrol Acetate, Melphalan, Mercaptopurine, Mesna, Mesnex™, Methotrexate, Methotrexate Sodium, Methylprednisolone, Meticorten®, Mitomycin, Mitomycin-C, Mitoxantrone, M-Prednisol®, MTC, MTX, Mustargen®, Mustine, Mutamycin®, Myleran®, Mylocel™, Mylotarg®, Navelbine®, Nelarabine, Neosar®, Neulasta™, Neumega®, Neupogen®, Nexavar®, Nilandron®, Nilutamide, Nipent®, Nitrogen Mustard, Novaldex®, Novantrone®, Octreotide, Octreotide acetate, Oncospar®, Oncovin®, Ontak®, Onxal™, Oprevelkin, Orapred®, Orasone®, Oxaliplatin, a taxol or taxol derivative e.g. Paclitaxel or Paclitaxel Protein-bound, Pamidronate, Panitumumab, Panretin®, Paraplatin®, Pediapred®, PEG Interferon, Pegaspargase, Pegfilgrastim, PEG-INTRON™, PEG-L-asparaginase, PEMETREXED, Pentostatin, Phenylalanine Mustard, Platinol®, Platinol-AQ®, Prednisolone, Prednisone, Prelone®, Procarbazine, PROCRIT®, Proleukin®, Prolifeprospan 20 with Carmustine Implant, Purinethol®, R, Raloxifene, Revlimid®, Rheumatrex®, Rituxan®, Rituximab, Roferon-A® (Interferon Alfa-2a), Rubex®, Rubidomycin hydrochloride, Sandostatin®, Sandostatin LAR®, Sargramostim, Solu-Cortef®, Solu-Medrol®, Sorafenib, SPRYCEL™, STI-571, Streptozocin, SU11248, Sunitinib, Sutent®, Tamoxifen, Tarceva®, Targretin®, Taxol®, Taxotere®, Temodar®, Temozolomide, Teniposide, TESPA, Thalidomide, Thalomid®, TheraCys®, Thioguanine, Thioguanine Tabloid®, Thiophosphoamide, Thioplex®, Thiotepa, TICE®, Toposar®, Topotecan, Toremifene, Tositumomab, Trastuzumab, Tretinoin, Trexall™, Trisenox®, TSPA, TYKERB®, VCR, Vectibix™, Velban®, Velcade®, VePesid®, Vesanoid®, Viadur™, Vidaza®, Vinblastine, Vinblastine Sulfate, Vincasar Pfs®, Vincristine, Vinorelbine, Vinorelbine tartrate, VLB, VM-26, Vorinostat, VP-16, Vumon®, Xeloda®, Zanosar®, Zevalin™, Zinecard®, Zoladex®, Zoledronic acid, Zolinza, Zometa®; radiopharmaceuticals such as: Carbon-11, Carbon-14, Chromium-51, Cobalt-57, Cobalt-58, Erbium-169, Fluorine-18, Gallium-67, Gold-198, Indium-111, Indium-113m, Iodine-123, Iodine-125, Iodine-131, Iron-59, Krypton-81m, Nitrogen-13, Oxygen-15, Phosphorous-32, Rhenium-186, Rubidium-82, Samarium-153, Selenium-75, Strontium-89, Technetium-99m, Thallium-201, Tritium, Xenon-127, Xenon-133, Yttrium-90; imaging agents such as Gadolinium, magnetite, manganese, technetium, 1125, 1131, P32, TI201, lopamidol, PET-FDG.

Further fusion partners, conjugation partners and/or molecules for inclusion in a nanoparticle, associate or composition according to the invention include: acromegaly drugs *e.g.* somatuline, lanreotide, octreotide, Sandostatin; antithrombotics *e.g.* bivalirudin, Angiomax, dalteparin, Fragmin, enoxaparin, Lovenox, Drotrecogin alfa (*e.g.* Activated), Xigris, heparin; assisted reproductive therapy compounds *e.g.* choriogonadotropin, Ovidrel, follitropin, alpha/beta; enzymes *e.g.* hyaluronidase, Hylenex; diabetes drugs *e.g.* exenatide, Byetta, glucagon, insulin, liraglutide, albiglutide, GLP-1 agonists, exendin or an exendin analog; compounds useful in diagnosis *e.g.* protirelin, Thyrel TRH Thypinone, secretin (*e.g.* synthetic human), Chirhostim, thyrotropin (*e.g.* alpha), Thyrogen' erythropoiesis drugs *e.g.* Darbepoetin alfa, Aranesp, Epoetin alfa, Epogen, Eprex, drugs for the treatment of genetic defects *e.g.* pegademase, drugs for the treatment of growth failure *e.g.* Adagen, mecasermin, rinfabate, drugs for the treatment of cystic fibrosis *e.g.* Dornase alfa, Pulmozyme, drugs for the treatment of metaoblic disorders *e.g.* Agalsidase beta, Fabrazyme, alglucosidase alpha, Myozyme, Laronidase, Aldurazyme, drugs for the treatment of genital wart intralesional *e.g.* Interferon alfa-n3, Alferon N, drugs for the treatment of granulomatous disease *e.g.* Interferon gamma-1b, Actimmune; drugs for the treatment of growth failure *e.g.* pegvisomant, Somavert, somatropin, Genotropin, Nutropin, Humatrope,Serostim, Protropin; drugs for the treatment of heart failure *e.g.* nesiritide, Natrecor; drugs for the treatment of hemophilia *e.g.* a coagulation factor *e.g.* Factor VIII, Helixate FS, Kogenate FS, Factor IX, BeneFIX, Factor VIIa, Novoseven, desmopressin, Stimate, DDAVP; hemopoetic drugs *e.g.* Filgrastim (G-CSF), Neupogen, Oprelvekin, Neumega, Pegfilgrastim, Neulasta, Sargramostim, Leukine; drugs for the treatment of hepatitis C *e.g.* Interferon alfa-2a, Roferon A, Interferon alfa-2b, Intron A, Interferon alfacon-1, Infergen, Peginterferon alfa-2a, Pegasys, Peginterferon alfa-2b, PEG-Intron; drugs for the treatment of HIV *e.g.* enfuvirtide, Fuzeon; Fabs *e.g.* Fab (antithrombin), Abciximab, ReoPro; monoclonal antibodies *e.g.* Daclizumab, Zenapax; antiviral monoclonal antibodies *e.g.* Palivizumab, Synagis; monoclonal antibodies for the treatment of asthma *e.g.* Omalizumab, Xolair; monoclonal antibodies for use in diagnostic imaging *e.g.* Arcitumomab, CEA-Scan, Capromab Pendetide, ProstaScint, Satumomab Pendetide, OncoScint CR/OV, Fabs for use in diagnostic imaging *e.g.* Nofetumomab, Verluma; iimmuno-supressant monoclonal antibodies *e.g.* Basiliximab, Simulect, Muromonab-CD3, Orthoclone OKT3; monoclonal antibodies for the treatment of malignancy *e.g.* Alemtuzumab, Campath, Ibritumomab tiuxetan, Zevalin, Rituximab, Rituxan, Trastuzumab, Herceptin; monoclonal antibodies for the treatment of rheumatoid arthritis (RA) *e.g.* Adalimumab, Humira, Infliximab, Remicade; monoclonal antibodies for use as a radio-immuno-therapeutic *e.g.* Tositumomab and Iodine I¹³¹, Tositumomab, Bexxar; drugs for the treatment of macular degeneration *e.g.* pegaptanib, Macugen; drugs for the treatment of malignancy *e.g.* Aldesleukin, Proleukin, Interleukin-2, Asparaginase, Elspar, Rasburicase, Elitek, Denileukin diftitox, Ontak, Pegaspargase, Oncaspar, goserelin, leuprolide; drugs for the treatment of multiple sclerosis (MS) *e.g.* Glatiramer acetate (*e.g.* copolymer-1), Copaxone, Interferon beta-1a, Avonex, Interferon beta-1a, Rebif, Interferon beta-1 b, Betaseron; drugs for the treatment of mucositis *e.g.* palifermin, Kepivance; drug for the treatment of dystonia *e.g.,* neurotoxin, Botulinum Toxin Type A, BOTOX, BOTOX Cosmetic, Botulinum Toxin Type B, MYOBLOC; drugs for the treatment of osteoporosis *e.g.* teriparatide,Forteo; drugs for the treatment of psoriasis *e.g.* Alefacept, Amevive; drugs for the treatment of RA *e.g.* abatacept, Orencia, Anakinra, Kineret, Etanercept, Enbrel; thrombolytics *e.g.* Alteplase, Activase, rtPA, Anistreplase, Eminase, Reteplase, Retavase, Streptokinase, Streptase, Tenecteplase, TNKase, Urokinase, Abbokinase, Kinlytic; drugs for the treatment of osteoporosis *e.g.* calcitonin (*e.g.* salmon), Miacalcin, Fortical, drugs for the treatment of skin ulcers e.g. Becaplermin, Regranex, Collagenase, Santyl.

It is not clear what determines the plasma half-life of albumin conjugates, fusion polypeptides or associates (for example but not limited to Levemir®, Kurtzhals P et al. Biochem. J. 1995; 312:725-731), but it appears to be a result of the combination of the albumin and the selected therapeutic agent. It would be desirable to be able to control the plasma half-life of given albumin conjugates, associates or albumin fusion polypeptides so that a longer or shorter plasma half-life can be achieved than given by the individual components of the association, conjugation or fusion, in order to be able to design a particular drug according to the particulars of the indication intended to be treated.

In order to aid this design the present invention provides a method to assess one or more (several) pharmacokinetic properties of a variant HSA compared to wild type HSA using a non-primate animal model. "Pharmacokinetics" is understood as the determination of the fate of substances administered externally to a living organism. Routes of administration can, for example, be oral, enteral (rectal), mucosal or parenteral. Preferably, the administration is parenteral. Preferred parenteral administration routes are selected from intravenous, intramuscular, subcutaneous or intrapleural administration. In the present invention the preferred pharmacokinetic property or properties measured are selected from half-life, volume of distribution, bioavailability, area under the curve (AUC), clearance rate and immunogenicity. The preferred pharmacokinetic property measured in relation to albumin, albumin variants and fragments and modifications thereof is half-life. The half-life of HSA is generally measured by injecting a model animal subcutaneously, intramuscularly or intravenously with a relevant HSA sample (e.g. Wt HSA, or variant HSA or modified HSA). For formulations targeting oral, enteral (rectal) or mucosal delivery other administration routes can be selected. The dose can be varied depending on the animal model, a dose between 1 to 100mg/kg, preferably 5 to 50 mg/kg, preferably from 10 to 40 mg/kg, more preferably from 15 to 30 mg/kg will generally be appropriate unless the molecule is very large then the dose may be increased. Blood samples are collected from the animals before injection and at subsequent intervals. The intervals will depend on the animal model as the half-life is expected to vary with the size of the animal (see Table 2). For a mouse, sampling times can for example be 12, 24, 72, 168, 240 and 300 hours post injection. For a pig the sampling times can for example be 2, 4, 8, 12, 16, 20, 24, 28 and 32 days. The skilled person will generally know which sampling times are appropriate based on the molecules and the animal. The plasma can be separated from the samples and stored at -80°C. The amount of HSA sample remaining in the serum can be analyzed e.g. by ELISA, mass spectrometry or Meso Scale Discovery (MSD). Briefly, plates coated with antibody directed to the therapeutic agent or detection tag can be used to capture the various albumin molecules and a secondary detection antibody (e.g. either directed towards HSA or alternate epitope of the therapeutic agent or detection tag) is added to quantitate the amount of HSA present in the sample or alternately radiolabelled albumin molecules may be used to quantify the amount of albumin remaining in the serum (in all cases the means of detections should not alter the engagement of the albumin with the FcRn receptor in its characteristic manner. Furthermore, the references sited in Table 2 describe various methods of measuring half-life of albumin in different animal models. Methods using radioactive labeling of the albumin are less preferred since this is an additional modification to the albumin or variant and may therefore influence the half-life of the albumin.

### Embodiments

1. A method for assessing one or more (several) pharmacokinetic properties of a variant HSA compared to wild type HSA comprising
   a. Selecting a non-primate animal species where the binding affinity at pH 6 of wild type HSA to the native FcRn of said animal is the same as or higher than the binding affinity of the native albumin of said animal to said FcRn;
   b. Administering the variant HSA to one animal and the wild type HSA to another animal of the non-primate animal species selected in a); and
   c. Measuring the one or more (several) pharmacokinetic properties of the variant HSA and the wild type HSA.
2. The method according to embodiment 1, wherein the binding affinity of wild type HSA to the native FcRn of said animal is between 0.8 and 3.5 fold when compared with the binding affinity of the native albumin of said animal.
3. The method according to embodiment 1 or 2, wherein the binding affinity is assed using surface plasmon resonance and a soluble animal FcRn comprising a FcRn heavy chain and a beta2-globulin from the same animal species.
4. The method according to any of the preceding embodiments, wherein the native FcRn has a histidine in the position corresponding to position 161 when aligned to SEQ ID NO: 16.
5. The method according to any of the preceding embodiments, wherein the native FcRn has a valine in the position corresponding to position 52 when aligned to SEQ ID NO: 16.
6. The method according to any of the preceding embodiments, wherein the native FcRn has a valine in the position corresponding to position 52 and a histidine in position 161 when aligned to SEQ ID NO: 16.
7. The method according to any one of the preceding embodiments, wherein the non-primate animal species is a wild type animal or a transgenic animal.
8. The method according to embodiment 7, wherein the wild type animal is selected from the group consisting of a pig, cow, goat, sheep and camel.
9. The method according to embodiment 7, wherein the wild type animal is a pig.
10. The method according to embodiment 7, wherein the transgenic animal is a double transgenic rabbit or a double transgenic rodent, preferably a mouse, guinea pig or rat, and the transgenes are human albumin and an FcRn with a histidine in the position corresponding to position 161 when aligned to SEQ ID NO: 16.
11. The method according to embodiment 10 wherein the FcRn furthermore has a valine in position 52 when aligned to SEQ ID NO: 16.
12. The method according to embodiment 10 or 11, wherein the the FcRn furthermore has a glutamic acid in position 54 a glutamine at position 56 and a histidine at position166 when aligned to SEQ ID NO: 16.
13. The method according to embodiments 10 to 12, wherein the transgene FcRn is selected from human, chimpanzee, macaque, cow, goat, sheep, camel and pig.
14. The method according to embodiment 10 to 12, wherein the transgene FcRn is human.
15. The method according to any of the preceding embodiments, wherein the variant HSA and wild type HSA is modified by fusion, conjugation or association with a partner.
16. The method according to embodiment 15, wherein the partner is a therapeutic agent or a vaccine.
17. The method according to any of the preceding embodiments, wherein the one or more (several) pharmacokinetic properties measured are selected from half-life, volume of distribution, AUC, bioavailability, clearance rate and immunogenicity.
18. The method according to any of the preceding embodiments, wherein the pharmacokinetic property measured is half-life.
19. The method according to any of the preceding embodiments, wherein the albumin is administered subcutaneously, intramuscular or intravenously.
20. The method according to any of the preceding embodiments, wherein a variant HSA or modified variant HSA with one or more (several) improved pharmacokinetic properties when compared with wild type HSA or modified wild type HSA, is selected for use in a pre-clinical trial.
21. The method according to embodiment 20, wherein the variant HSA has a longer half-life than wild type HSA.
22. A variant HSA selected by the method of any one of embodiments 19 to 21.
23. A variant HSA modified by fusion, conjugation or association with a partner, where the fusion, conjugation or association is selected by the method of any one of embodiments 19 to 21.
24. The variant according to embodiment 23, wherein the partner is a therapeutic agent or a vaccine.
25. Use of a pig animal model to compare one or more (several) pharmacokinetic properties of a control molecule and a test molecule in which the control molecule comprises wild type HSA and the test molecule comprises a variant of HSA.
26. Use of a transgenic rabbit or rodent model that expresses an FcRn with a histidine in the position corresponding to position 161 when aligned to SEQ ID NO: 16 and human albumin and which has been knocked out for the corresponding native proteins to compare one or more (several) pharmacokinetic properties of a control molecule with one or more (several) pharmacokinetic properties of a test molecule in which the control molecule comprises wild type HSA and the test molecule comprises a variant of HSA.
27. The use according to embodiment 26, wherein the FcRn furthermore has a valine in position 52 when aligned to SEQ ID NO: 16.
28. The use according to embodiment 26 or 27, wherein the transgene FcRn is selected from human, chimpanzee, macaque, sheep, cattle and pig.
29. The use according to any one of embodiments 26 to 28, wherein the molecule comprising wild type HSA and the molecule comprising variant HSA further comprise a conjugation partner, fusion partner or association partner.
30. The use according to embodiment 29, wherein the partner is a therapeutic agent or a vaccine.
31. The use according to any of embodiments 26 to 30, wherein the effect on the pharmacokinetic properties of a selected partner and the method of joining it to the control and test molecule is assessed.
32. The use according to embodiment 31, wherein the control molecule is Wt HSA joined to the partner and the test molecule is a variant HSA joined to the same partner using the same method of joining the molecules.
33. The use according to embodiment 31 or 32, wherein the partner is a conjugation partner conjugated at different positions on the albumin (positions are identical for both control and test molecule).
34. The use according to embodiment 31 or 33, wherein the partner is a conjugation partner conjugated with different conjugation technologies to the albumin (conjugation technology is identical for control and test molecule).
35. The use according to embodiment 31 or 33, wherein the partner is a fusion partner fused with or without different linkers at the C-terminal and/or N-terminal of the albumin (linkers are identical for control and test molecule).
36. A transgenic rabbit or rodent whose genome comprises a homozygous disruption in its endogenous FcRn heavy chain gene and endogenous serum albumin gene that prevents the expression of a functional rabbit or rodent FcRn HC protein and functional rabbit or rodent serum albumin and the genome further comprises a heterologous DNA sequence that expresses an FcRn with a histidine in the position corresponding to position 161 when aligned to SEQ ID NO: 16 and a heterologous DNA sequence encoding human serum (HSA)and wherein said homozygous disruption, and wherein the animal expresses a functional FcRn HC protein with a histidine in position 161 when aligned to SEQ ID NO: 16 and functional HSA.
37. A transgenic animal whose genome comprises a homozygous disruption in its endogenous FcRn HC gene and serum albumin gene that prevents the expression of a functional animal FcRn HC protein and functional animal serum albumin and the genome further comprises a heterologous DNA sequence encoding a human FcRn HC (hFcRn HC) and a heterologous DNA sequence encoding human serum albumin (HSA), and wherein the animal expresses a functional hFcRn HC protein and functional HSA.
38. The transgenic animal according to embodiment 36 or 37, wherein the FcRn is 90, 91, 92, 93, 94, 95, 96, 97, 98, 99, 99.5, 100% identical to the mature sequence of SEQ ID NO: 9 or to SEQ ID NO: 16 and the FcRn HC has a histidine in position 161 when aligned to SEQ ID NO: 16.
39. The transgenic animal according to embodiment 36 or 37, wherein the FcRn HC comprises SEQ ID NO: 16
40. The transgenic animal according to any one of embodiments 37 to 39, wherein the human HSA is 90, 91, 92, 93, 94, 95, 96, 97, 98, 99, 99.5, 100% identical to SEQ ID NO: 2.
41. The transgenic animal according to any one of embodiments 37 to 40, wherein the animal or rodent is a mouse.
42. The transgenic animal according to any one of embodiments 36 to 41, wherein the heterologous DNA replaces the endogens gene and thereby disrupts them
43. The transgenic animal according to any one of embodiments 36 to 41, wherein the disruption of the endogens gene is done independently of the insertion of the heterologous DNA, allowing the heterologous DNA to be inserted in a different place of the genome than the endogenous gene.

### Preferred embodiments include:

1. A method for assessing one or more (several) pharmacokinetic properties of a variant HSA compared to wild type HSA comprising
   a. Selecting a non-primate animal species where the binding affinity at pH 6 of wild type HSA to the native FcRn of said animal is the same as or higher than the binding affinity of the native albumin of said animal to said FcRn;
   b. Administering the variant HSA to one animal and the wild type HSA to another animal of the non-primate animal species selected in a); and
   c. Measuring the one or more (several) pharmacokinetic properties of the variant HSA and the wild type HSA.
2. The method according to preferred embodiment 1, wherein the binding affinity of wild type HSA to the native FcRn of said animal is between 0.8 and 3.5 fold when compared with the binding affinity of the native albumin of said animal.
3. The method according to preferred embodiment 1 or 2, wherein the native FcRn has a histidine in the position corresponding to position 161 when aligned to SEQ ID NO: 16.
4. The method according to any of the preceding preferred embodiments, wherein the native FcRn has a valine in the position corresponding to position 52 when aligned to SEQ ID NO: 16.
5. The method according to any one of the preceding preferred embodiment s, wherein the non-primate animal species is a wild type animal or a transgenic animal.
6. The method according to preferred embodiment 5, wherein the wild type animal is a pig.
7. The method according to preferred embodiment 5, wherein the transgenic animal is a double transgenic rabbit or a double transgenic rodent, preferably a mouse, guinea pig or rat, and the transgenes are human albumin and an FcRn with a histidine in the position corresponding to position 161 when aligned to SEQ ID NO: 16.
8. The method according to preferred embodiment 1, wherein the FcRn furthermore has a valine in position 52 when aligned to SEQ ID NO: 16.
9. The method according to preferred embodiment 1 or 1, wherein the transgene FcRn is selected from human, chimpanzee, macaque, cow, goat, sheep, camel and pig.
10. The method according to any of the preceding preferred embodiments, wherein the variant HSA and wild type HSA is modified by fusion, conjugation or association with a partner, such as a therapeutic agent.
11. The method according to any of the preceding preferred embodiments, wherein a variant HSA or modified variant HSA with one or more (several) improved pharmacokinetic properties when compared with wild type HSA or modified wild type HSA, is selected for use in a pre-clinical trial.
12. The method according to preferred embodiment 9, wherein the variant HSA has a longer half-life than wild type HSA.
13. A variant HSA selected by the method of preferred embodiment 9 or 10.
14. A variant HSA modified by fusion, conjugation or association with a partner, where the fusion, conjugation or association is selected by the method of preferred embodiment 9 or 10.
15. Use of a pig animal model to compare one or more (several) pharmacokinetic properties of a control molecule and a test molecule in which the control molecule comprises wild type HSA and the test molecule comprises a variant of HSA.
16. Use of a transgenic rabbit or rodent model that expresses an FcRn with a histidine in the position corresponding to position 161 when aligned to SEQ ID NO: 16 and human albumin and which has been knocked out for the corresponding native proteins to compare one or more (several) pharmacokinetic properties of a control molecule with one or more (several) pharmacokinetic properties of a test molecule in which the control molecule comprises wild type HSA and the test molecule comprises a variant of HSA.
17. The use according to preferred embodiment 12 or 0, wherein the molecule comprising wild type HSA and the molecule comprising variant HSA further comprise a conjugation partner, fusion partner or association partner, such as a therapeutic agent.
18. The use according to any of preferred embodiments 12 to 0, wherein the effect on the pharmacokinetic properties of a selected partner and the method of joining it to the control and test molecule is assessed.
19. The use according to preferred embodiment 0, wherein the partner is a fusion partner fused with or without different linkers at the C-terminal and/or N-terminal of the albumin (linkers are identical for control and test molecule).
20. A transgenic animal whose genome comprises a homozygous disruption in its endogenous FcRn HC gene and serum albumin gene that prevents the expression of a functional animal FcRn HC protein and functional animal serum albumin and the genome further comprises a heterologous DNA sequence encoding a human FcRn HC (hFcRn HC) that is at least 90% identical to SEQ ID NO: 16 and has a histidine in position 161 when aligned to SEQ ID NO: 16 and a heterologous DNA sequence encoding human serum albumin that is at least 95% identical to SEQ ID NO: 2, and wherein the animal expresses a functional hFcRn HC protein and functional.
21. The transgenic animal according to preferred embodiment 0, wherein the animal is a mouse.

### EXAMPLES

### Materials and Methods

Amine Coupling Kit from GE Healthcare (BR-1000-50) comprising 1-ethyl-3-(3-dimethylaminopropyl) carbodiimide hydrochloride (EDC), N-hydroxysuccinimide (NHS), 1.0 M ethanolamine-HCl pH 8.5

### Albumins:

The albumins used in the present examples were in the mature form. Serum albumin from human, mouse, rabbit and sheep were produced recombinantly using sequences provided from publicly available databases (see sequences below) using the production method as described for the variants in WO 2012/059486 methods 1 (hereby incorporated by reference). Serum albumins from dog, pig, cynomolgus macaque, guinea pig and rat were prepared using the same methods.
- Wild type human serum albumin (HSA) (SEQ ID NO: 1, mature sequence from amino acid 25 to 609 also indicated in SEQ ID NO: 2).
- Wild type rhesus monkey serum albumin (rmSA) (SEQ ID NO: 3, mature sequence from amino acid 25 to 608).
- Wild type dog serum albumin (DSA) (SEQ ID NO: 4, mature sequence from amino acid 25 to 608).
- Wild type pig serum albumin (PSA) (SEQ ID NO: 5, mature sequence from amino acid 25 to 607).
- Wild type rat serum albumin (RSA) (SEQ ID NO: 6, mature sequence from amino acid 25 to 608).
- Wild type mouse serum albumin (MSA) (SEQ ID NO: 7, mature sequence from amino acid 25 to 608).
- Wild type guinea pig (GPSA) (SEQ ID NO: 18, mature sequence from amino acid 25 to 608).
- Wild type cynomolgus macaque (CSA) (SEQ ID NO: 19, mature sequence from amino acid 24-608)

The variant HSA molecules were generated by substituting the native K573 with P, F, W, H or Y or the native HSA K500 with A. The K500A variant is known not to bind to human FcRn (a null binder). The variants were prepared as described in WO 2011/051489 Example 1, method 3 and 4 (hereby incorporated by reference). The variant HSA molecules with the following substitutions T83N+N111E+K573P was prepared as described in WO 2013/135896 Example 1 (hereby incorporated by reference). The variant HSA molecule with the following substitutions E492G+K573P+K574H+Q580K was prepared as described in PCT/EP2013/073426 Example 1, method 2 (hereby incorporated by reference).

Albumin and albumin variants with a c-myc tag were generated to enable detection of human albumin and human albumin variants in monkey and pig since antibodies towards HSA cannot distinguish native rmSA and native PSA from HSA. The c-myc tag was added by fusing the epitope with the sequence EQKLISEEDL to the N-terminal end of the albumin sequence, for details see Example 3.

### Soluble FcRn molecules:

Vectors encoding truncated versions of the three ectodomains (a1-a3) of mouse and human FcRn heavy chain cDNAs, genetically fused to a cDNA encoding the Schistosoma japonicum glutathione S-transferase (GST), have been described (Andersen et al (2008) FEBS J 275(16), 4097-4110; Andersen et al. (2010) J Biol. Chem. 12; 285(7): 4826-36, Andersen et al., 2011 J. Biol. Chem286:5234-5241). Truncated versions of the three ectodomains (a1-a3) of FcRn HC cDNAs from rhesus monkey, rat, pig and dog were synthesized by Genscript, and subcloned into the same vector system using the restriction sites Xhol (NEB) and HindIII (NEB). All vectors also contain a cDNA encoding human β2-microglobulin and the Epstein-Barr virus origin of replication (oriP). Soluble GST-tagged FcRn molecules were produced in adherent human embryonic kidney 293E cells, and secreted receptors were purified using a GSTrap column as described (Berntzen et al. (2005) J Immunol Methods 298, 93-104).

The protein sequences of the FcRn chains are listed below.
- human (shFcRn) corresponds to amino acid 24 to 291 of SEQ ID NO: 9
- rhesus monkey (srmFcRn) corresponds to amino acid 22 to 290 of SEQ ID NO: 10
- pig (pFcRn) corresponds to amino acid 23 to 290 of SEQ ID NO: 11
- dog (dFcRn) corresponds to amino acid 23 to 289 of SEQ ID NO: 12
- mouse (smFcRn) corresponds to amino acid 21 to 293 of SEQ ID NO: 13
- rat (srFcRn) corresponds to amino acid 23 to 292 of SEQ ID NO: 14
- human β2-microglobulin is indicated in SEQ ID NO: 15 (mature sequence from amino acids 21 to 119).
- guinea pig (gpFcRn) corresponds to amino acid 25 to 298 of SEQ ID NO: 17
- cynomolgus macaque (CynoFcRn) corresponds to amino acid 24-297 of SEQ ID NO: 20. Cynomolgus macaque β2-microglobulin is indicated in SEQ ID NO: 21 (mature sequence from amino acids 21 to 119)
- Pig β2-microglobulin is indicated in SEQ ID NO: 22 (mature sequence is from amino acids 21 to 118)
- guinea pig β2-microglobulin is indicated in SEQ ID NO: 23 (mature sequence from amino acids 27 to 125)

### Surface Plasmon Resonance (SPR):

SPR experiments were carried out using a Biacore 3000 instrument (GE Healthcare). Flow cells of CM5 sensor chips were coupled with soluble FcRn from one of the following species, human (shFcRn), rat (srFcRn), mouse (smFcRn), rhesus monkey (srmFcRn), dog (dFcRn) or pig (pFcRn) (∼ 900 - 2500 resonance units (RU)) using GE Healthcare amine coupling chemistry as per the manufacturer's instructions. The coupling was performed by injecting 2 or 10µg/ml FcRn in 10 mM sodium acetate pH 5.0 (GE healthcare). Phosphate buffer (67 mM phosphate buffer, 0.15 M NaCl, 0.005% Tween 20) at pH 6.0) was used as running buffer and dilution buffer. Regeneration of the surfaces were done using injections of HBS-EP buffer (0.01 M HEPES, 0.15 M NaCl, 3 mM EDTA, 0.005% surfactant P20) at pH 7.4 (GE Healthcare). For determination of binding kinetics, serial dilutions of albumin (10-0.3 µM) were injected over immobilized receptors at a constant flow rate (50 µl/min) at 25 °C. In all experiments, data were zero adjusted and the reference cell subtracted. Association rates (kₐ) and dissociation rates (k_{d}) were calculated using a simple one-to-one Langmuir binding model (BIAevaluation 4.1 software (BIAcore AB)) (Karlsson et al. (1997). J. Immunol. Methods 200, 121-133).

### Assay for quantification of human serum albumin animal model

EIA Maxisorb plates (Nunc) were coated overnight with anti-c-myc capture antibody (Abcam ab9132) at 1.25µg/mL in phosphate buffered saline (PBS) then washed 3 times with 300µL PBS+0.05%(v/v) Tween-20 (PBST), pH 7.4. Plates were blocked for 2h with 300µL PBS + 5%(w/v) skimmed milk powder + 1% (v/v) Tween-20 + 10% (v/v) rat serum (Sigma), pH 7.4. Plasma samples were diluted 1:10 in PBST then mixed with 10% Gottingen mini pig sodium citrate plasma or female cynomolgus monkey sodium citrate plasma (SeraLab catalog no. PSCGOF-444-M-32556 and PSCF-118-M-32555, respectively) in PBST. A standard curve with a top concentration of 1000ng/mL and twelve 2-fold dilution points was included on each plate with purified c-myc HSA diluted in 10% mini pig plasma or cynomolgus monkey plasma in PBST. Plates were incubated at room temperature (RT) for 1h then washed as above. 100µl of anti-HSA Biotin (Abcam ab81426) at 1.5µg/mL in PBST were added to each well and the plates were incubated for 30 min at RT. Plates were washed as above and 100µl of 1.25µg/mL streptavidin-HRP (Sigma) in PBST were added to each well and the plates were incubated for 30 min at RT. Plates were washed as above and the signal was developed with 100µL of TMB-Ultra substrate (Pierce). Absorbance was measured at 450nm using an EnSpire Multimode plate reader (Perkin Elmer). In both cases the standard curve on each plate was fitted to a 4-parameter nonlinear regression model and the plasma HSA concentration calculated at each time point using the dilutions that fell within the linear range of the standard curve.

### PK analysis

Group mean plasma concentration profiles were subjected to non-compartmental pharmacokinetic analysis using Phoenix WinNonLin 6.3. Nominal time points and doses were used and all data points were equally weighted in the analysis. Mean plasma concentrations versus time profiles for each of the HSA variants were fit with a 2-compartment model to generate the curve fit. The following pharmacokinetic parameters were assessed:
- Cₘₐₓ: The maximum serum concentration
- AUC: Area under the serum concentration curve from 0 to infinity
- t_{½}: Terminal elimination half-life in plasma
- V_{Z}: Volume of distribution during the elimination phase
- Cl: Total body clearance

### Example 1 Binding kinetics of albumins toward human, rat, mouse and rhesus monkey FcRn

The binding affinity of HSA and variant HSA to soluble FcRn from human, mouse, rat and rhesus monkey was analyzed and the HSA binding was compared with the binding of the native albumin.

The SPR results are shown in Figure 1 to 4 and the binding kinetic are summarized in Table 5.

**Table 5: Binding kinetics of albumins toward human, rat, mouse and rhesus monkey FcRn**

| **Albumin variant** | **ka (10³/Ms)** | **kd (10⁻³/s)** | **KD^{a} (µM)** | **KD^{b} (µM)** |
|---|---|---|---|---|
| **Human FcRn** | | | | |
| HSA Wt | 7.4±0.1 | 8.40±0.1 | 1.1 | 1.2 |
| K573P | 4.4±0.1 | 0.43±0.1 | 0.097 | ND |
| K573F | 7.3±0.2 | 0.48±0.1 | 0.065 | ND |
| K573H | 7.2±0.0 | 0.57±0.1 | 0.079 | ND |
| K573W | 4.4±0.1 | 0.30±0.1 | 0.068 | ND |
| K573Y | 7.4±0.1 | 0.29±0.1 | 0.040 | ND |
| K500A | NA | NA | NA | 25.0^{c} |

| **Rat FcRn** | | | | |
|---|---|---|---|---|
| HSA Wt | NA | NA | NA | ND |
| RSA Wt | 7.6±0.1 | 26.0±0.0 | 3.20 | 4.1 |
| K573P | 3.8±0.1 | 7.7±0.1 | 2.00 | 2.3 |
| K573F | 5.6±0.1 | 8.5±0.1 | 1.50 | 2.2 |
| K573H | 7.0±0.0 | 19.0±0.2 | 2.70 | 2.3 |
| K573W | 3.2±0.2 | 5.7±0.0 | 1.80 | 2.9 |
| K573Y | 4.8±0.1 | 4.6±0.1 | 1.00 | 2.0 |
| K500A | NA | NA | NA | NA |

| **Mouse FcRn** | | | | |
|---|---|---|---|---|
| HSA Wt | NA | NA | NA | 25.0 |
| MSA Wt | 16.1±0.1 | 12.2±0.0 | 0.80 | 1.0 |
| RSA Wt | 13.0±0.0 | 34.1±0.1 | 2.60 | 2.6 |
| K573P | 7.7±0.2 | 12.2±0.0 | 1.60 | 1.7 |
| K573F | 12.0±0.1 | 17.0±0.0 | 1.40 | 1.5 |
| K573H | 12.1±0.0 | 28.1±0.2 | 2.3 | 2.3 |
| K573W | 8.2±0.1 | 8.4±0.1 | 1.00 | 1.4 |
| K573Y | 12.3±0.1 | 8.0±0.0 | 0.70 | 0.9 |
| K500A | ND | ND | ND | ND |

| **Rhesus monkey FcRn** | | | | |
|---|---|---|---|---|
| HSA Wt | 8.9±0.2 | 16.0±0.1 | 1.80 | ND |
| rmSA Wt | 5.3±0.2 | 13.0±0.1 | 2.4 | ND |
| RSA Wt | 4.5±0.1 | 10.1±0.2 | 2.20 | ND |
| MSA Wt | 4.9±0.2 | 6.6±0.1 | 1.30 | ND |
| K573P | 5.7±0.1 | 1.3±0.1 | 0.23 | ND |
| K573F | 6.8±0.0 | 1.4±0.2 | 0.21 | ND |
| K573H | 7.6±0.1 | 1.8±0.1 | 0.24 | ND |
| K573W | 4.8±0.2 | 0.8±0.2 | 0.17 | ND |
| K573Y | 6.4±0.1 | 0.8±0.1 | 0.12 | ND |
| K500A | ND | ND | ND | ND |

| | | | | |
|---|---|---|---|---|
| a. The kinetic rate constants were obtained using a simple first-order (1:1) bimolecular interaction model. The kinetic values represent the average of duplicates. b. The steady state affinity constant was obtained using an equilibrium (Req) binding model supplied by the BIAevaluation 4.1 software. c. Data are taken from Andersen et al., Nature Commun., In press ND = Not determined. NA = Not acquired because of fast kinetics. | | | | |

### Conclusions

All the HSA variants showed considerably improved binding to human FcRn at acidic pH compared to Wt, and the increase was mainly due to slower dissociation rates.

HSA binds very weakly to rat and mouse FcRn when compared to the native albumins. Consequently, rat or mouse albumin will out-compete HSA for the binding to rat or mouse FcRn. For this reason rat and mouse are not desired animal models for assessing one or more (several) pharmacokinetic properties of HSA in vivo. On the other hand HSA has a measurable binding affinity to rhesus monkey FcRn, and the binding is 1.3 fold better that the binding of the native rhesus monkey albumin. This indicates that rhesus monkey is a suitable animal model for pharmacokinetic studies of HSA and HSA variants since the native rhesus monkey albumin will not out-compete HSA for FcRn binding.

### Example 2 Binding kinetics of albumins towards dog and pig FcRn

In this example the binding affinity of HSA and variant HSA to FcRn from dog and pig was analyzed and the HSA binding was compared with the binding of the native albumin.

The SPR results are shown in Figure 5 and 6 and the binding kinetics are summarized in Table 6.

**Table 6: Binding kinetics of albumins towards dog and pig FcRn**

| **Albumin variant** | **ka (10³/Ms)** | **kd (10⁻³/s)** | **KD^{a} (µM)** |
|---|---|---|---|
| **Dog FcRn** | | | |
| HSA Wt | 9.10±0.10 | 17.0±0.02 | 1.90 |
| DSA | 12.0±0.20 | 3.40±0.1 | 0.28 |
| PSA | NA | NA | 23.0^{b} |
| rmSA | 1.30±0.10 | 20.0±0.0 | 1.54 |
| RSA | 8.30±0.00 | 48.0±0.01 | 5.80 |
| MSA | 7.40±0.10 | 13.0±0.02 | 1.75 |
| K573P | 8.30±0.10 | 2.00±0.00 | 0.24 |
| K573W | 6.70±0.20 | 2.60±0.10 | 0.38 |
| K573F | 9.20±0.00 | 3.80±0.20 | 0.41 |
| K573Y | 8.10±0.10 | 2.40±0.10 | 0.29 |
| K573H | 13.0±0.10 | 5.10±0.00 | 0.39 |
| HSA K500A | NA | NA | 36.0^{b} |

| **Pig FcRn** | | | |
|---|---|---|---|
| HSA Wt | 16.0±0.31 | 15.0±0.05 | 0.93 |
| PSA | 20.0±0.30 | 53.0±0.10 | 2.70 |
| DSA | 9.20±0.10 | 13.0±0.02 | 1.40 |
| rmSA | 10.0±0.20 | 12.0±0.01 | 1.20 |
| RSA | 11.0±0.20 | 71.0±0.02 | 6.40 |
| MSA | 10.0±0.10 | 22.0±0.01 | 2.20 |
| K573P | 9.20±0.10 | 2.60±0.03 | 0.28 |
| K573W | 8.70±0.20 | 2.20±0.10 | 0.25 |
| K573F | 10.0±0.10 | 2.80±0.20 | 0.28 |
| K573Y | 9.80±0.05 | 2.30±0.02 | 0.23 |
| K573H | 14.0±0.02 | 3.50±0.01 | 0.25 |
| HSA K500A | NA | NA | 38.0^{b} |

| | | | |
|---|---|---|---|
| a. The kinetic rate constants were obtained using a simple first-order (1:1) bimolecular interaction model. The kinetic values represent the average of duplicates. b. Estimated KD based on steady-state affinity measurements. NA = Not acquired because of fast kinetics. | | | |

### Conclusions

HSA binds dog FcRn with an affinity that is 6.8 times lower than the binding affinity of native dog albumin. Consequently, it is highly likely that dog albumin will out-compete HSA binding to dog FcRn. For this reason dog is not a desired animal model for assessing one or more (several) pharmacokinetic properties of HSA in vivo.

On the other hand HSA has binding affinity to pig FcRn that is 2.9 fold higher than the binding of the native pig albumin. This indicates that pig is a suitable animal model for pharmacokinetics studies of HSA and HSA variants since the native pig albumin, even though present in excess amounts compared to HSA, will not outcompete HSA for pFcRn binding.

### Example 3 Preparation of c-myc tagged HSA and HSA variants

HSA and HSA variants were expressed using standard molecular biology techniques, such as described in Sambrook, J. and D.W. Russell, 2001 (Molecular Cloning: a laboratory manual, 3rd ed. Cold Spring Harbor Laboratory Press, Cold Spring Harbor, N.Y).

The objective was to secrete from yeast, wild-type (Wt) human albumin and variant human albumins incorporating the modifications K573P, or E492G+K573P+K574H+Q580K or T83N+N111E+K573P which incorporated a c-myc epitope at the N-terminal end of the mature albumin sequence. Secretion from yeast was enabled by the use of a prepro leader sequence, known as the modified fusion leader (mFL with the amino acid sequence MKWVFIVSILFLFSSAYS), incorporating a further modification to the pro region (amino acids RSLD). The modified fusion leader (mFL), was further modified by the inclusion of the amino acid sequence EEAEAEAESSRLKR including the Kex2p dibasic (KR) cleavage site 12568and a c-myc epitope with the sequence EQKLISEEDL inserted at the N-terminal end of the albumin sequence.

All expression cassettes comprise the *S*. *cerevisiae PRB1* promoter and a modified *S*. *cerevisiae ADH1* terminator (mADHt)

Plasmids encoding Wt albumin and the variants have previously been described (see reference in the Materials and Methods section). The plasmids where similarly digested to completion with *Bg*/II/*Sac*II and the large 8.585kb fragment isolated and replaced with the analogous 0.26kb *Bg*/II/*Sac*II fragment encoding the modified fusion leader sequence MKWVFIVSILFLFSSAYS RSLDEEAEAEAESSRLKREQKLISEEDL.

Expression plasmids were generated *in vivo* (i.e. via homologous recombination in S. *cerevisiae*; a technique referred to as gap repair or in vivo cloning- see Orr-Weaver & Szostak. 1983. Proc. Natl. Acad. Sci. USA. 80:4417-4421). The modified plasmids comprising the c-myc tag were digested to completion with *Nsi*I/*Pvu*I and the 7.41 kb fragments isolated. 100ng of the 4 isolated fragments were mixed individually with 100ng *Acc*65I/*Bam*HI-digested pDB3936 (disclosed in WO 2010/092135) and used to directly transform *S*. *cerevisiae* Strain A cir0. Strain A is a derivative of *S*. *cerevisiae* DYB7 (Payne et al (2008) Applied and Environmental Microbiology Vol 74(24): 7759-7766) with four copies of PDI integrated into the genome. Transformation of Strain A was achieved using the Sigma Yeast Transformation kit as described in WO2011/051489. The growth of yeast transformants in shake flask culture, the preparation of yeast trehalose stocks and fed-batch fermentation of the c-myc tagged albumin and albumin variants were essentially done as also described in WO 2011/051489 Example 1 (hereby incorporated by reference), with the modification that BMMS broth (0.17% (w/v) yeast nitrogen base without amino acid and ammonium sulphate (Difco), 37.8mM ammonium sulphate, 36 mM citric acid, 126mM disodium hydrogen orthophosphate pH6.5, 2% (w/v) sucrose, adjusted to pH 6.5 with NaOH) was used in both cases. A second purification step of the c-myc tagged albumin and albumin variants was performed using AlbuPure™ matrix chromatography (ProMetic BioSciences) followed by DE-FF as described in (Evans et al. (2010) Protein Expression and Purification Volume 73, Issue 2, Pages 113-124) followed by purification on Sephacryl S200 high resolution gel filtration (GE Healthcare) as to reduce the level of a +2058 Da miscleaved leader to below 5% (w/v).

### Example 4 Binding kinetics of albumins towards pig, cyno and guinea pig FcRn

In this example the binding affinity of HSA, variant HSA albumin and c-myc tagged HSA to FcRn from pig, cynomolgus macaque and guinea pig was analyzed and the HSA binding was compared with the binding of the native albumin (pig or guinea pig).

The SPR assay was conducted with the following variations to the assay described in the Materials and Method section:
The soluble FcRn receptors were synthesized by GeneArt and expressed in HEK cells. The soluble FcRn contained a HIS-tag on the C-terminal of the Beta-chain instead of a GST on the heavy chain and the beta-chain used was the native beta-chain of the animal (see Materials and Method section for the corresponding sequences). The soluble FcRn was purified by a HIS trap column followed by an additional purification using an IgG column. The soluble FcRn receptor was diluted to 20µg/mL. The phosphate buffer used as running buffer in the SPR assay was at pH 5.5 instead of pH 6.0. Albumins were diluted in the range of 20µM to 0.156µM and flowed at 30µL/min. 1:1 Langmuir or Steady state model was used.

The binding kinetics are summarized in Table 7.

**Table 7: Binding kinetics of albumins towards pig and guinea pig**

| **Albumin Variant** | **Ka (10³/Ms)** | **Kd (10⁻³/s)** | **KD µM** | Fold > PSA |
|---|---|---|---|---|
| **Pig FcRn** | | | | |
| PSA | - | - | 210* | - |
| HSA Wt | - | - | 232* | 0.9 |
| c-myc-HSA Wt | - | - | 468* | 0.4 |
| K573P | 32.7 | 62.2 | 1.9 | 110 |
| c-myc-K573P | 31.2 | 40.1 | 1.3 | 161 |
| T83N+N111E+K573P | 60.1 | 62.5 | 1.0 | 210 |
| c-myc-T83N+N111E+K573P | 48.8 | 49.0 | 1.0 | 210 |
| E492G+K573P+K574H+Q580K | 27.8 | 12.1 | 0.4 | 525 |
| c-myc-E492G+K573P+K574H+Q580K | 34.5 | 13.6 | 0.4 | 525 |

| **Cyno FcRn** | | | | |
|---|---|---|---|---|
| CSA | 2.8 | 98 | 35 | - |
| WT HSA | 2.7 | 108 | 40 | 0.9 |
| Cmyc-WT HSA | 2.5 | 108 | 43 | 0.8 |
| K573P | 5.1 | 22 | 4 | 8.8 |
| c-myc-K573P | 5.7 | 20 | 4 | 8.8 |
| T83N+N111E+K573P | 7.6 | 18 | 2 | 17.5 |
| c-myc-T83N+N111E+K573P | 7.0 | 22 | 3 | 11.7 |
| E492G+K573P+K574H+Q580K | 4.6 | 5.5 | 1 | 35 |
| c-myc-E492G+K573P+K574H+Q580K | 4.9 | 5.9 | 1 | 35 |

| **gpFcRN** | | | | |
|---|---|---|---|---|
| GPSA | 36.2 | 41.9 | 1.1 | |
| PSA | NA | NA | NA | |
| HSA Wt | NA | NA | NA | |
| c-myc-HSA | NA | NA | NA | |
| K573P | 1.5 | 297 | 190 | |
| c-myc-K573P | 0.5 | 181 | 360 | |
| T83N+N111E+K573P | 0.7 | 279 | 410 | |
| c-myc-T83N+N111E+K573P | 0.4 | 216 | 550 | |
| E492G+K573P+K574H+Q580K | 5.8 | 62.8 | 10.9 | |
| c-myc-E492G+K573P+K574H+Q580K | 5.7 | 76.8 | 13 | |

| | | | | |
|---|---|---|---|---|
| *KD values without kinetics were determined using a steady state model NA = Not acquired because of fast kinetics. | | | | |

### Conclusions

Wt HSA and PSA binding affinities to pig FcRn are comparable. The binding affinities are somewhat lower than expected and may be due to the quality of the soluble receptor not being as high as expected. The 0.9 fold difference between native pig albumin and Wt HSA however still indicates that pig is a suitable animal model for pharmacokinetics studies of HSA and HSA variants since the native pig albumin, even though present in excess amounts compared to HSA, will not outcompete HSA for pFcRn binding.

The c-myc tag appears to affect the binding of Wt HSA to the pig FcRn, however at such low affinity (high KD) very little disturbance in the molecule may affect the binding. For the albumin variants the c-myc tag appears to either enhance binding (K573P) or not affect it. All HSA variants bind to pig FcRn with greater affinity than Wt HSA and PSA. HSA variant T83N+N111E+K573P has the fastest association to pig FcRn and HSA variant E492G+K573P+K574H+Q580K forms the most stable complexes (slowest off rate(kd)).

A comparative study using cyno FcRn showed that the binding affinity of Wt HSA is 0.9 fold when compared to the binding of native cyno albumin to cyno FcRn. This indicates that cyno and pig are comparable with respect to ratios although the binding affinities as such are somewhat higher for the cyno FcRn, as mentioned above this may however be due to low quality pig FcRn in the present study. The c-myc tags have very little effect on the binding affinity.

Wt HSA and pig SA bind very poorly to guinea pig FcRn. Fusion of a c-myc tag to the albumins appears to inhibit binding. The guinea pig SA has the highest affinity to guinea pig FcRn when compared to Wt pig and human SA as well as human albumin variants. For this reason guinea pig is not a desired animal model for assessing one or more (several) pharmacokinetic properties of HSA in vivo.

### Example 5 Pig PK study

PK studies were performed in 8 female Göttingen minipigs (Ellegaard Göttingen Minipigs A/S, Soro Landevej 302, DK-4261 Dalmose). At start of the acclimatisation period the minipigs were about 5 months old and had a body weight of 7.8 - 9.7 kg. The animals were divided into 4 groups with 2 animals per group. N-terminally C-myc tagged Wt HSA, N-terminally C-myc tagged albumin variant K573P, N-terminally C-myc tagged albumin variant E492G+K573P+K574H+Q580K and N-terminally C-myc tagged albumin variant T83N+N111 E+K573P were given i.v. via a venflon in an ear vein to two animals in a dose of 1 mg/kg (0.2 ml/kg) of a dose solution of 5 mg/ml according to table 8:

**Table 8: Dose administration table for pig PK study**

| Group no. | Animal nos. | No. of animals | Compound | Route of admin. | Dose (mg/kg) | Dose volume (ml/kg) |
|---|---|---|---|---|---|---|
| 1 | 1-2 | 2 | N-terminally C-myc tagged Wt HSA | i.v. | 1 | 0.2 |
| 2 | 3-4 | 2 | N-terminally C-myc tagged albumin variant K573P | i.v. | 1 | 0.2 |
| 3 | 5-6 | 2 | N-terminally C-myc tagged albumin variant E492G+K573P+K574H+Q580K | i.v. | 1 | 0.2 |
| 4 | 7-8 | 2 | N-terminally C-myc tagged albumin variant T83N+N111E+K573P | i.v. | 1 | 0.2 |

Two ml (2ml) blood samples were collected from a jugular vein into sodium citrate test tubes at the following intervals: pre-dose, 0.25, 2, 8, 24, 48, 72, 96, 120, 144, 168, 192, 216, 240, 264, 288, 312, 360, 408, 456, and 504 hours after dosing. Blood samples were kept on ice for max 20 min. before centrifugation at 4°C, 10 min., 2000 G. Plasma was immediately transferred from each sample to three appropriately labelled 750 µL Micronic tubes, approximately 250 µl in each and eventually remaining plasma shared between the three tubes, and placed in racks. The plasma samples were stored at -80°C until assayed.

### Results:

The PK-analysis was only successful for 4 animals out of the 8 animals tested as judged from the coefficient of determination for the slope of terminal elimination phase of the In-plasma concentration-time curve, R² (Rsq), which needs to be above 0.85 to get acceptable estimates of AUC, t_{½}, V_{z}, and CL. The result from the pharmacokinetic study of animals 1,2, 5 and 7 is shown in table 9.

**Table 9 pharmacokinetic parameters from pig PK study**

| **Compound** | **Animal** | **R²** | **Cmax (µg/mL)** | **AUC (h.µg/mL)** | **t_{1/2} (h)** | **Vz (mL/Kg)** | **Cl (mL/h/Kg)** |
|---|---|---|---|---|---|---|---|
| **WtHSA** | 1 | 0.86 | 16.4 | 926.12 | 192.34 | 299.63 | 1.08 |
| | 2 | 0.95 | 9.25 | 554.03 | 197.75 | 514.94 | 1.8 |
| **Mean** | | **0.9** | **12.83** | **740.08** | **195.05** | **407.28** | **1.44** |
| **SD** | | **0.06** | **5.06** | **263.11** | **3.82** | **152.25** | **0.51** |
| **E492G+K573P+K574H+ Q580K** | 5 | 0.95 | 8.55 | 817.82 | 354.95 | 626.15 | 1.22 |
| **T83N+N111E+K573P** | 7 | 0.93 | 10.47 | 1059.89 | 333.24 | 453.6 | 0.94 |

### Conclusion:

The human albumin variants E492G+K573P+K574H+Q580K and T83N+N111E+K573P showed a 1.8 and 1.7 fold longer half-life, respectively, compared to Wt human albumin. This clearly indicates that half-life extension of albumin variants can be compared to the half-life of Wt HSA and a clear differentiation in the half-life between a variant HSA and Wt HSA can be observed with the current model.

Interestingly enough the HSA variant with the longest half-life correlates with the HSA variant having the strongest binding affinity in Biacore on pig FcRn (See example 4). However, before concluding that such a correlation exist it must be kept in mind that the PK data for the variants are based on only one animal for each variant.

The prolongation in half-life is a result of decreased clearance rate which in turn reflects increased AUC.

### Example 6 Comparative Cyno PK study

PK studies were performed in 8 female cynomolgus macaques (Huntingdon Life Sciences, Huntingdon Research Centre. Woolley Road, Alconbury. Huntingdon. Cambridgeshire PE28 4HS. UK). At start of the acclimatisation period the cynomolgus macaques were 2.5 - 3.0 years old and had a body weight of 2.5 - 3.5kg. The animals were divided into 4 groups with 2 animals per group. N-terminally C-myc tagged Wt HSA, N-terminally C-myc tagged albumin variant K573P, N-terminally C-myc tagged albumin variant E492G+K573P+K574H+Q580K and N-terminally C-myc tagged albumin variant T83N+N111E+K573P were given i.v. via a saphenous vein to two animals in a dose of 1 mg/kg (1 ml/kg) of a dose solution of 1 mg/ml according to table 10.

**Table 10: Dose administration table for cyno PK study**

| Group no. | Animal nos. | No. of animals | Compound | Route of admin. | Dose (mg/kg) | Dose volume (ml/kg) |
|---|---|---|---|---|---|---|
| 1 | 412, 414 | 2 | N-terminally C-myc tagged Wt HSA | i.v. | 1 | 1.0 |
| 2 | 416, 418 | 2 | N-terminally C-myc tagged albumin variant K573P | i.v. | 1 | 1.0 |
| 3 | 420, 422 | 2 | N-terminally C-myc tagged albumin variant E492G, K573P, K574H, Q580K | i.v. | 1 | 1.0 |
| 4 | 424, 426 | 2 | N-terminally C-myc tagged albumin variant T83N+N111E+K573P | i.v. | 1 | 1.0 |

Blood samples of 0.8mL were drawn from unanaesthetized animals via suitable vein puncture using a sterile lancet into HLS standard tubes with Sodium citrate 3.8% (0.13M) at the following intervals: Pre-dose, 0.25, 2, 8, 24, 48, 72, 96, 120, 144, 192, 240, 288, 336, 384, 432, 480, 528, 576, 624, 720, 816, 912, 1008, 1104 and 1200 hours after dosing. Samples were mixed thoroughly by inversion and immediately placed on wet ice for a maximum of 10 minutes prior to centrifugation (2000 x g for 10 minutes at 4°C). Plasma was pipetted into labelled 1.4 mL Micronic tubes, snap frozen on dry ice and kept at - 80°C. The plasma samples were stored at -80°C until assayed.

### Results

The results from the pharmacokinetic study is shown in table 11.

**Table 11 pharmacokinetic parameters from cyno PK study**

| **Compound** | **R²** | **Cmax (µg/mL)** | **AUC (h.µg/mL)** | **t_{1/2} (h)** | **Vz (mL/Kg)** | **Cl (mL/h/Kg)** |
|---|---|---|---|---|---|---|
| **Wt HSA** | 0.97 | 11.48 | 511.46 | 133.29 | 375.98 | 1.96 |
| | 0.98 | 14.36 | 573.58 | 130.2 | 327.49 | 1.74 |
| **Mean** | **0.97** | **12.92** | **542.52** | **131.76** | **351.74** | **1.85** |
| **SD** | **0.004** | **2.035** | **43.92** | **2.19** | **34.29** | **0.15** |
| **K573P** | 0.97 0.98 | 11.78 10.32 | 865.94 830.54 | 212.85 208.64 | 354.61 362.42 | 1.15 1.2 |
| **Mean** | **0.98** | **11.05** | **848.24** | **210.75** | **358.52** | **1.18** |
| **SD** | **0.01** | **1.03** | **25.03** | **2.97** | **5.52** | **0.04** |
| **E492G+K573P+K574H+ Q580K** | 0.94 | 10.54 | 908.44 | 293.88 | 466.71 | 1.1 |
| | 0.93 | 25.51 | 1560.2 | 279.04 | 258.02 | 0.64 |
| **Mean** | **0.94** | **18.02** | **1234.32** | **286.46** | **362.37** | **0.87** |
| **SD** | **0.01** | **10.58** | **460.86** | **10.50** | **147.57** | **0.33** |
| **T83N+N111E+K573P** | 0.96 | 12.21 | 1383.1 | 339.36 | 353.98 | 0.72 |
| | 0.92 | 15.8 | 1357.29 | 301.28 | 320.24 | 0.74 |
| **Mean** | **0.94** | **14.00** | **1370.19** | **320.32** | **337.11** | **0.73** |
| **SD** | **0.03** | **2.54** | **18.25** | **26.93** | **23.86** | **0.01** |

### Conclusion:

The human albumin variants K573P, E492G+K573P+K574H+Q580K and T83N+N111E+K573P showed a 1.6, 2.1 and 2.4 fold longer half-life, respectively, compared to Wt human albumin

The prolongation in half-life is a result of decreased clearance rate which in turn reflects increased AUC.

## Claims

1. A method for assessing one or more (several) pharmacokinetic properties of a variant HSA compared to wild type HSA comprising
a. Selecting a non-primate animal species where the binding affinity at pH 6 of wild type HSA to the native FcRn of said animal is the same as or higher than the binding affinity of the native albumin of said animal to said FcRn;
b. Administering the variant HSA to one animal and the wild type HSA to another animal of the non-primate animal species selected in a); and
c. Measuring the one or more (several) pharmacokinetic properties of the variant HSA and the wild type HSA
wherein the non-primate animal species is selected from is selected from the group consisting of a pig, cow, goat, sheep and camel.

2. The method according to claim 1, wherein the binding affinity of wild type HSA to the native FcRn of said animal is between 0.8 and 3.5 fold when compared with the binding affinity of the native albumin of said animal.

3. The method according to claim 1 or 2, wherein the native FcRn has a histidine in the position corresponding to position 161 when aligned to SEQ ID NO: 16.

4. The method according to any of the preceding claims, wherein the native FcRn has a valine in the position corresponding to position 52 when aligned to SEQ ID NO: 16.

5. The method according to any one of the preceding claims, wherein the non-primate animal species is a wild type animal or a transgenic animal.

6. The method according to claim 5, wherein the wild type animal is a pig.

7. The method according to claim 5, wherein the transgene FcRn is selected from human, chimpanzee, macaque, cow, goat, sheep, camel and pig.

8. The method according to any of the preceding claims, wherein the variant HSA and wild type HSA is modified by fusion, conjugation or association with a partner, such as a therapeutic agent or a vaccine.

9. The method according to any of the preceding claims, wherein a variant HSA or modified variant HSA with one or more (several) improved pharmacokinetic properties when compared with wild type HSA or modified wild type HSA, is selected for use in a pre-clinical trial.

10. The method according to claim 9, wherein the variant HSA has a longer half-life than wild type HSA.

11. A variant HSA modified by fusion, conjugation or association with a partner, where the fusion, conjugation or association is selected by the method of claim 9 or 10.

12. Use of a pig, goat, sheep, cow or camel animal model to compare one or more pharmacokinetic properties of a control molecule and a test molecule in which the control molecule comprises wild type HSA and the test molecule comprises a variant of HSA.

13. The use according to claim 12, wherein the molecule comprising wild type HSA and the molecule comprising variant HSA further comprise a conjugation partner, fusion partner or association partner, such as a therapeutic agent.

14. The use according to claim 12 or 13, wherein the effect on the pharmacokinetic properties of a selected partner and the method of joining it to the control and test molecule is assessed.

15. The use according to claim 14, wherein the partner is a fusion partner fused with or without different linkers at the C-terminal and/or N-terminal of the albumin (linkers are identical for control and test molecule).
